(19) Europäisches Patentamt
European Patent Office
Office européen des brevets

(11) EP 3 950 683 A1

(12) EUROPEAN PATENT APPLICATION

(43) Date of publication:
**09.02.2022 Bulletin 2022/06**

(21) Application number: **20189671.9**

(22) Date of filing: **05.08.2020**

(51) International Patent Classification (IPC):
***C07D 401/14*** (2006.01) ***C07D 403/14*** (2006.01)
***C07D 409/14*** (2006.01) ***C07D 413/14*** (2006.01)
***C07D 498/04*** (2006.01) ***A61P 25/00*** (2006.01)
***A61K 31/501*** (2006.01)

(52) Cooperative Patent Classification (CPC):
**C07D 401/14; C07D 403/14; C07D 409/14; C07D 413/14; C07D 498/04**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Boehringer Ingelheim International GmbH**
**55216 Ingelheim am Rhein (DE)**

(72) Inventor: **The designation of the inventor has not yet been filed**

(74) Representative: **Simon, Elke Anna Maria et al**
**Boehringer Ingelheim GmbH**
**Binger Strasse 173**
**55216 Ingelheim am Rhein (DE)**

# (54) DIFLUOROMETHYL-PYRIDIN-2-YL TRIAZOLES

(57) The present invention relates to difluoromethyl-pyridin-2-yl triazoles of general formula (I) which are modulators of $GABA_A$ receptors containing the α5 subunit, useful in treating central nervous system diseases and other diseases. In addition, the invention relates to processes for preparing pharmaceutical compositions as well as processes for the manufacture of compounds according to the invention.

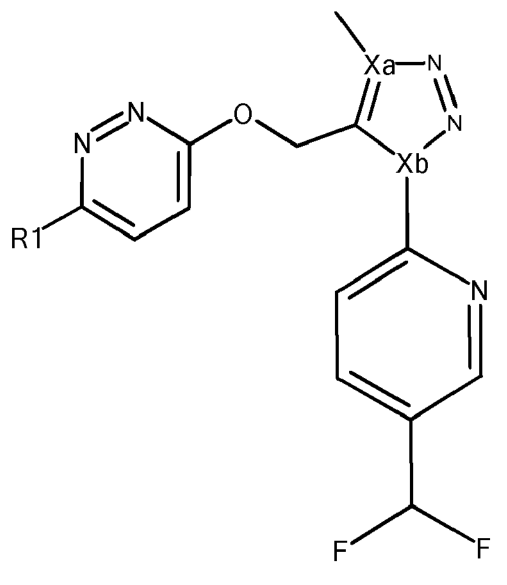
(I),

EP 3 950 683 A1

## Description

Field of the invention

The present invention relates to difluoromethyl-pyridin-2-yl triazoles of general formula (I) which are modulators of $GABA_A$ receptors containing the $\alpha$5 subunit. Thte compounds are useful in treating central nervous system and other diseases. In addition, the invention relates to processes for preparing pharmaceutical compositions as well as processes for manufacturing the compounds according to the invention.

Background of the invention

It has been suggested that the $GABA_A$ $\alpha$5 subunit represents a therapeutic target for treatment of various diseases and disorders of the central nervous system. A nexus has been established between the $GABA_A$ $\alpha$5 subunit as therapeutic target, and various neurological disorders, disorders of circadian rhythms, pain conditions. Compounds capable of modulating $GABA_A$ receptors containing the $\alpha$5 subunit are in particular expected to be useful candidates for the treatment of i.a. cognitive disorders, Alzheimer's disease, schizophrenia, positive, negative and/or cognitive symptoms associated with schizophrenia, cognitive impairment associated with schizophrenia and cognitive deficits associated with Down syndrome, with autism, with neurofibromatosis type I, or after stroke.

Aim of the invention

Surprisingly it has been found that pyridine-2-yl-triazoles of the general formula (I),

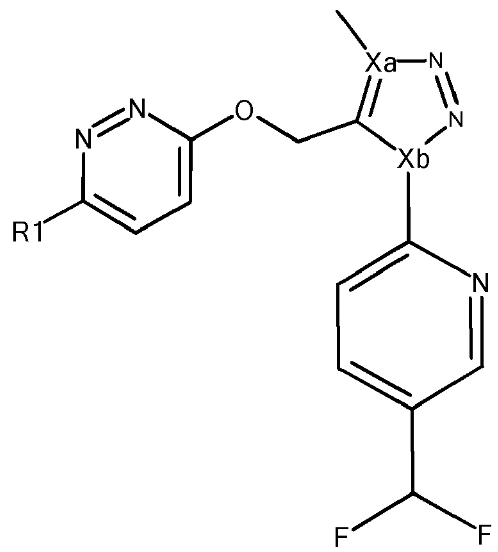

(I),

wherein:

- **Xa and Xb** are different from each other and represent C or N and
- **R1** is a substituted phenyl or a 5- or 6- membered heterocyclyl ring containing 1 or 2 or 3 heteroatoms

are excellent negative modulators of the $GABA_A5R$ (i.e. negative modulator of the $GABA_A$ $\alpha$5 subunit) having improved properties with respect to $GABA_A5R$ binding properties, which translates in lower doses of the compounds for disease treatment and minimization of side effects. Furthermore, the compounds of the present invention have excellent CNS penetration with low efflux ratio from the brain compartment which is necessary for drugs with an intended action in the CNS and a high metabolic stability.

Therefore, one aspect of the invention refers to compounds according to formula (I), or salts thereof which are negative modulators of the $GABA_A5R$.

Another aspect of the invention refers to compounds according to formula (I), or pharmaceutically acceptable salts thereof which are negative modulators of the $GABA_A5R$ with high $GABA_A5R$ binding properties.

Another aspect of the invention refers to compounds according to formula (I), or pharmaceutically acceptable

salts thereof which are negative modulators of the $GABA_A5R$ with high $GABA_A5R$ binding properties and excellent CNS penetration with low efflux ratio from the brain compartment and high metabolic stability.

Yet a further aspect the invention relates to pharmaceutical compositions, containing at least one compound according to formula (I), or pharmaceutically acceptable salts thereof, optionally together with one or more inert carriers and/or diluents.

An additional aspect of the invention relates to processes of manufacture of the compounds of the present invention.

In particular the present invention relates to compounds according to formula (I), or pharmaceutically acceptable salts thereof or pharmaceutical compositions comprising compounds according to formula (I), or pharmaceutically acceptable salts thereof for the use in the prevention and/or treatment of diseases or conditions which can be influenced by negative modulation of the $GABA_A5R$ such as acute neurological disorders, chronic neurological disorders, cognitive disorders, Alzheimer's disease, memory deficits, schizophrenia, positive, negative and/or cognitive symptoms associated with schizophrenia, cognitive impairment associated with schizophrenia, bipolar disorders, autism, Down syndrome, neurofibromatosis type I, post operative cognitive decline, sleep disorders, disorders of circadian rhythms, amyotrophic lateral sclerosis, dementia caused by AIDS, psychotic disorders, substance-induced psychotic disorder, anxiety disorders, generalized anxiety disorder, panic disorder, delusional disorder, obsessive compulsive disorders, acute stress disorder, drug addictions, movement disorders, Parkinson's disease, restless leg syndrome, cognition deficiency disorders, multi-infarct dementia, mood disorders, depression, major depressive disorder, neuropsychiatric conditions, psychosis, attention-deficit hyperactivity disorder, neuropathic pain, stroke, attentional disorders, eating disorders, anorexia, anorexia nervosa, cachexia, weight loss, muscle atrophy, pain conditions, chronic pain, nociceptive pain, post-operative pain, osteoarthritis pain, rheumatoid arthritis pain, musculoskeletal pain, burn pain, ocular pain, pain due to inflammation, pain due to bone fracture, hyperalgesia, neuropathic pain, herpes-related pain, HIV-related neuropathic pain, traumatic nerve injury, recovery after traumatic brain injury, post-stroke pain, post-ischemia pain, fibromyalgia, chronic headache, migraine, tension-type headache, diabetic neuropathic pain, phantom limb pain, visceral pain and cutaneous pain.

Other results or conclusions of the present invention become apparent to the skilled man directly from the foregoing and following remarks.

## Detailed description

The present invention relates to compounds of general formula (I)

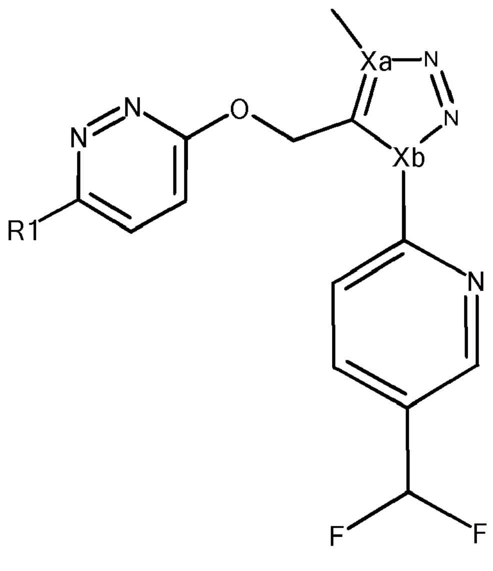

(I),

or a salt thereof, wherein

**Xa and Xb** are different from each other and represent C or N and

**R1** is a substituted phenyl or a 5- or 6- membered substituted heterocyclyl ring containing 1 or 2 or 3 heteroatoms.

In particular either **Xa** or **Xb** is C.

**R1** is preferably selected from the group consisting of

- phenyl substituted with carbamoyl;
- unsubstituted 2-pyridon or 2-pyridon substituted with halogen such as fluorine or substituted on the nitrogen with methyl or ethyl;
- 2, 3 or 4-pyridyl substituted with cyano- (NC-) or thiomethylate-, amino-, methyl-amino-, methylsulfonyl- or halogen;
- unsubstituted pyrimidinyl- or pyrimidinyl- substituted with $C_{1-6}$-alkyl-, amino-, - hydroxymethyl- or pyrazinyl- or pyridazinyl;
- pyrrolyl- substituted with $C_{1-6}$-alkyl- or NC-$CH_2$-$CH_2$- and pyrazolyl- substituted with $C_{1-6}$-alkyl-, $C_{3-5}$-cycloalkyl-, NC-$CH_2$-$CH_2$-, amino-, methyl-amino-, or halogen;
- imidazoly- substituted with $C_{1-6}$-alkyl-, carbamoyl-, NC-$CH_2$-$CH_2$-, amino- or - methylamino-;
- unsubstituted triazolyl- or triazolyl- substituted with $C_{1-3}$-alkyl- such as methyl;
- oxazolyl- substituted with methyl and thiophenyl- substituted with NC-$CH_2$-$CH_2$-,

Unless otherwise stated, the groups, residues, and substituents, particularly R1 are defined as above and hereinafter. If residues, substituents, or groups occur several times in a compound they may have the same or different meanings. Some preferred meanings of groups and substituents of the compounds according to the invention will be given hereinafter.

In a further embodiment of the invention **R1** is selected from the group consisting of

- phenyl substituted with carbamoyl such as

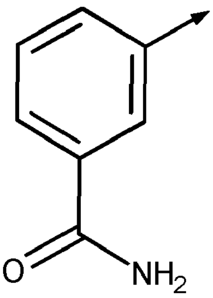

6

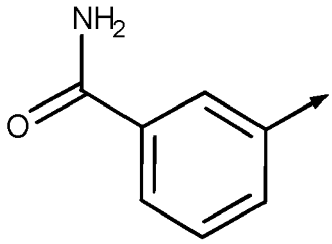

28

and

- unsubstituted 2-pyridon or 2-pyridon substituted with halogen such as fluorine or substituted on the nitrogen with methyl or ethyl such as

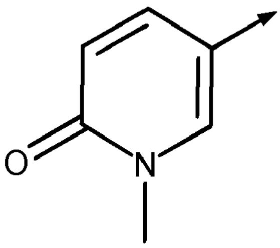

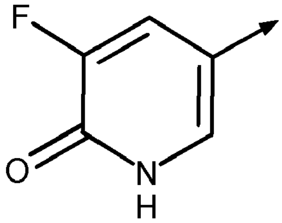

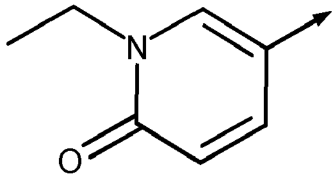

In a further embodiment of the invention **R1** is

- 2, 3 or 4-pyridyl substituted with NC- or -thiomethylate-, amino-, methyl-amino- or - methylsulfonyl or halogen such as

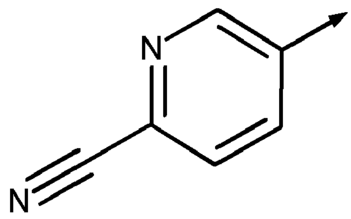
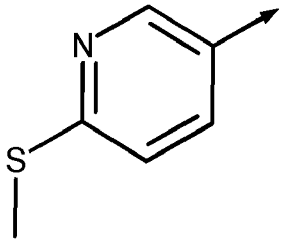
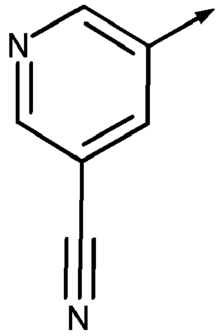

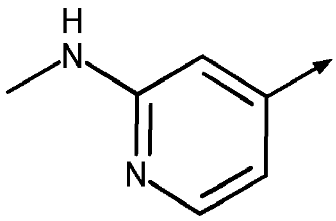
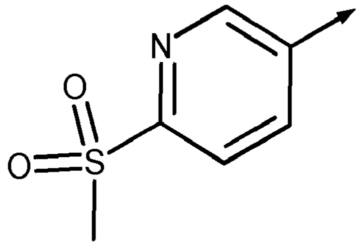

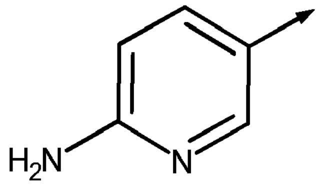

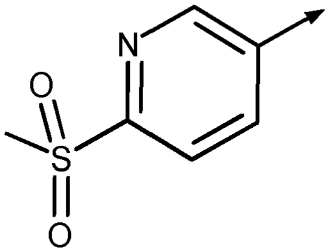
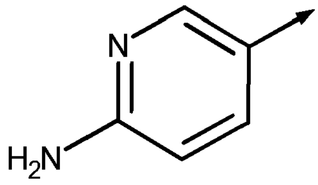
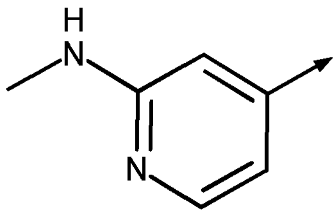

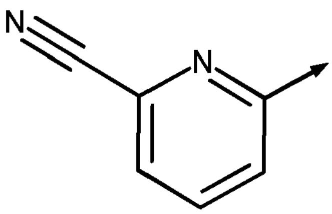
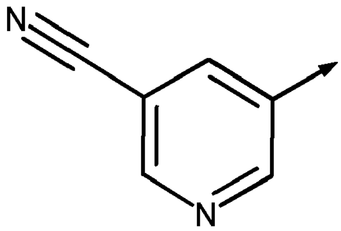
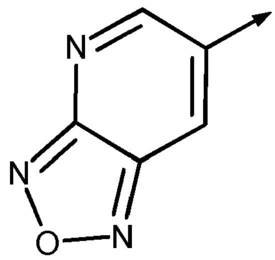

In a further embodiment of the invention **R1** is selected from the group consisting of compound of claim 1 or a salt thereof, wherein **R1** is

- unsubstituted pyrimidinyl- or pyrimidinyl- substituted with $C_{1-6}$-alkyl-, amino-, - hydroxymethyl- or pyrazinyl- or pyridazinyl such as

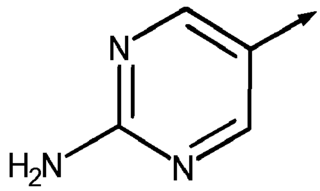 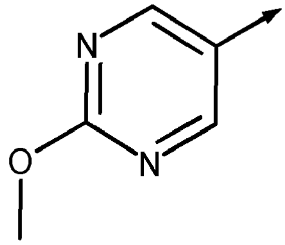 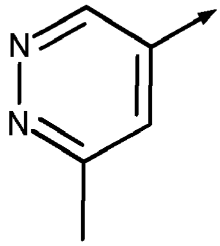

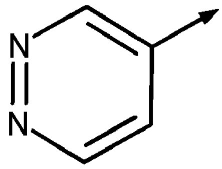 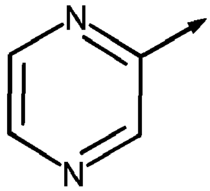

In a further embodiment of the invention **R1** is

- pyrrolyl- substituted with $C_{1-6}$-alkyl- or NC-$CH_2$-$CH_2$- and pyrazolyl- substituted with $C_{1-6}$-alkyl-, $C_{3-5}$-cycloalkyl-, NC-$CH_2$-$CH_2$-, -amino, -methyl-amino, or -halogen such as

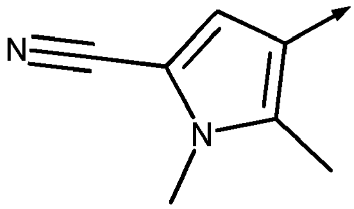

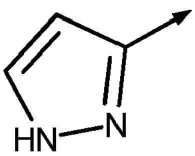 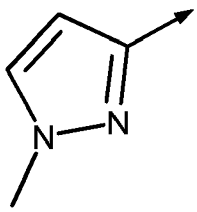 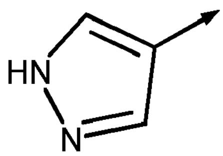 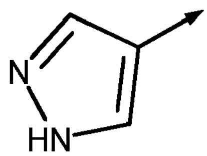

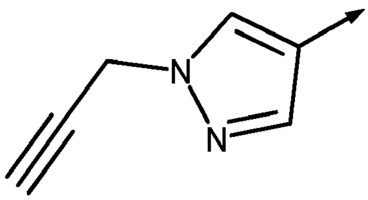 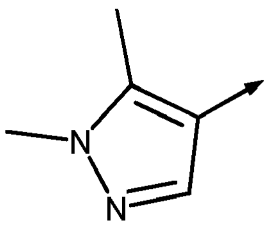 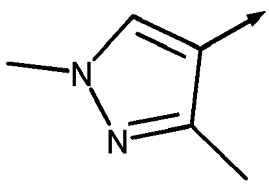

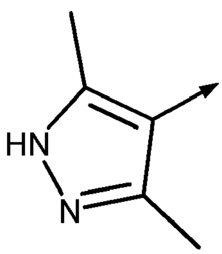 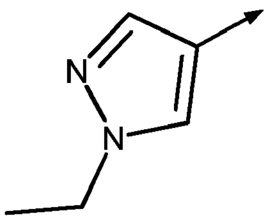 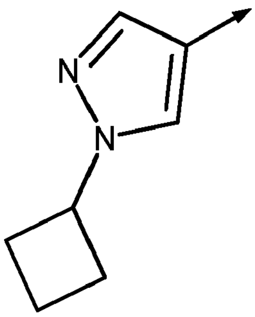

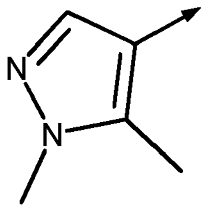
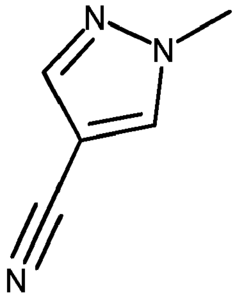
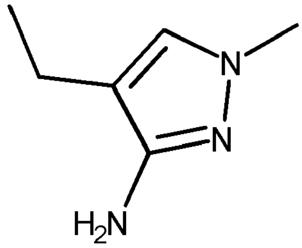
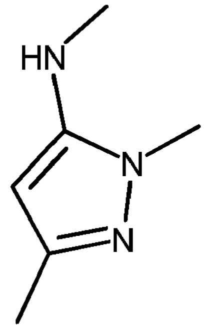
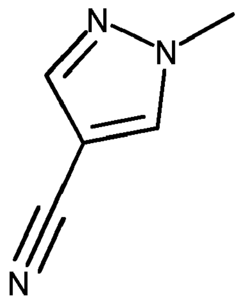
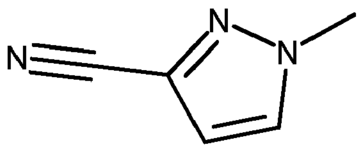
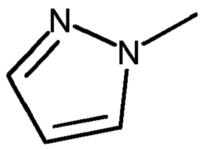
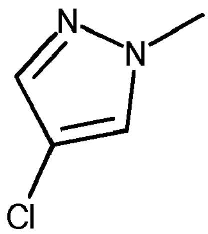
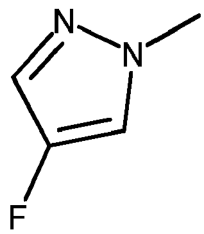

In a further embodiment of the invention **R1** is

- imidazoly- substituted with $C_{1-6}$-alkyl-, carbamoyl, NC-$CH_2$-$CH_2$-, amino- or - methylamino- such as

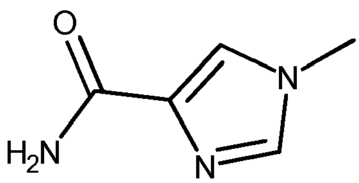
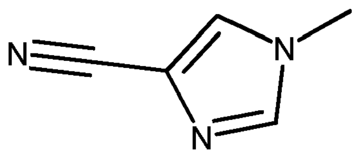
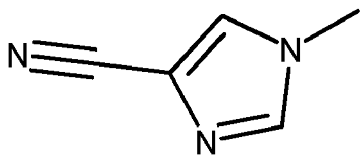

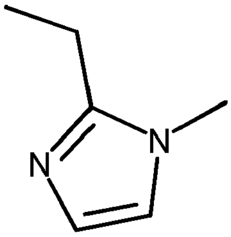 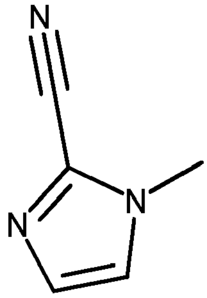 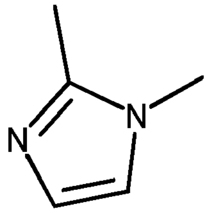

In a further embodiment of the invention **R1** is

- unsubstituted triazolyl- or triazolyl- substituted with $C_{1-3}$-alkyl- such as

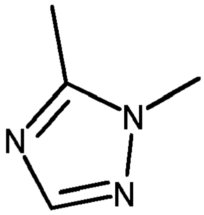 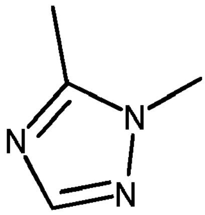 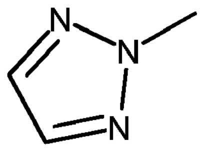 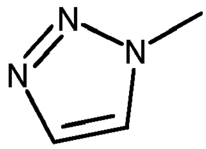

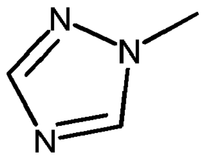

In yet a further embodiment of the invention **R1** is

- oxazolyl- substituted with methyl or thiophenyl- substituted with NC-$CH_2$-$CH_2$- such as

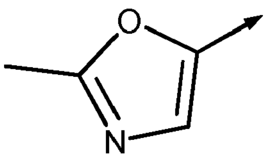 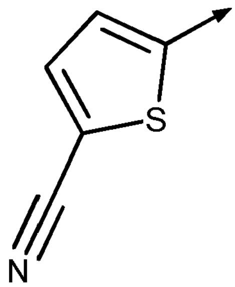

Further preferred are the following compounds listed in Table 1:

**Table 1:** Compounds (C#) 1 to 56

| C# | Structure |
| --- | --- |
| **1** | 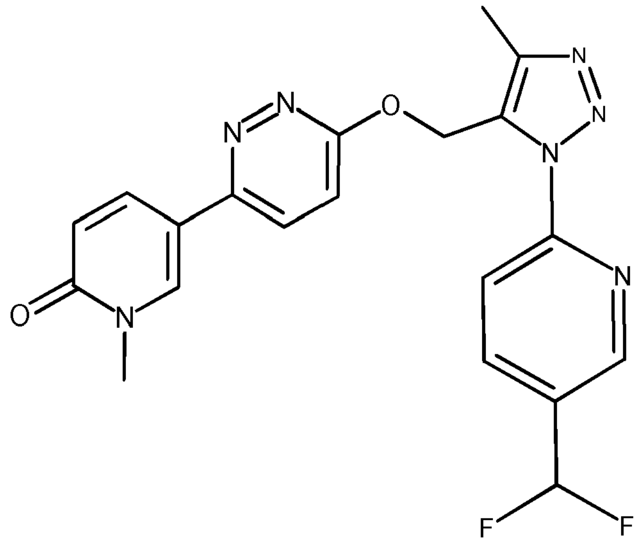 |
| **2** | 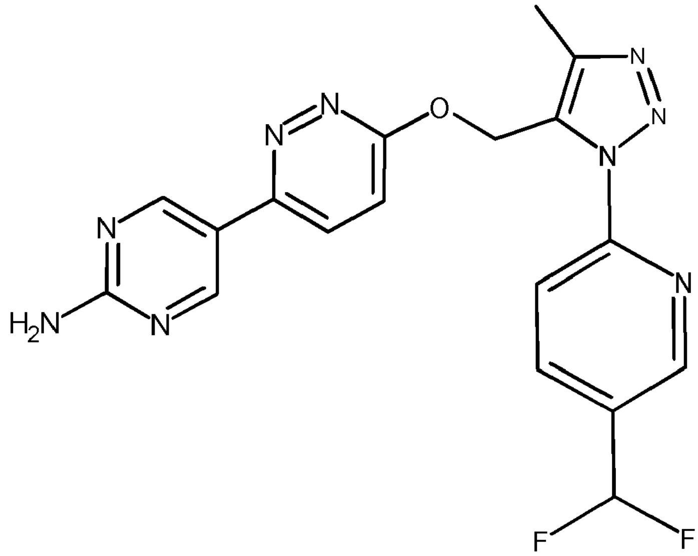 |
| **3** | 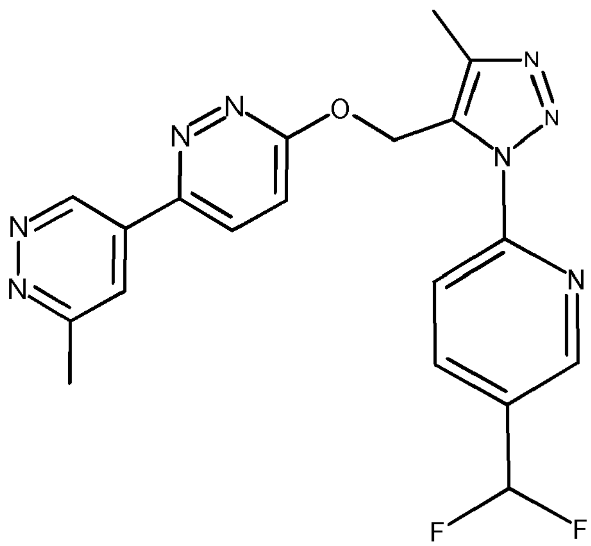 |

(continued)

| C# | Structure |
|---|---|
| **4** | 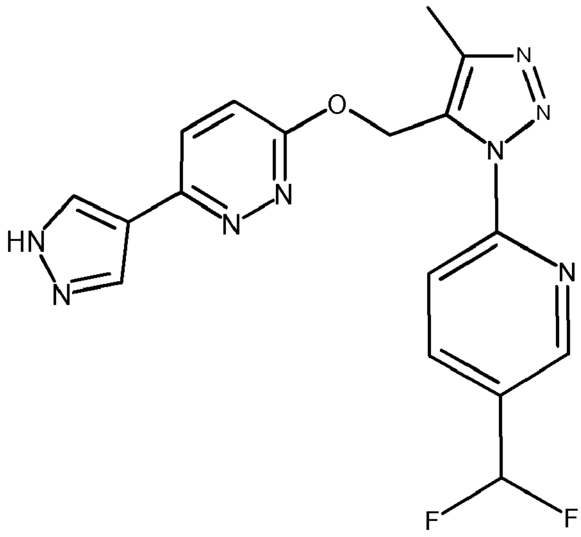 |
| **5** | 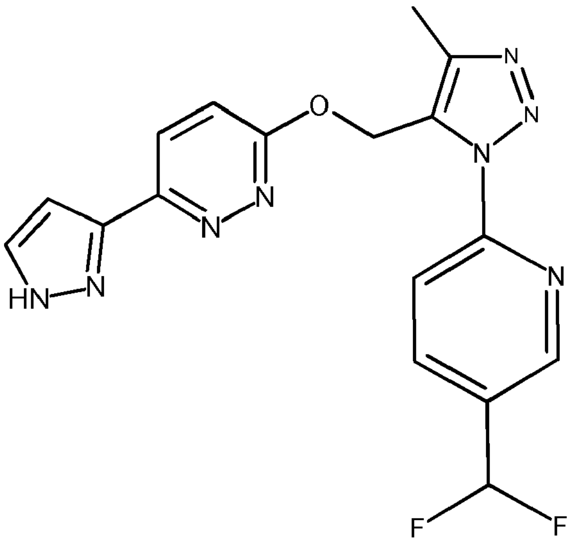 |
| **6** | 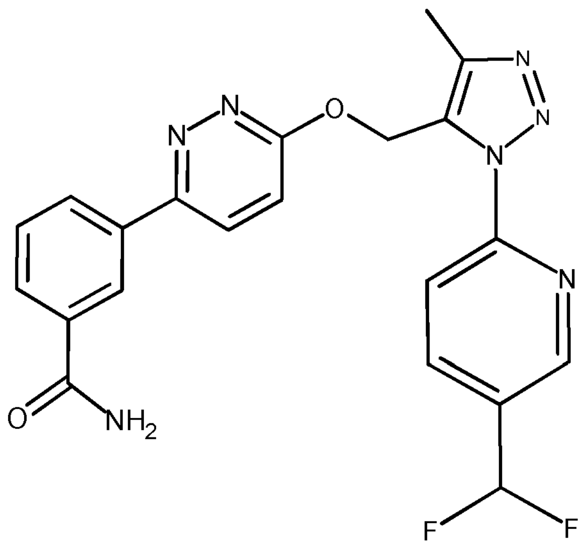 |

(continued)

| C# | Structure |
|---|---|
| **7** | 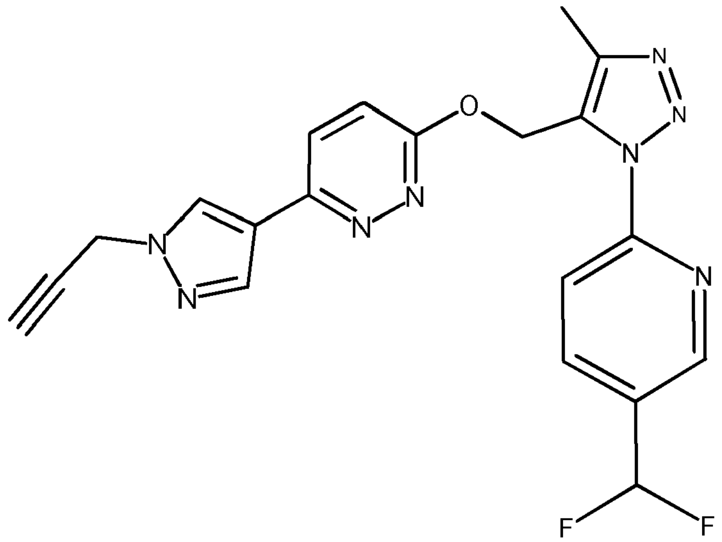 |
| **8** | 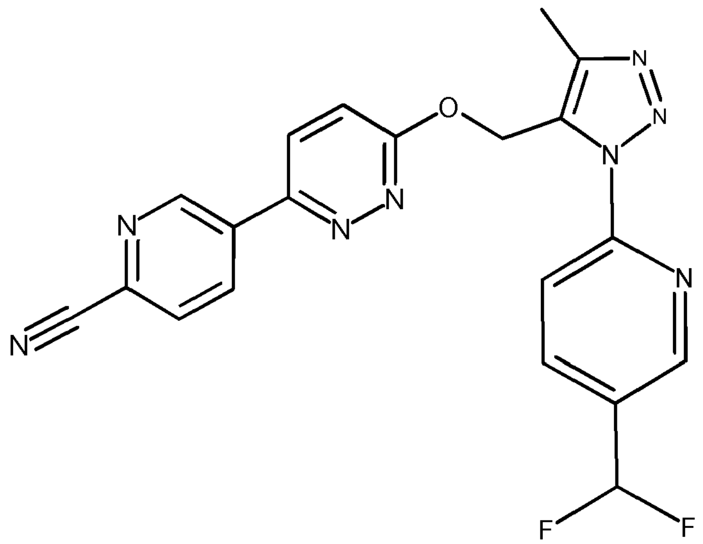 |
| **9** | 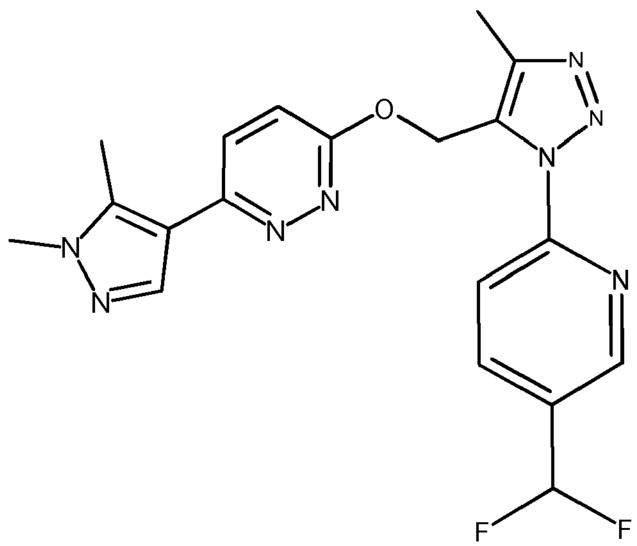 |

(continued)

| C# | Structure |
|---|---|
| **10** | 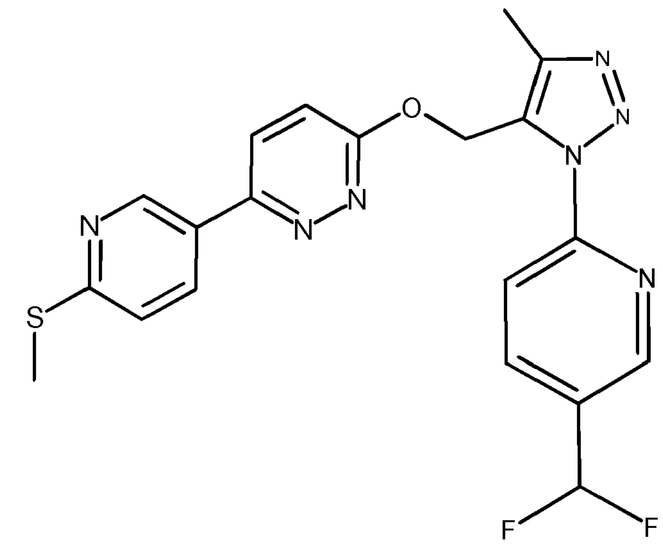 |
| **11** | 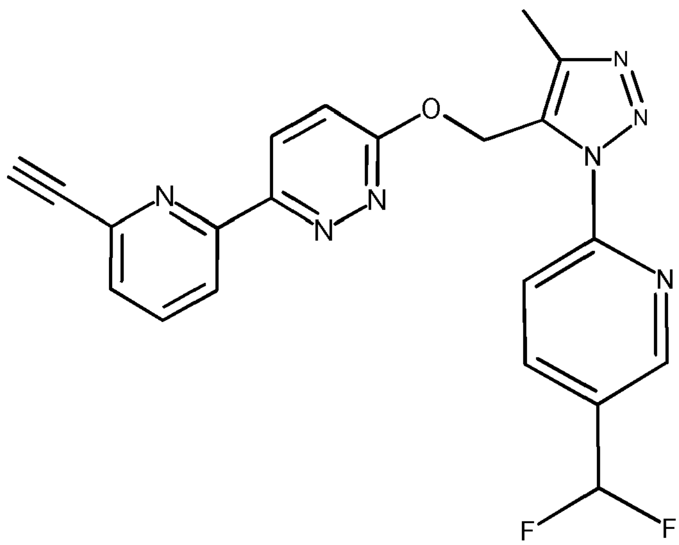 |
| **12** | 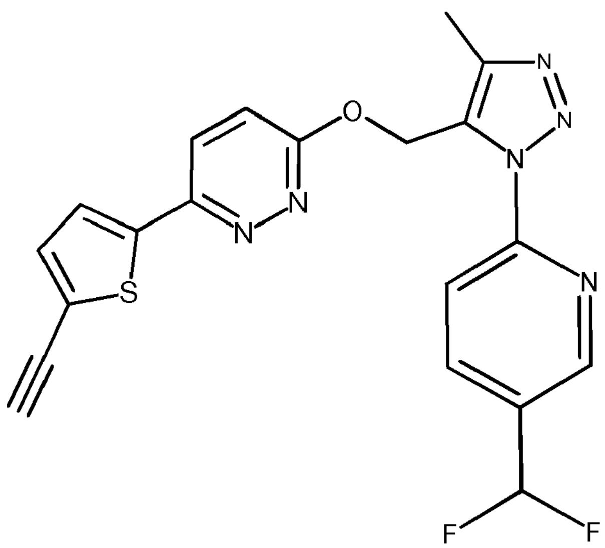 |

(continued)

| C# | Structure |
|---|---|
| **13** | 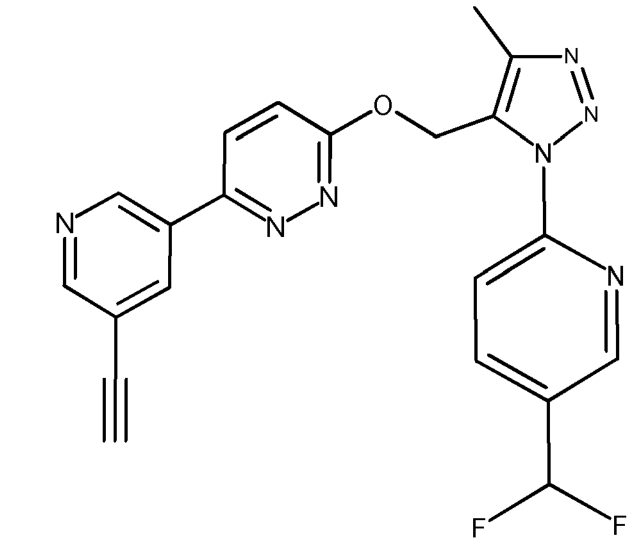 |
| **14** | 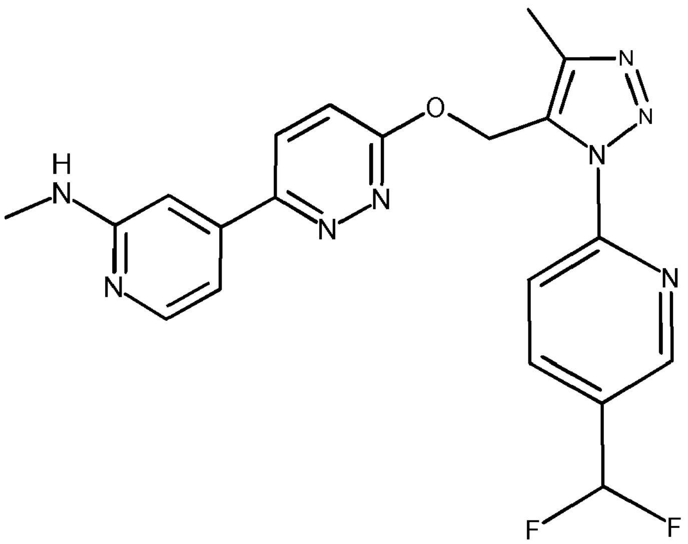 |
| **15** | 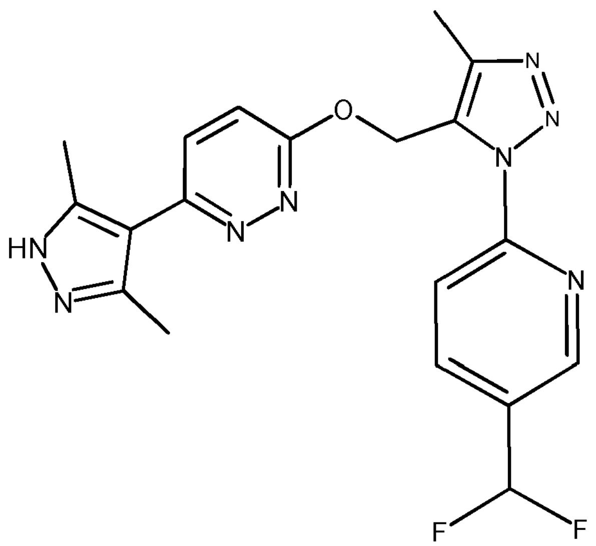 |

(continued)

| C# | Structure |
| --- | --- |
| **16** | 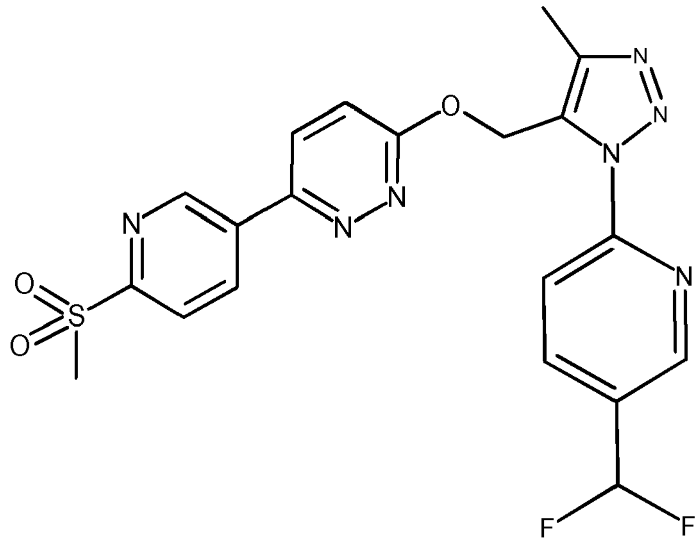 |
| **17** | 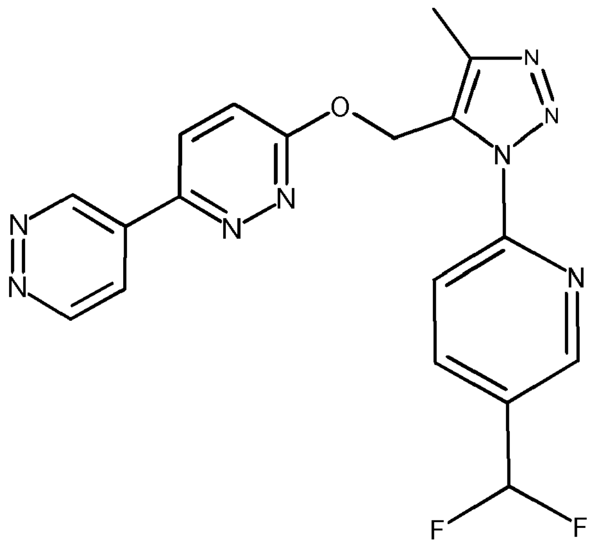 |
| **18** | 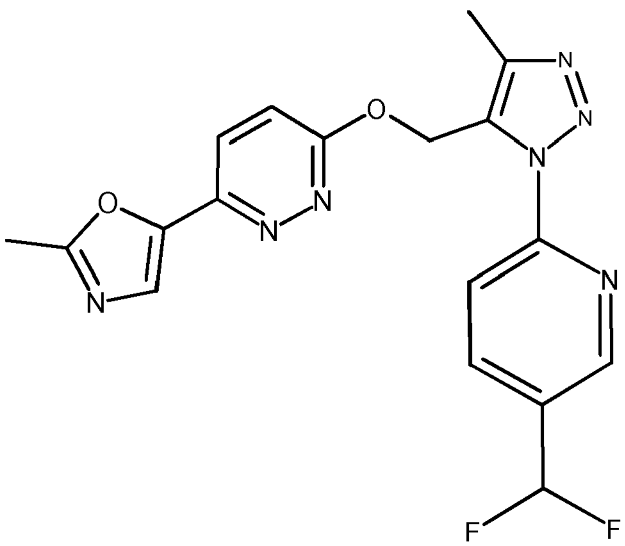 |

(continued)

| C# | Structure |
|---|---|
| **19** | 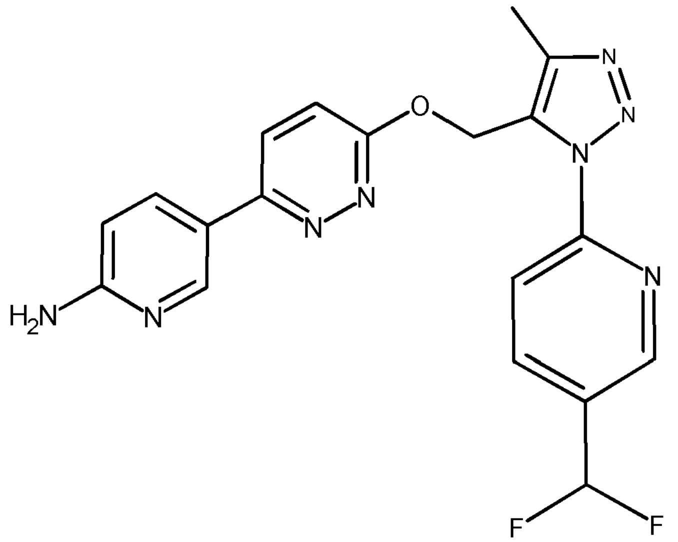 |
| **20** | 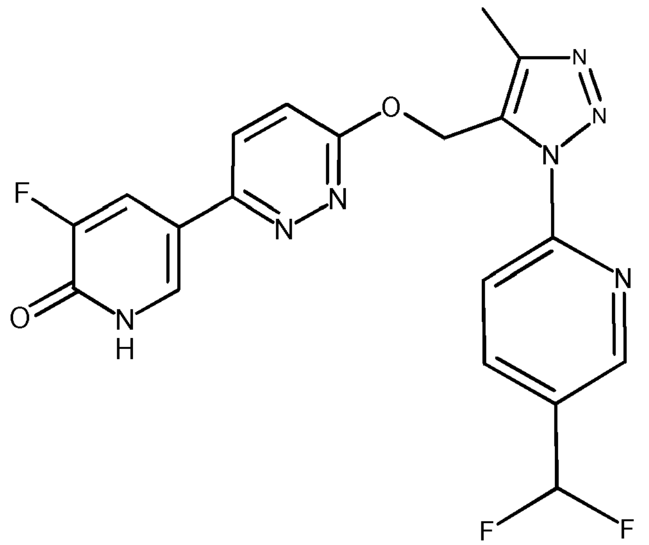 |
| **21** | 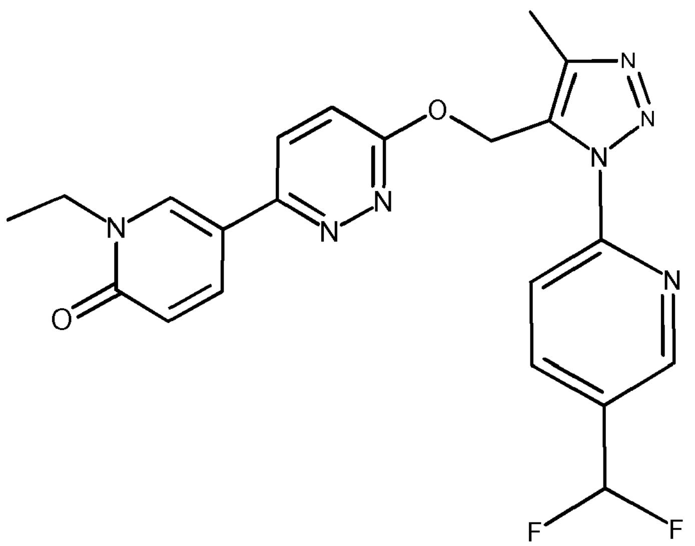 |

(continued)

| C# | Structure |
|---|---|
| **22** | 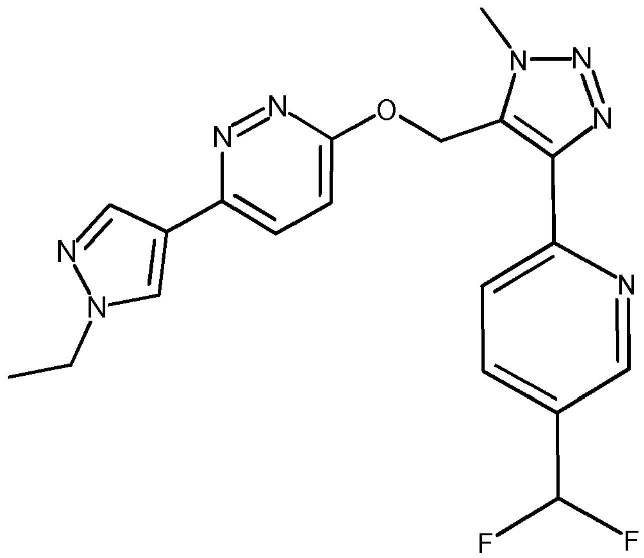 |
| **23** | 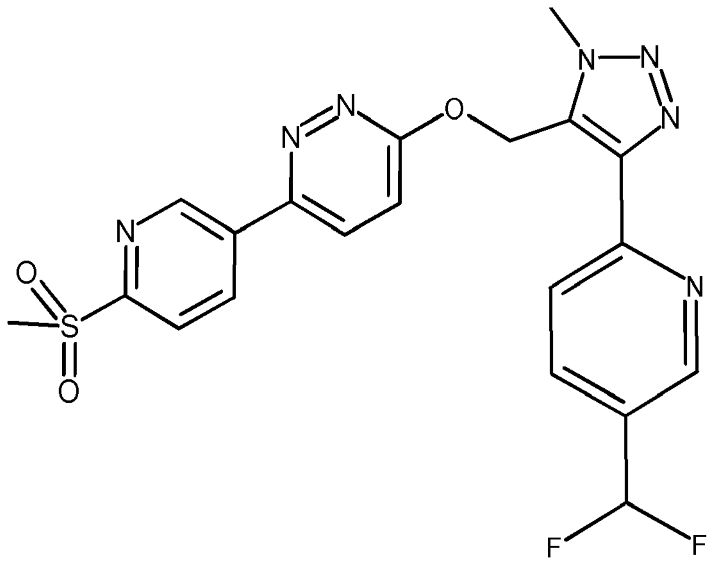 |

(continued)

| C# | Structure |
|---|---|
| **24** | 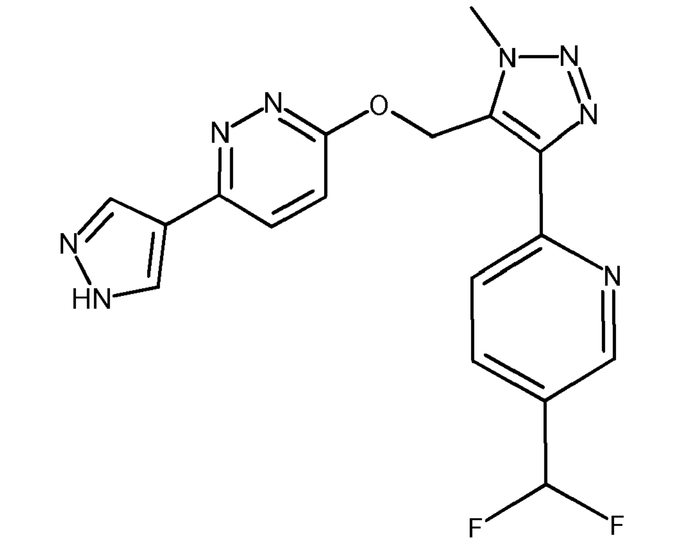 |
| **25** | 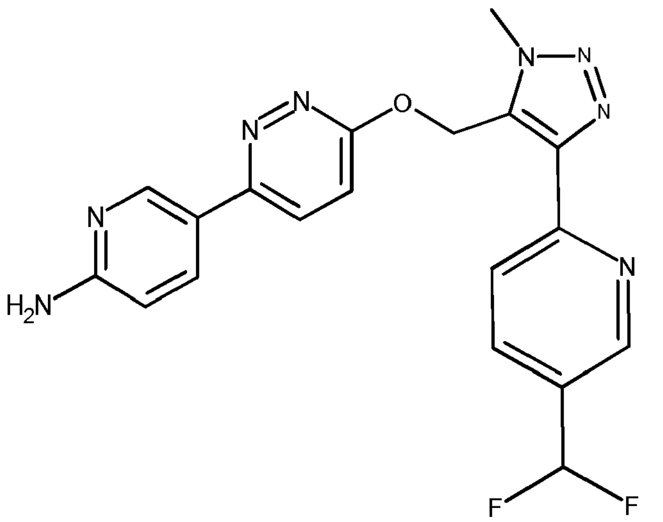 |
| **26** | 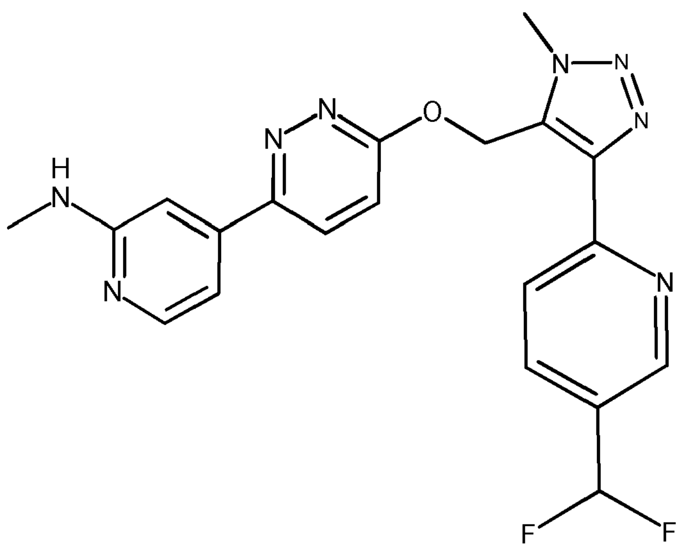 |

(continued)

| C# | Structure |
|---|---|
| **27** | 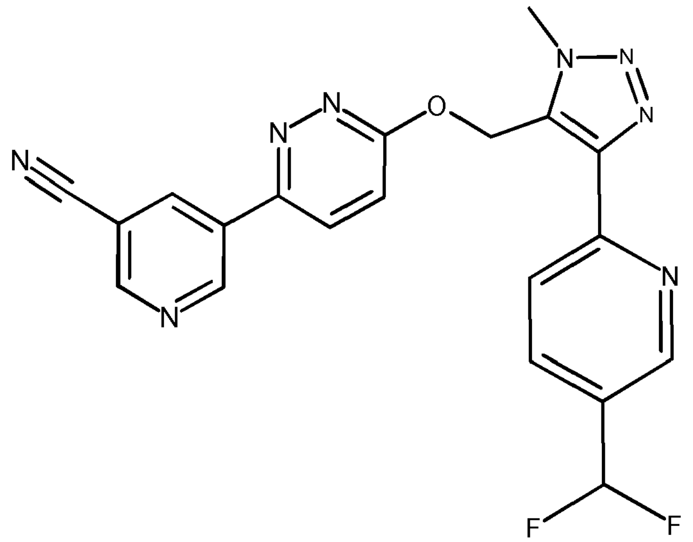 |
| **28** | 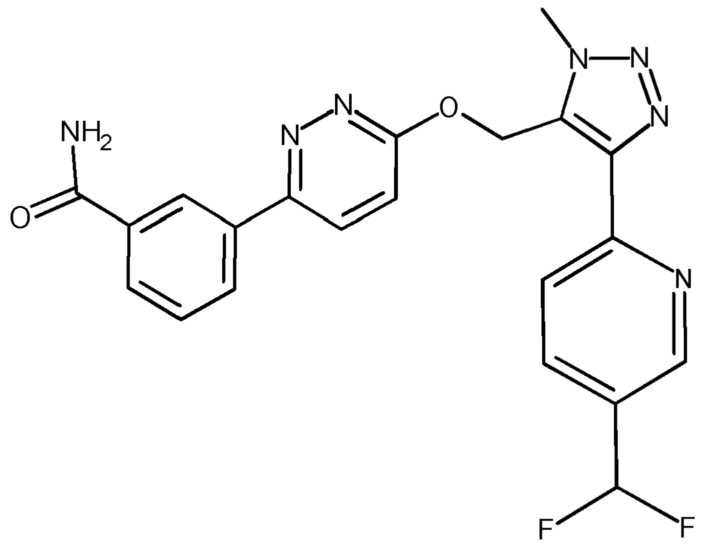 |
| **29** | 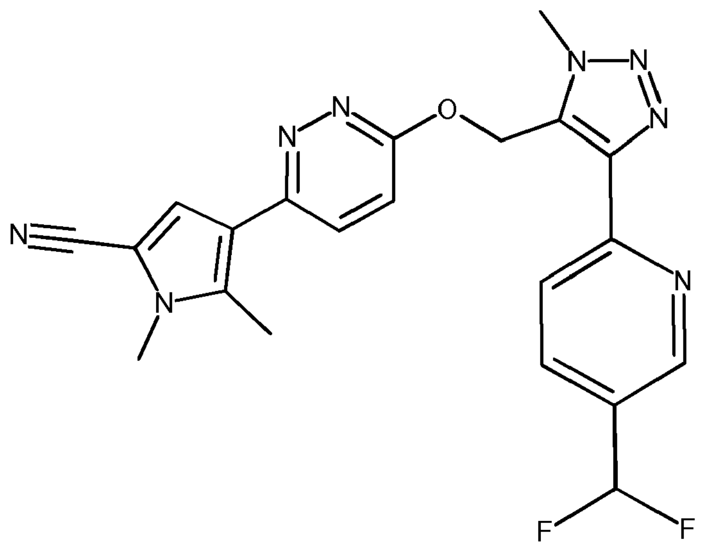 |

(continued)

| C# | Structure |
|---|---|
| **30** | 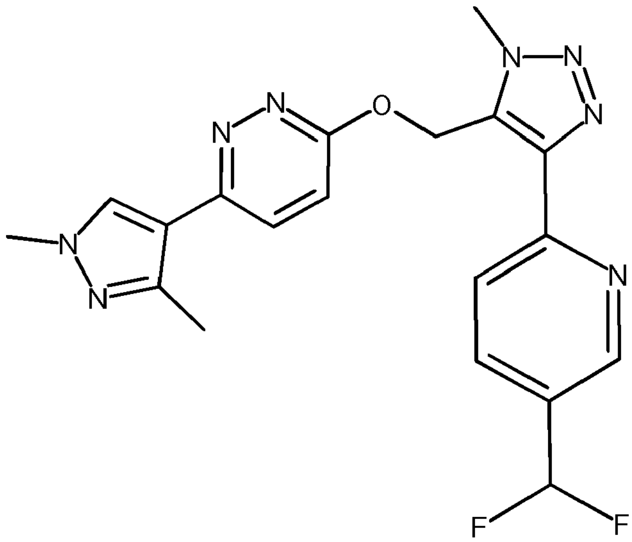 |
| **31** | 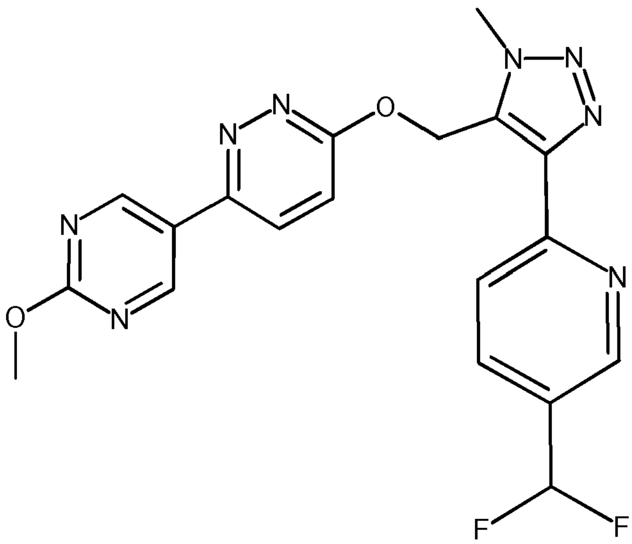 |
| **32** | 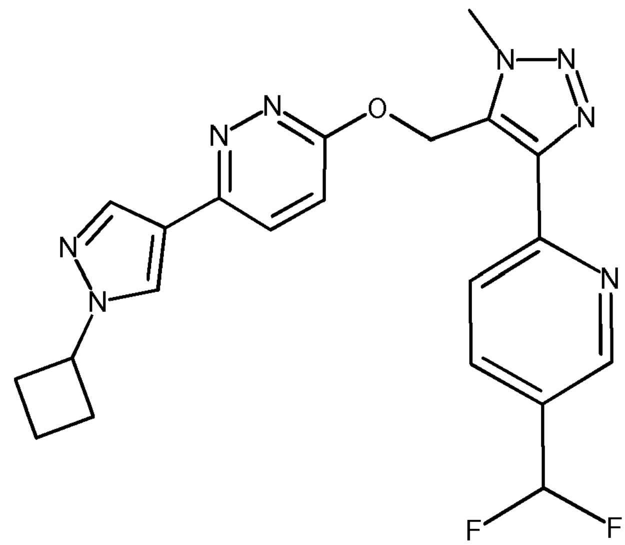 |

(continued)

| C# | Structure |
|---|---|
| **33** | 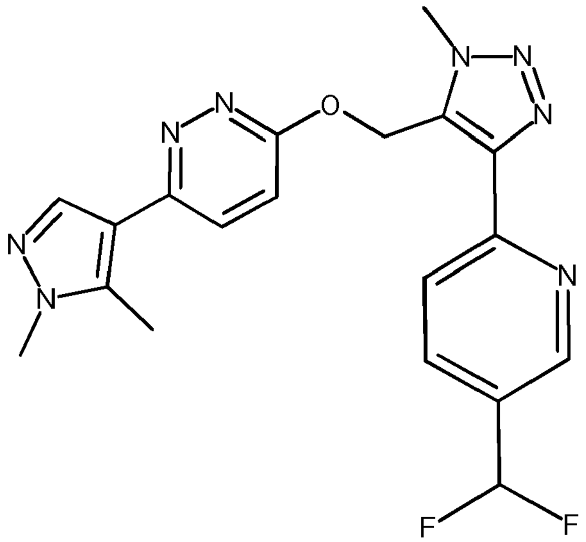 |
| **34** | 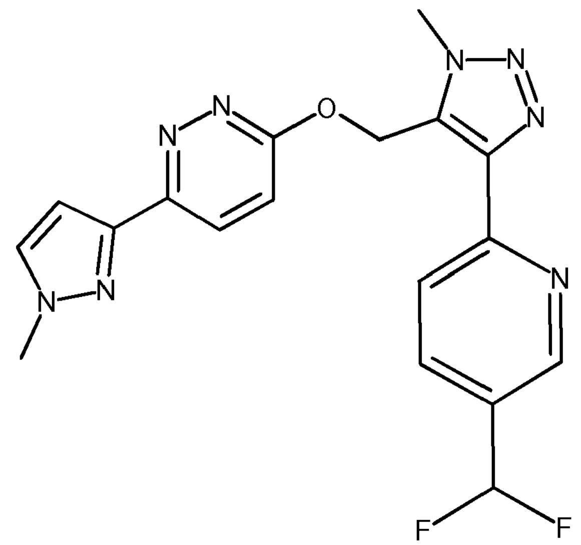 |
| **35** | 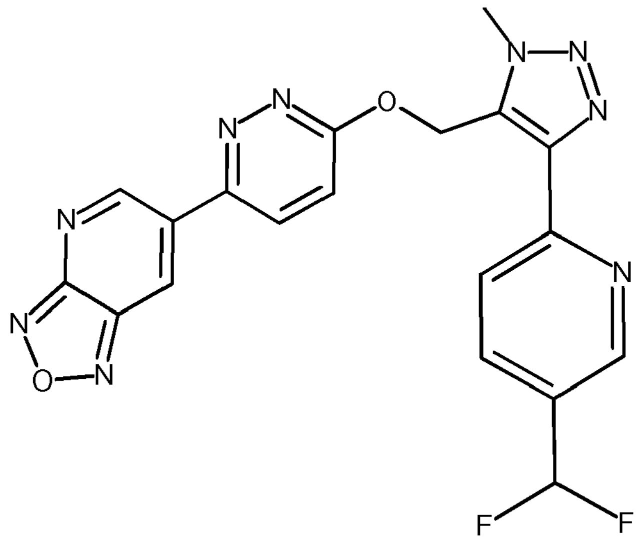 |

(continued)

| C# | Structure |
|---|---|
| **36** | 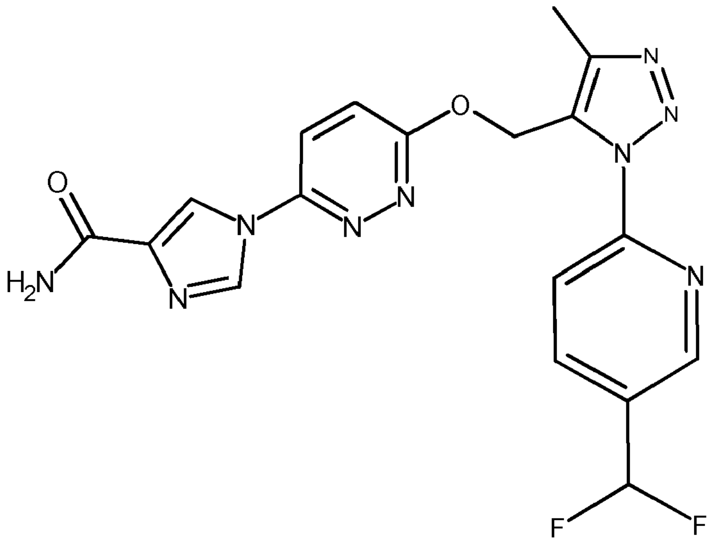 |
| **37** | 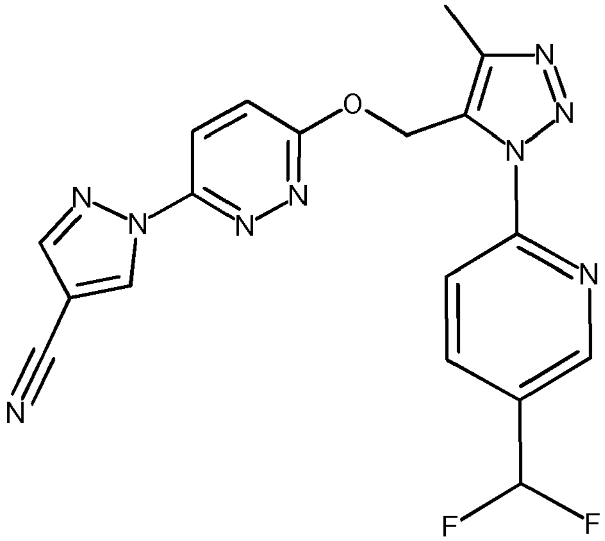 |
| **38** | 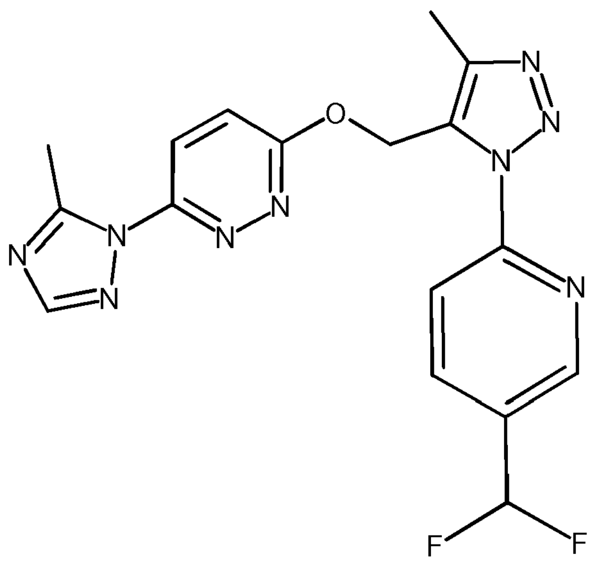 |

(continued)

| C# | Structure |
| --- | --- |
| **39** | 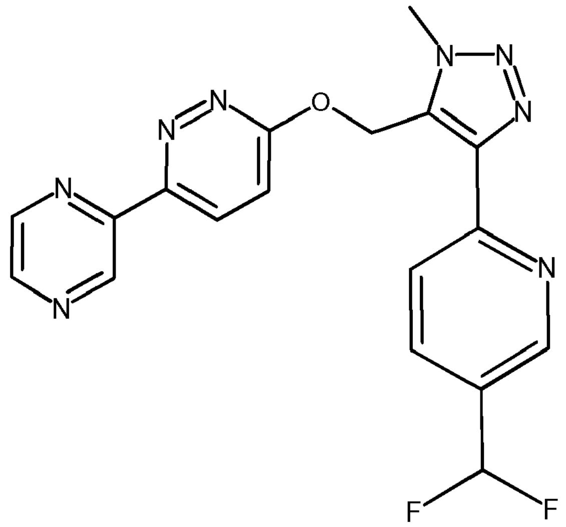 |
| **40** | 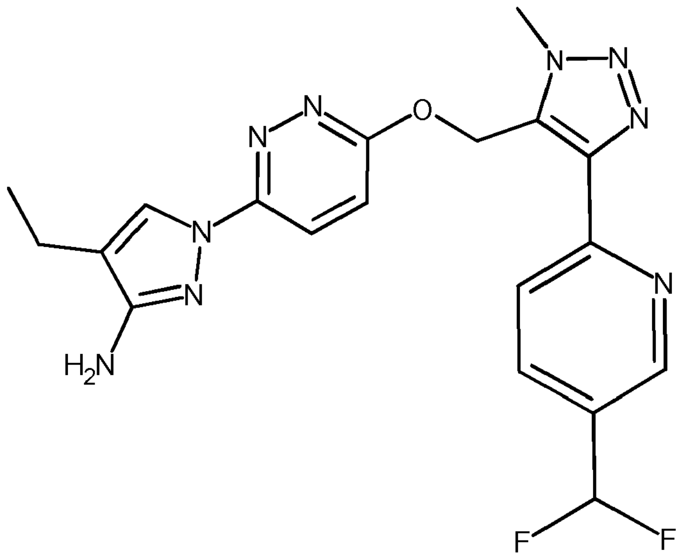 |
| **41** | 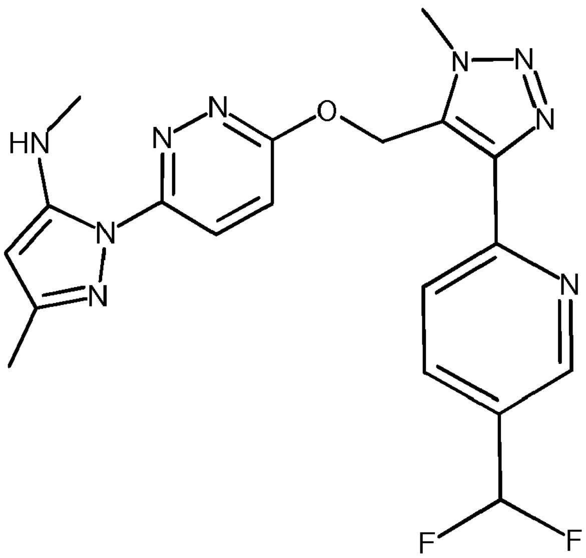 |

(continued)

| C# | Structure |
|---|---|
| **42** | 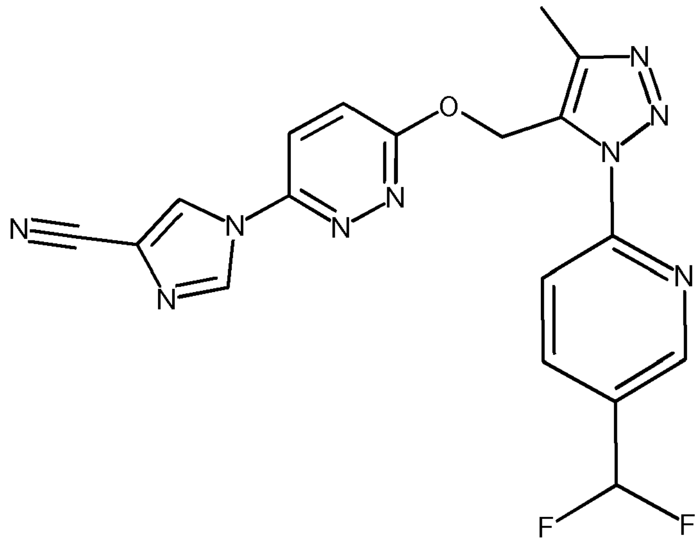 |
| **43** | 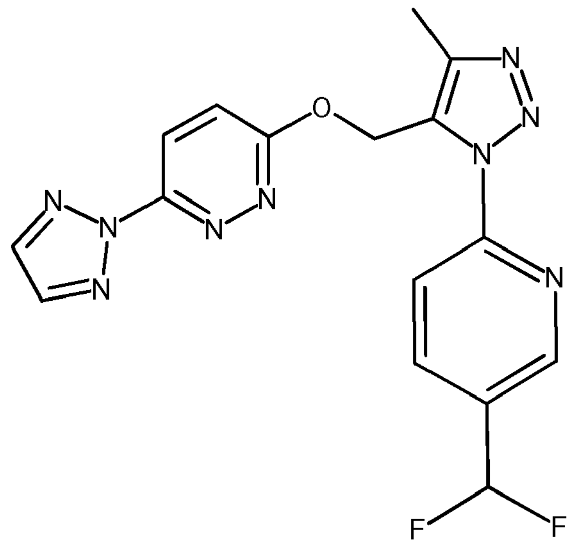 |
| **44** | 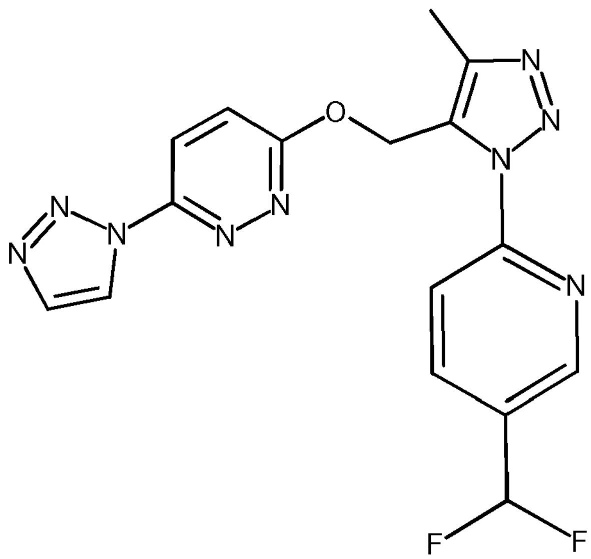 |

(continued)

| C# | Structure |
|---|---|
| **45** | 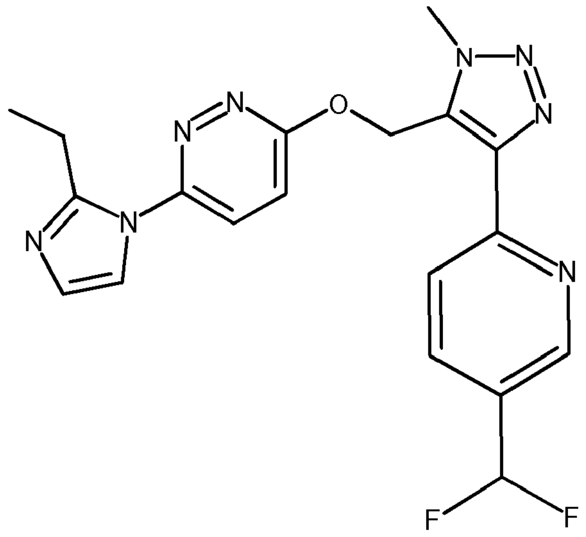 |
| **46** | 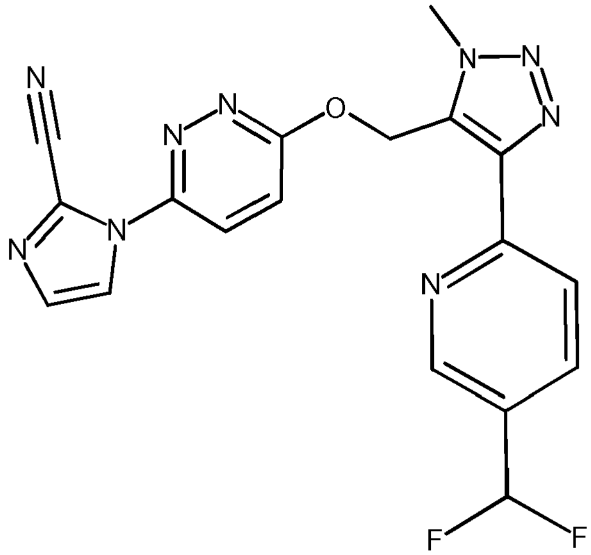 |
| **47** | 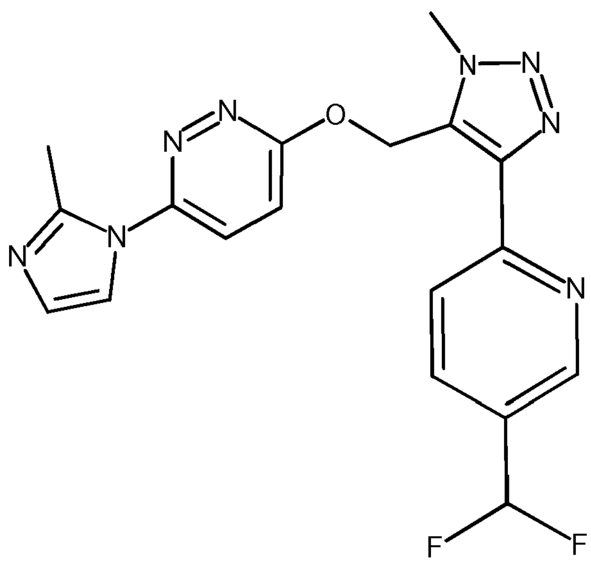 |

(continued)

| C# | Structure |
|---|---|
| **48** | 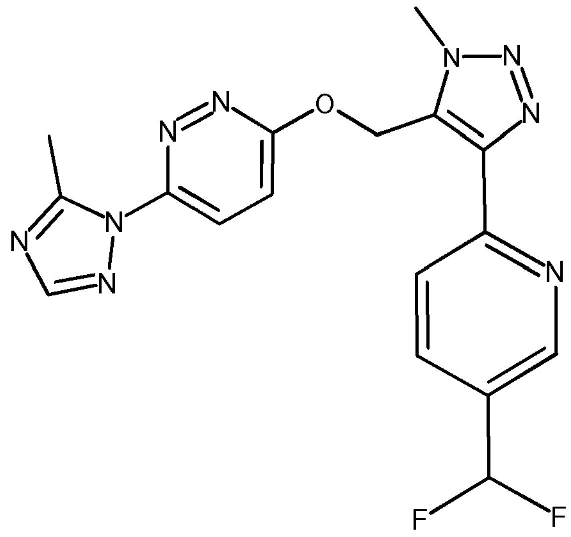 |
| **49** | 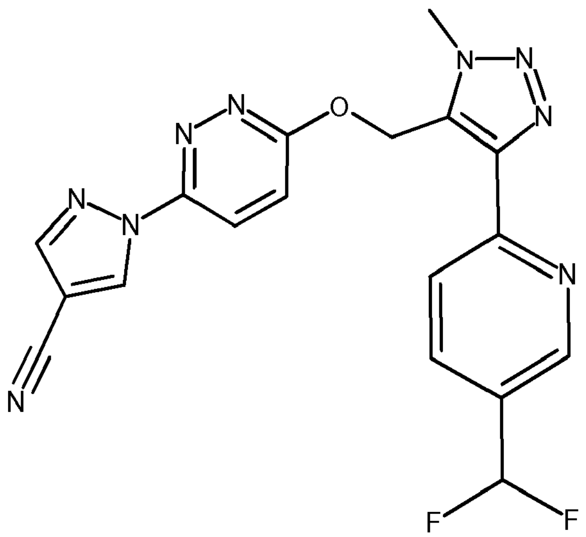 |
| **50** | 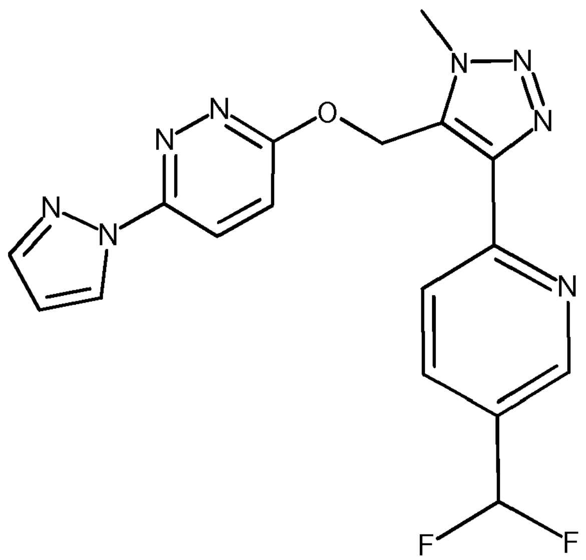 |

(continued)

| C# | Structure |
|---|---|
| **51** | 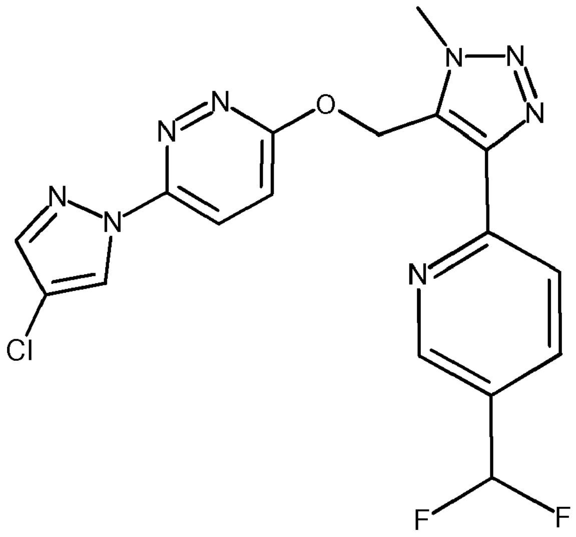 |
| **52** | 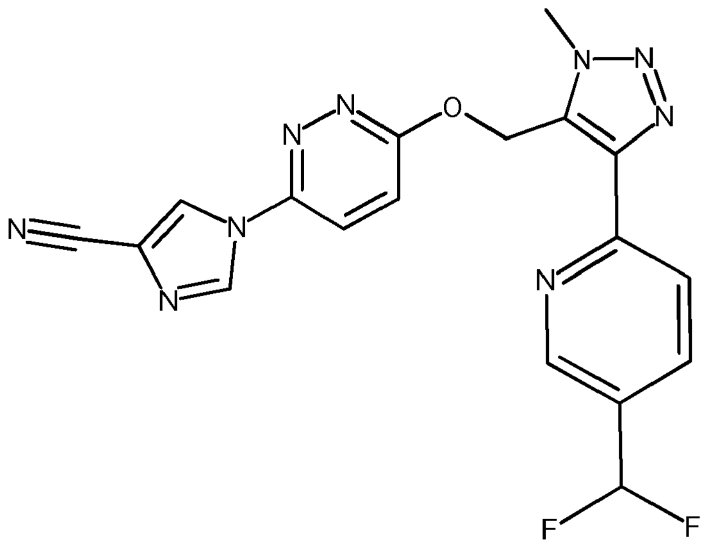 |
| **53** | 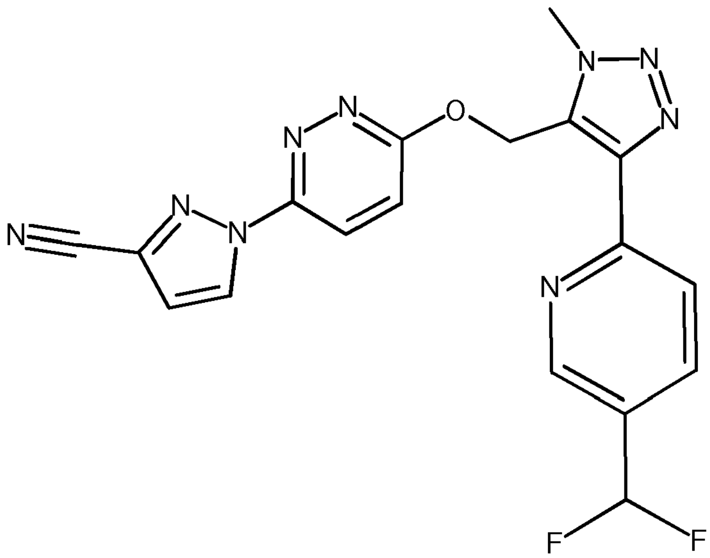 |

(continued)

| C# | Structure |
|---|---|
| **54** | 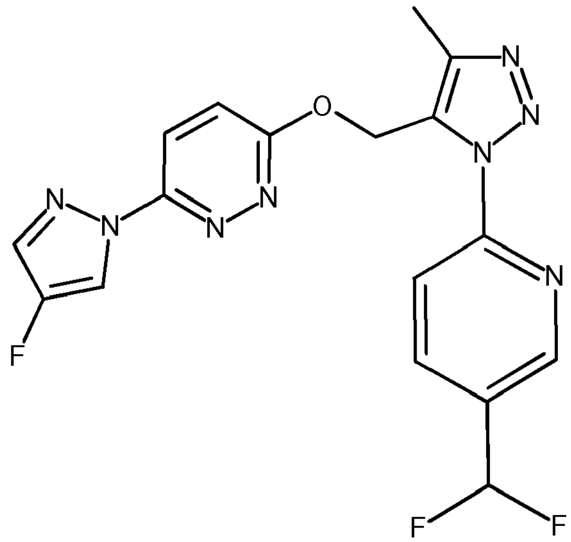 |
| **55** | 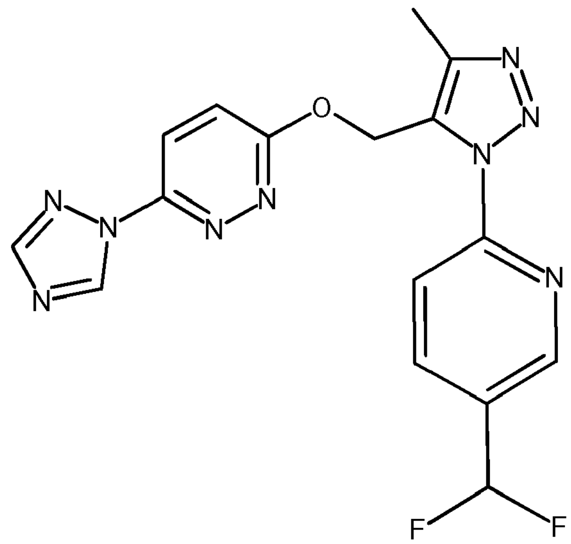 |
| **56** | 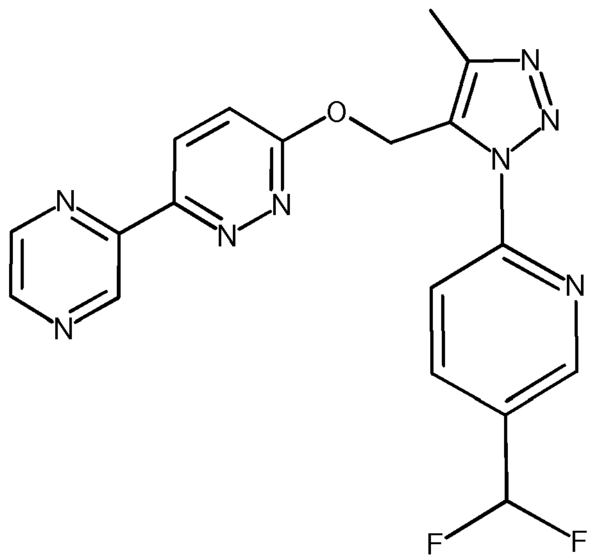 |

Some terms used above and hereinafter to describe the compounds according to the invention will now be defined more closely.

Terms not specifically defined herein should be given the meanings that would be given to them by one of skill in the art in light of the disclosure and the context. As used in the specification, however, unless specified to the contrary, the following terms have the meaning indicated and the following conventions are adhered to.

In the groups, radicals, or moieties defined below, the number of carbon atoms is often specified preceding the group, for example, $C_{1-6}$-alkyl means an alkyl group or radical having 1 to 6 carbon atoms. In general in groups like HO-, $H_2N$-, (O)S-, $(O)_2S$-, NC- (cyano), HOOC-, $F_3C$- or the like, the skilled artisan can see the radical attachment point(s) to the molecule from the free valences of the group itself. For combined groups comprising two or more subgroups, the last named subgroup is the radical attachment point, for example, the substituent "aryl-$C_{1-3}$-alkyl-" means an aryl group which is bound to a $C_{1-3}$-alkyl-group, the latter of which is bound to the core or to the group to which the substituent is attached.

In general, the attachment site of a given residue to another group shall be variable, i.e. any capable atom, bearing hydrogens to be replaced, within this residue may be the linking spot to the group being attached, unless otherwise indicated.

In case a compound of the present invention is depicted in form of a chemical name and as a formula in case of any discrepancy the formula shall prevail.

Unless specifically indicated, throughout the specification and the appended claims, a given chemical formula or name shall encompass tautomers and all stereo, optical and geometrical isomers (e.g. enantiomers, diastereomers, E/Z isomers etc...) and racemates thereof as well as mixtures in different proportions of the separate enantiomers, mixtures of diastereomers, or mixtures of any of the foregoing forms where such isomers and enantiomers exist, as well as salts, including pharmaceutically acceptable salts thereof and solvates thereof such as for instance hydrates including solvates of the free compounds or solvates of a salt of the compound.

The phrase "pharmaceutically acceptable" or "physiologically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, and commensurate with a reasonable benefit/risk ratio.

As used herein, "pharmaceutically acceptable salt" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. For example, such salts include salts from benzenesulfonic acid, benzoic acid, citric acid, ethanesulfonic acid, fumaric acid, gentisic acid, hydrobromic acid, hydrochloric acid, maleic acid, malic acid, malonic acid, mandelic acid, methanesulfonic acid, 4-methyl-benzenesulfonic acid, phosphoric acid, salicylic acid, succinic acid, sulfuric acid and tartaric acid.

Further pharmaceutically acceptable salts can be formed with cations from ammonia, L-arginine, calcium, 2,2'-iminobisethanol, L-lysine, magnesium, *N*-methyl-D-glucamine, potassium, sodium and tris(hydroxymethyl)-aminomethane.

The pharmaceutically acceptable salts of the present invention can be synthesized from the parent compound which contains a basic or acidic moiety by conventional chemical methods. Generally, such salts can be prepared by reacting the free acid or base forms of these compounds with a sufficient amount of the appropriate base or acid in water or in an organic diluent like ether, ethyl acetate, ethanol, isopropanol, or acetonitrile, or a mixture thereof. Salts of other acids than those mentioned above which for example are useful for purifying or isolating the compounds of the present invention (e.g. trifluoro acetate salts,) also comprise a part of the invention.

The term "substituted" as used herein means that any one or more hydrogens on the designated atom is replaced with a selection from the indicated group, provided that the designated atom's viable valence number is not exceeded, and that the substitution results in a stable compound.

The term "partially unsaturated" as used herein means that in the designated group or moiety 1, 2, or more, preferably 1 or 2, double bonds are present. Preferably, as used herein, the term "partially unsaturated" does not cover fully unsaturated groups or moieties.

The term "halogen" generally denotes fluorine (F), chlorine (Cl), bromine (Br) and iodine (I).

The term "$C_{1-n}$-alkyl", wherein n is an integer from 2 to n, either alone or in combination with another radical denotes an acyclic, saturated, branched or linear hydrocarbon radical with 1 to n C atoms. For example the term $C_{1-5}$-alkyl embraces the radicals $H_3C$-, $H_3C-CH_2$-, $H_3C-CH_2-CH_2$-, $H_3C-CH(CH_3)$-, $H_3C-CH_2-CH_2-CH_2$-, $H_3C-CH_2-CH(CH_3)$-, $H_3C-CH(CH_3)-CH_2$-, $H_3C-C(CH_3)_2$-, $H_3C-CH_2-CH_2-CH_2-CH_2$-, $H_3C-CH_2-CH_2-CH(CH_3)$-, $H_3C-CH_2-CH(CH_3)-CH_2$-, $H_3C-CH(CH_3)-CH_2-CH_2$-, $H_3C-CH_2-C(CH3)_2$-, $H_3C-C(CH_3)_2-CH_2$-, $H_3C-CH(CH_3)-CH(CH_3)$- and $H_3C-CH_2-CH(CH_2CH_3)$-.

The term "$C_{3-n}$-cycloalkyl", wherein n is an integer from 4 to n, either alone or in combination with another radical denotes a cyclic, saturated, unbranched hydrocarbon radical with 3 to n C atoms. For example the term $C_{3-7}$-cy-

cloalkyl includes cyclopropyl, cyclobutyl, cyclopentyl, cyclohexyl and cycloheptyl.

Many of the terms given above may be used repeatedly in the definition of a formula or group and in each case have one of the meanings given above, independently of one another.

The compounds according to the invention may be obtained using methods of synthesis known in principle. Preferably, the compounds are obtained by the following methods according to the invention which are described in more detail hereinafter.

General chemical synthetic routes used for the compounds disclosed herein are

**Route 1**:

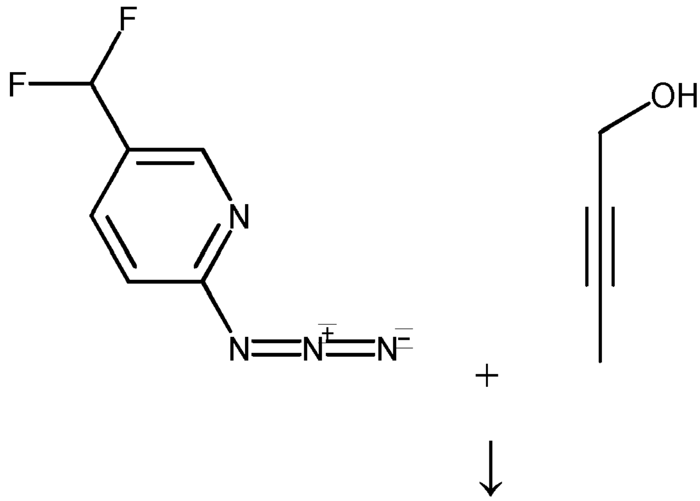

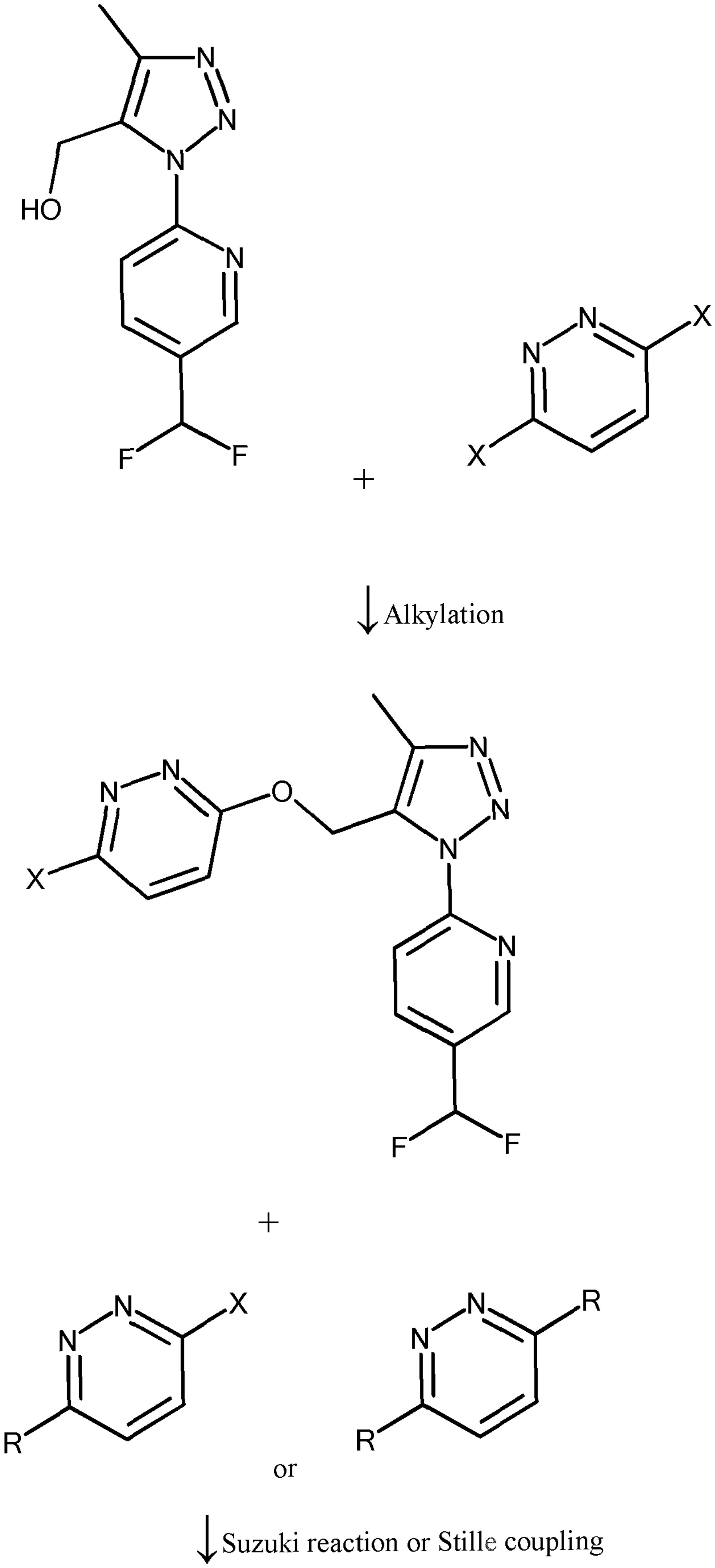
Alkylation
or
Suzuki reaction or Stille coupling

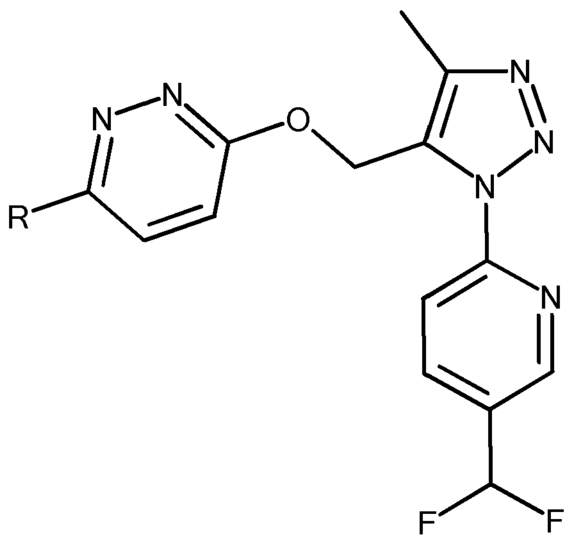

**Route 2:**

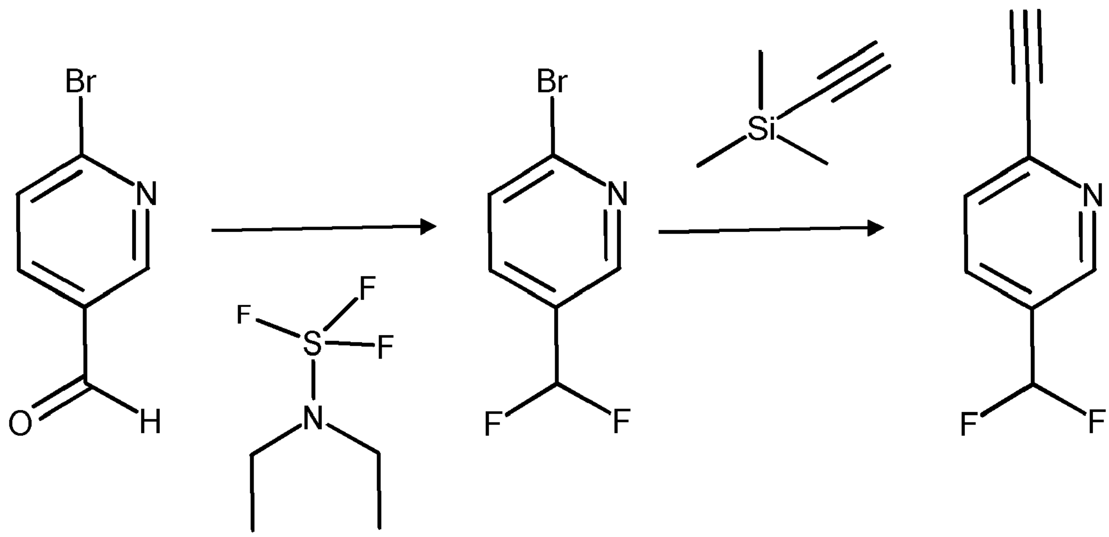

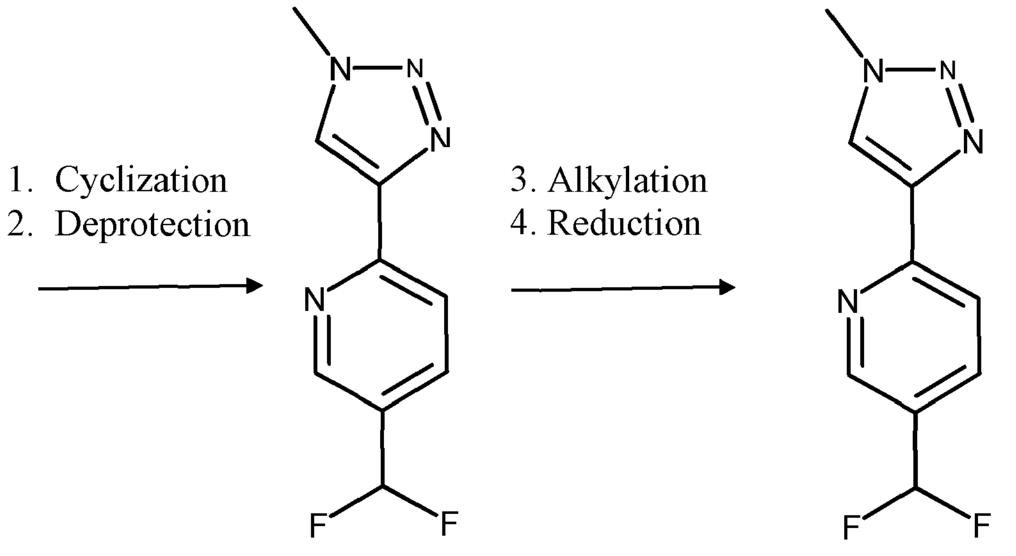
1. Cyclization
2. Deprotection
3. Alkylation
4. Reduction

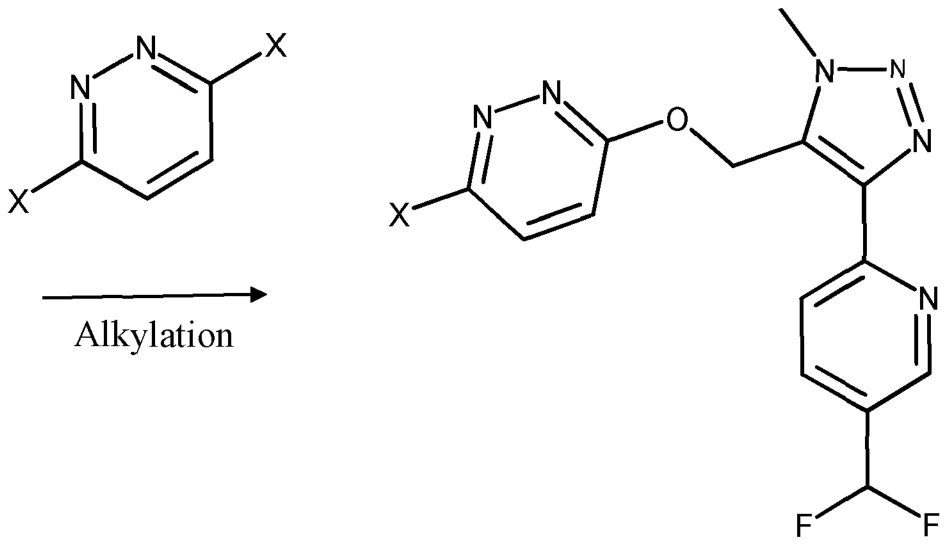
Alkylation

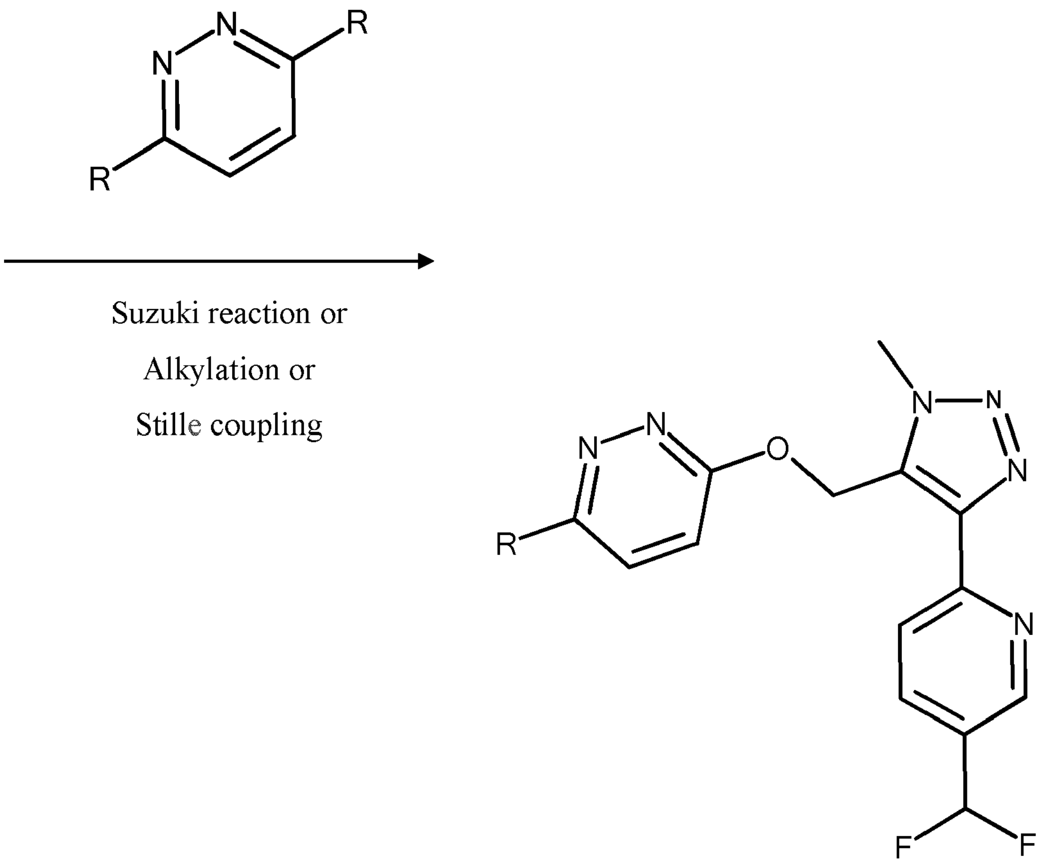


**Abbreviations:**

| | |
|---|---|
| ACN | acetonitrile |
| °C | Degree celsius |
| CH | cyclohexane |
| DCM | dichloro methane |
| DMF | *N,N*-dimethylformamide |
| DMSO | dimethyl sulfoxide |
| dppf | 1,1 ’-Ferrocenediyl-bis(diphenylphosphine) |
| ESI-MS | Electrospray ionisation mass spectrometry |
| Ex | example |
| Eq | equivalent |
| h | hour |
| HCl | Hydrochlorid acid |
| HPLC | High performance liquid chromatography |
| L | liter |
| MeOH | methanol |
| $NaHCO_3$ | sodium bicarbonate |
| NMP | N-Methyl-2-pyrrolidone |
| min | minute |

(continued)

| | |
|---|---|
| mL | milliliter |
| PE | petroleum ether |
| RT | room temperature (about 20°C) |
| TFA | trifluoroacetic acid |
| temp | temperature |
| THF | tetrahydrofuran |
| TLC | Thin-layer chromatography on $SiO_2$ |
| XPhos | 2-Dicyclohexylphosphino-2',4',6'-triisopropylbiphenyl |

**Analytical HPLC methods**

**Method A**

| time (min) | Vol% water (incl. 0.1% TFA) | Vol% ACN | Flow [mL/min] |
|---|---|---|---|
| 0.00 | 95 | 5 | 1.5 |
| 1.30 | 0 | 100 | 1.5 |
| 1.50 | 0 | 100 | 1.5 |
| 1.60 | 95 | 5 | 1.5 |
| Analytical column: Sunfire C18 (Waters) 2.5 μm; 3.0 x 30 mm; column temp: 60°C | | | |

**Method B**

| time (min) | Vol% water (incl. 0.1% TFA) | Vol% ACN | Flow [mL/min] |
|---|---|---|---|
| 0.0 | 99 | 1 | 1.6 |
| 0.02 | 99 | 1 | 1.6 |
| 1.00 | 0 | 100 | 1.6 |
| 1.10 | 0 | 100 | 1.6 |
| Analytical column: Xbridge BEH C18, 2.1 x 30 mm, 1.7 μm; column temp: 60°C | | | |

**Method C**

| time (min) | Vol% water (incl. 0.1% TFA) | Vol% ACN | Flow [mL/min] |
|---|---|---|---|
| 0.00 | 97 | 3 | 2.2 |
| 0.20 | 97 | 3 | 2.2 |
| 1.20 | 0 | 100 | 2.2 |
| 1.25 | 0 | 100 | 3.0 |
| 1.40 | 0 | 100 | 3.0 |
| Analytical column: Sunfire (Waters) 2.5 μm; 3.0 x 30 mm; column temp: 60°C | | | |

**Method D**

| time (min) | Vol% water (incl. 0.1% $NH_3$) | Vol% ACN | Flow [mL/min] |
|---|---|---|---|
| 0.00 | 95 | 5 | 1.5 |
| 1.30 | 0 | 100 | 1.5 |
| 1.50 | 0 | 100 | 1.5 |
| 1.60 | 95 | 5 | 1.5 |
| Analytical column: XBridge C18 (Waters) 2.5 μm; 3.0 x 30 mm; column temp: 60°C | | | |

**Method E**

| time (min) | Vol% water (incl. 0.1% TFA) | Vol% ACN (incl. 0.08% TFA) | Flow [mL/min] |
|---|---|---|---|
| 0.00 | 95 | 5 | 1.5 |
| 1.30 | 0 | 100 | 1.5 |
| 1.50 | 0 | 100 | 1.5 |
| 1.60 | 95 | 5 | 1.5 |
| Analytical column: Sunfire C18 (Waters) 2.5 μm; 3.0 x 30 mm; column temp: 60°C | | | |

**Method F**

| time (min) | Vol% water (incl. 0.1% $NH_3$) | Vol% ACN | Flow [mL/min] |
|---|---|---|---|
| 0.00 | 95 | 5 | 1.5 |
| 1.30 | 0 | 100 | 1.5 |
| 1.50 | 0 | 100 | 1.5 |
| 1.60 | 95 | 5 | 1.5 |
| Analytical column: Sunfire C18 (Waters) 2.5 μm; 3.0 x 30 mm; column temp: 60°C | | | |

**Method G**

| time (min) | Vol% water (incl. 0.1% $NH_4OH$) | Vol% ACN | Flow [mL/min] |
|---|---|---|---|
| 0.00 | 97 | 3 | 2.2 |
| 0.20 | 97 | 3 | 2.2 |
| 1.20 | 0 | 100 | 2.2 |
| 1.25 | 0 | 100 | 3 |
| 1.40 | 0 | 100 | 3 |
| Analytical column: XBridge C18 (Waters) 2.5 μm; 3.0 x 30 mm; column temp: 60°C | | | |

**Method H**

| time (min) | Vol% water (incl. 0.1% $NH_4OH$) | Vol% ACN | Flow [mL/min] |
|---|---|---|---|
| 0.00 | 95 | 5 | 1.5 |
| 1.30 | 0 | 100 | 1.5 |
| 1.50 | 0 | 100 | 1.5 |

(continued)

| time (min) | Vol% water (incl. 0.1% $NH_4OH$) | Vol% ACN | Flow [mL/min] |
|---|---|---|---|
| 1.60 | 95 | 5 | 1.5 |
| Analytical column: XBridge C18 (Waters) 2.5 μm; 3.0 x 30 mm; column temp: 60°C | | | |

**Method I**

| time (min) | Vol% water (incl. 0.1% $NH_3$) | Vol% ACN | Flow [mL/min] |
|---|---|---|---|
| 0.00 | 95 | 5 | 1.3 |
| 0.02 | 95 | 5 | 1.3 |
| 1.1 | 0 | 100 | 1.3 |
| 1.3 | 0 | 100 | 1.3 |
| Analytical column:XBridge BEH C18_2.1 x 30 mm_1.7 μm; column temp:60°C | | | |

**Method J**

| time (min) | Vol.-% water (incl. 0.1 % TFA | Vol.-% ACN (incl. 0.08 % TFA) | Flow [mL/min] |
|---|---|---|---|
| 0.00 | 95 | 5 | 1.5 |
| 1.3 | 0 | 100 | 1.5 |
| 1.5 | 0 | 100 | 1.5 |
| 1.6 | 95 | 5 | 1.5 |
| Analytical column: Sunfire C18 (Waters) 2.5 μm; 3.0 x 30 mm; column temp: 60°C | | | |

**Method K**

| time (min) | Vol.-% water (incl. 0.1 % TFA) | Vol.-% ACN (incl. 0.08 % TFA) | Flow [mL/min] |
|---|---|---|---|
| 0.00 | 95 | 5 | 1.5 |
| 1.30 | 0 | 100 | 1.5 |
| 1.50 | 0 | 100 | 1.5 |
| 1.60 | 95 | 5 | 1.5 |
| Analytical column: Sunfire C18 (Waters) 2.5 μm; 3.0 x 30 mm; column temp: 60 °C | | | |

**Method L**

| time (min) | Vol.-% water (incl. 0.1 % TFA) | Vol.-% ACN (incl. 0.08 % TFA) | Flow [mL/min] |
|---|---|---|---|
| 0.00 | 95 | 5 | 1.5 |
| 1.30 | 0 | 100 | 1.5 |
| 1.50 | 0 | 100 | 1.5 |
| 1.60 | 95 | 5 | 1.5 |
| Analytical column: Hypercarb_3.0 x 30 mm_3 μm; column temp: 60 °C | | | |

**Method M**

| time (min) | Vol.-% water (incl. 0.1 % TFA) | Vol.-% ACN (incl. 0.08 % TFA) | Flow [mL/min] |
|---|---|---|---|
| 0.00 | 95 | 5 | 0.5 |
| 4.00 | 5 | 95 | 0.5 |
| 5.00 | 4.5 | 95.5 | 0.5 |
| 5.20 | 95 | 5 | 0.5 |
| 6.00 | 95 | 5 | 0.5 |
| Analytical column: Acquity UPLC 1.8 µm C18 (2.1 x 50 mm), 100 Å; column temp: 25 °C | | | |

**Method N**

| time (min) | Vol.-% water (incl. 0.1 % TFA) | Vol.-% ACN (incl. 0.08 % TFA) | Flow [mL/min] |
|---|---|---|---|
| 0.00 | 95 | 5 | 1.0 |
| 1.00 | 95 | 5 | 1.0 |
| 4.75 | 20 | 80 | 1.0 |
| 5.25 | 20 | 80 | 1.0 |
| 6.00 | 95 | 5 | 1.0 |
| 7.00 | 95 | 5 | 1.0 |
| Analytical column: Kinetex XB-C18 2.6 µm (4.6 x 50mm), 100Å; column temp: 25 °C | | | |

## Preparation of Starting Compounds I to XVIII

**Example I:** [3-(5-Difluoromethyl-pyridin-2-yl)-5-methyl-3H-[1,2,3]triazol-4-yl]-methanol

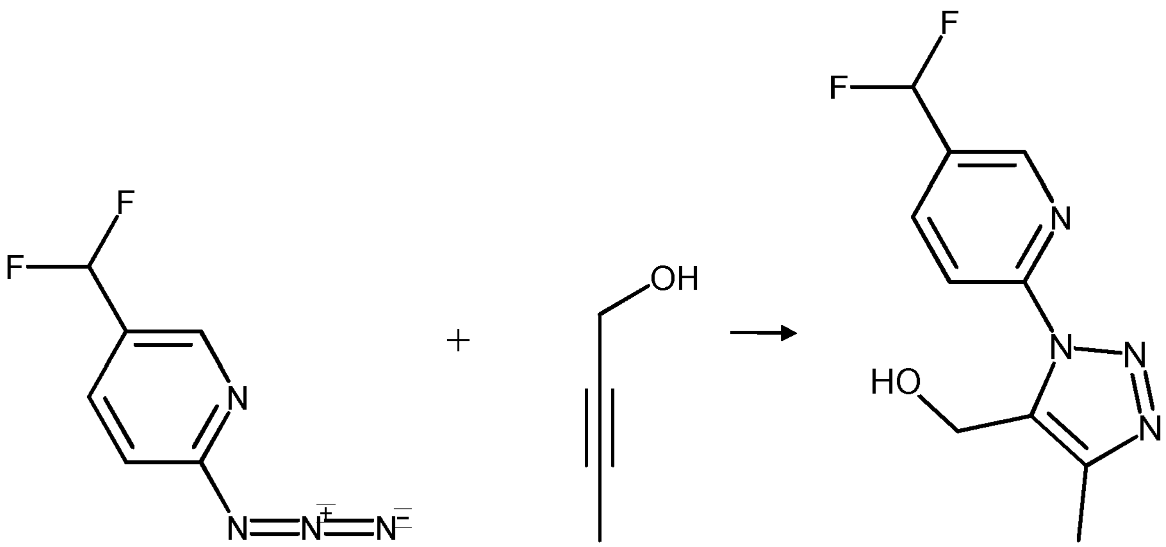

2-Azido-5-difluoromethyl-pyridine (25.7 g, 151 mmol) in 50mL But-2-an-1-ol is stirred for 5 days at 120°C. The excess of the alcohol is evaporated in vacuo as much as possible. The 3-(5-Difluoromethyl-pyridin-2-yl)-5-methyl-3H-[1,2,3]triazol-4-yl]-methanol 9.00 g is obtained by column chromatography on silica gel as a white solid utilazing DCM/acetone (10: 1) as the eluent.

$^1H$ NMR (400 MHz, DMSO-$d_6$) δ ppm 2.32 - 2.43 (m, 3 H) 4.86 (s, 2 H) 7.26 (t, J=56Hz 1 H) 8.11 (d, *J*=8.59 Hz, 1 H) 8.31 (s, 1 H) 8.84 (d, *J*=0.76 Hz, 1 H)

**Example II:** 2-bromo-5-(difluoromethyl)pyridine

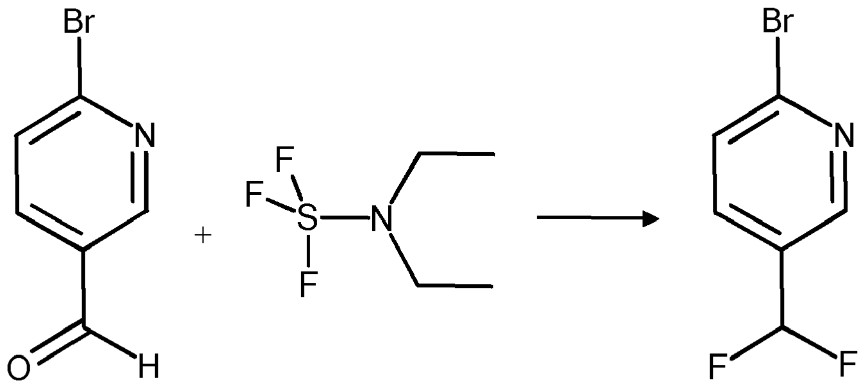

To a solution of 6-Bromo-pyridine-3-carbaldehyde (186 g, 1.00 mol) in 1.86 L Dichloromethane at 0°C is added dropowise diethylaminosulfur trifluoride (185 ml; 1.40 mol). The mixture is stirred at rt for 18 h. The reaction mixture is poured into ice and saturated $NaHCO_3$. The aqueous phase is extracted 3 times with DCM. The combined organic layers are dried over $Na_2SO_4$, filtered and concentrated. The residue is purified by column chromatography (silica gel, hexane/EE (4/1)) to afford 170 g of the product.

| | |
|---|---|
| $C_6H_4BrF_2N$ | (M = 208.0 g/mol) |
| ESI-MS: | 208 $[M+H]^+$ |

$^1H$ NMR (DMSO-$d_6$, 400 MHz): δ = 8.65 (d, *J* = 1.3 Hz, 1H), 7.91-8.08 (m, 1H), 7.76-7.92 ppm (m, 1H), 7.17 (t, J = 56 Hz, 1H)

**Example III:** 5-(difluoromethyl)-2-[2-(trimethylsilyl)ethynyl]pyridine

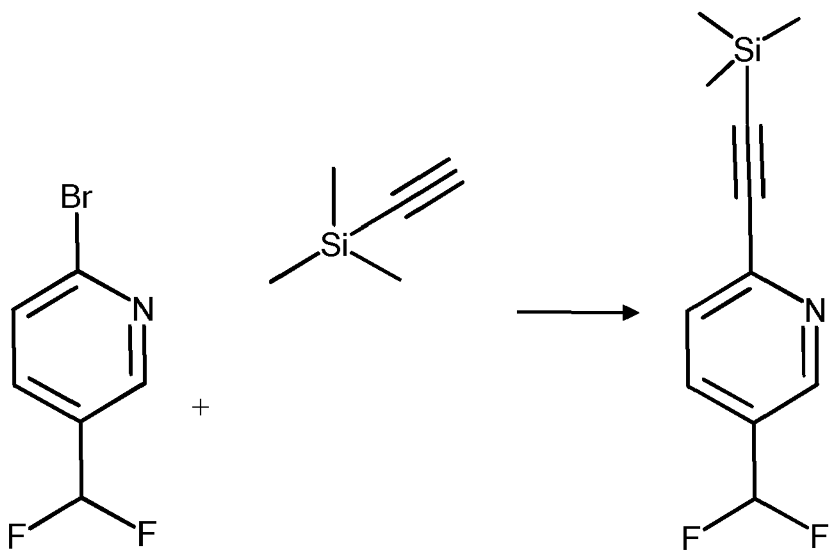

To a mixture of **example II** (120 g, 0.58 mol), bis(triphenylphosphine)palladium(II)chloride (20.2 g, 0.03 mol), copper(I) iodide (5.49 g, 0.03 mol) and triethylamine (250 mL, 1.73 mol) in 600 mL tetrahydrofuran at 0°C is added

dropwise ethynyl-trimethyl-silane (160 mL, 1.15 mol). The resulting mixture is stirred ar RT for 18h. The mixture is filtered through celite. The cake is washed with EtOAc. The filtrate is washed with water, dried over $Na_3SO_4$, filtered and concentrated. Purification by column chromatography (silica gel, hexane/EE (19/1)) afforded 117 g of the product.

| | |
|---|---|
| $C_{11}H_{13}F_2NSi$ | (M = 225.3 g/mol) |
| ESI-MS: | 226 $[M+H]^+$ |

$^1H$ NMR (DMSO-$d_6$, 400 MHz): $\delta$ = 8.76 (d, *J* = 0.8 Hz, 1H), 8.01 (dt, *J* = 8.1, 0.9 Hz, 1H), 7.69 (d, *J* = 8.1 Hz, 1H), 7.16 (t, J = 56 Hz, 1H) 0.23-0.30 ppm (m, 9H)

**Example IV:** 5Difluoromethyl-2-ethynyl-pyridine

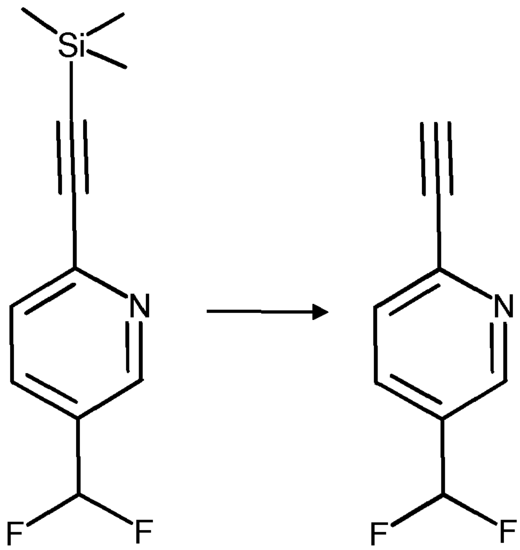

To a solution of **example III** (100.0 g, 0.42 mol) 800 mL tetrahydrofuran is added water (15.0 mL, 834 mmol). The resulting solution is cooled to 0°C an then tetrabutylammonium fluoride 1.0 M in THF (143 mL; 0.50 mol) is added dropwise. After 1h, TLC indicated that the reaction is completed. Water is added and the aqueous layer is extracted 3 times with diethyl ether. The combined organic layers are dried, filtered and carefully concentrated. The residue is purified by column chromatography (silica gel, hexane/DCM (1/1 to 0/1)) to afford the product.

| | |
|---|---|
| $C_8H_5F_2N$ | (M= 153.1 g/mol) |
| ESI-MS: | 154 $[M+H]^+$ |

$^1H$ NMR (DMSO-$d_6$, 400 MHz): $\delta$ = 8.77 (d, *J* = 0.8 Hz, 1H), 8.03 (br d, *J* = 8.1 Hz, 1H), 7.72 (d, *J* = 8.1 Hz, 1H), 7.17 (t, J = 56 Hz, 1H) 4.50 ppm (s, 1H)

**Example V:** 5-Difluoromethyl-2-(1-trimethylsilanylmethyl-1H-[1,2,3]triazol-4-yl)-pyridine

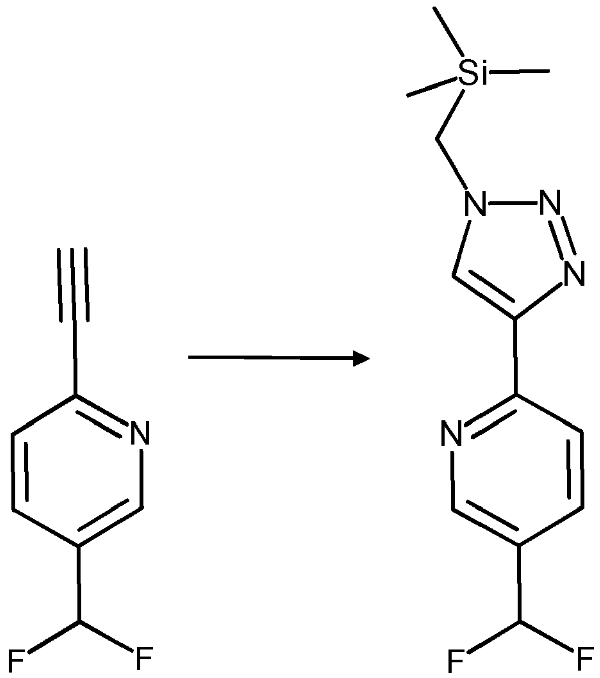

To a solution of **example IV** (50.0 g, 0.30 mol) in 1,5 L DMF, Copper (I) iodide (10.9 g, 0.06 mol) and N,N-diisopropylethylamine (50.360 mL 0.29 mol) is added, followed by dropwise addition of trimethylsilylmethyl azide (50.6 mL, 0.34 mol). The resulting mixture is stirred at rt for 24h. The reaction is quenched by addition of water/brine, then EtOAc is added and the mixture is filtered through celite. The filtrate is extracted 3 times with EtOAc, dried over Na2SO4, filtered and concentraced. Purification by column chromatography (silica gel, hexane/EE (3/1)) followed by trituration of the solids with pentane and drying afforded 68.0 g of the product.

| | |
|---|---|
| $C_{12}H_{16}F_2N_4Si$ | (M = 282.4 g/mol) |
| ESI-MS: | 283 $[M+H]^+$ |
| $R_t$ (HPLC): | 3.52 min (Method M) |

$^1H$ NMR (DMSO-$d_6$, 400 MHz): $\delta$ = 8.78 (s, 1H), 8.52 (s, 1H), 8.13-8.20 (m, 1H), 8.05-8.12 (m, 1H), 7.17 (t, J = 56 Hz, 1H) 4.10 (s, 2H), 0.11 ppm (s, 9H)

**Example VI:** 5-Difluoromethyl-2-(1-methyl-1H-[1,2,3]triazol-4-yl)-pyridine

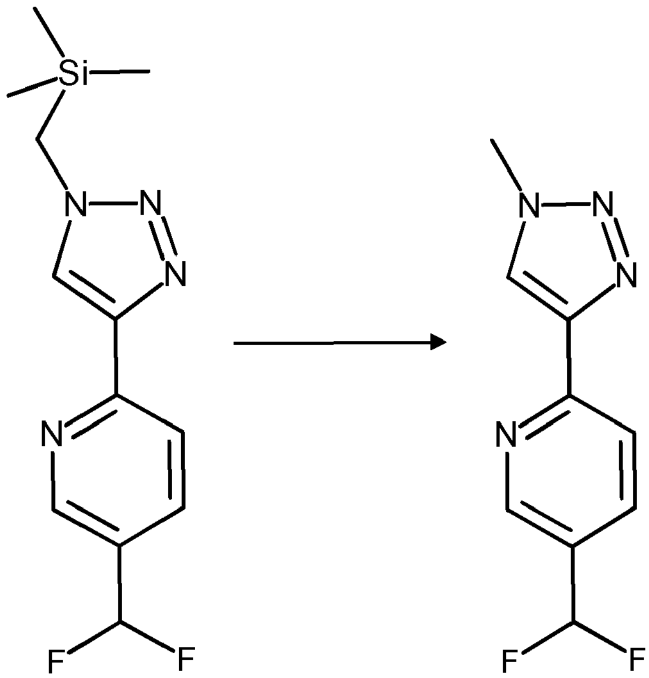

To a solution of **example V** (93.0 g, 0.33 mol in 1.86 L Tetrahydrofuran is added water (11.9 ml, 0.66 mol). The resulting solution is cooled to 0°C and tetrabutylammonium fluoride (395.2 ml, 0.40 mol) is added dropwise. The reaction mixture is stirred at 0°C for 1.5 h. Water is added and the THF is evaporated. The precipitate formed is filtered, washed with water and dried to afford 51.0 g of the product

| | |
|---|---|
| $C_9H_8F_2N_4$ | (M = 210.1 g/mol) |
| ESI-MS: | 211 $[M+H]^+$ |

$^1$H NMR (DMSO-$d_6$, 400 MHz): $\delta$ = 8.80 (d, *J* = 1.0 Hz, 1H), 8.65 (s, 1H), 8.14-8.19 (m, 1H), 8.03-8.13 (m, 1H), 7.17 (t, J = 56 Hz, 1H), 4.13 ppm (s, 3H)

**Example VII:** 5-(5-Difluoromethyl-pyridin-2-yl)-3-methyl-3H-[1,2,3]triazole-4-carbaldehyde

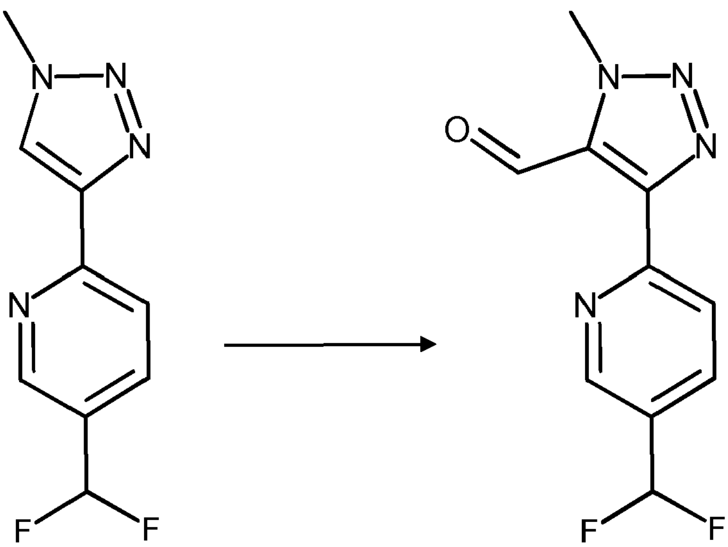

To a solution of **example VI** (40.0g, 0.19 mol) in 1.2 L Tetrahydrofuran at - 65°C is added dropwise a 2.5 M soution of N-Butyllithium (114.2 mL, 0.29 mol) in hexanes. The resulting mixture is stirred at this temperature for 1.5 h. Then, N,N-Dimethylformamide (147.4 mL, 1.90 mol) is added dropwise and then reaction mixture is stirred at 0°C for 30 min. The reaction is quenched by slowly addition of aqueous $NH_4Cl$. The aqueous layer is extracted 3 times with EtOAc. The combined organic layers are dried over $Na_2SO_4$, filtered and concentrated. Purification by column chromatography (silica gel, DCM/EE (7/3 to 5/5)) followed by trituration with pentane afforded 19.9 g of the product.

| | |
|---|---|
| $C_9H_8F_2N_4$ | (M = 238.2 g/mol) |
| ESI-MS: | 239 $[M+H]^+$ |
| $R_t$ (HPLC): | 2.68 min (Method M) |

$^1H$ NMR (DMSO-$d_6$, 400 MHz): $\delta$ = 10.68 (s, 1H), 8.92 (d, *J* = 0.8 Hz, 1H), 8.34 (d, *J* = 8.3 Hz, 1H), 8.22 (dd, *J* = 8.1, 1.0 Hz, 1H), 7.23 (t, J = 56 Hz, 1H), 4.29 ppm (s, 3H)

**Example VIII:** 5-(5-Difluoromethyl-pyridin-2-yl)-3-methyl-3H-[1,2,3]triazole-4-carbaldehyde

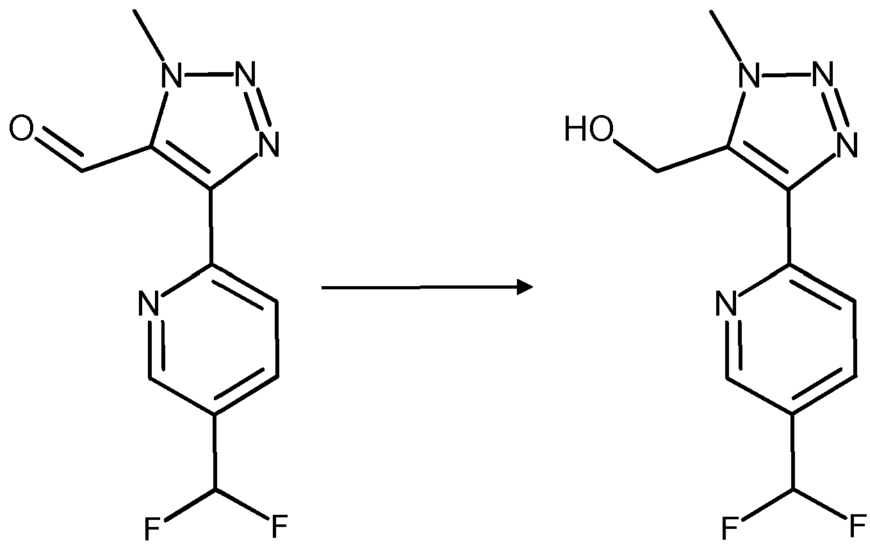

Example VII (19.9 g, 0.08 mol) is dissolved in 199 mL Methanol and 99.5 mL Tetrahydrofuran. The resulting solution is cooled to 0°C. Then sodium borohydride (6.32 g, 0.17 mol) is added portionwise and the reaction mixture is stirred at this temperature for 2 h. The reaction is quenched by addition of water. The MeOH is evaporated and the resulting precipitate is collected by filtration, washed with water and dried. The solids are triturated with pentane to afford 19.4 g of the product.

| | |
|---|---|
| $C_9H_8F_2N_4$ | (M = 240.2 g/mol) |
| ESI-MS: | 241 $[M+H]^+$ |
| $R_t$ (HPLC): | 3.52 min (Method N) |

$^1H$ NMR (DMSO-$d_6$, 400 MHz): $\delta$ = 8.83 (s, 1H), 8.22 (d, *J* = 8.1 Hz, 1H), 8.11 (br d, *J* = 8.1 Hz, 1H), 7.18 (t, J = 56 Hz, 1H), 5.53 (s, 1H), 5.09 (d, *J* = 3.8 Hz, 2H), 4.11 ppm (s, 3H)

**Example IX.1:** 3-[3-(5-Difluoromethyl-pyridin-2-yl)-5-methyl-3H-[1,2,3]triazol-4-ylmethoxy]-6-iodo-pyridazine

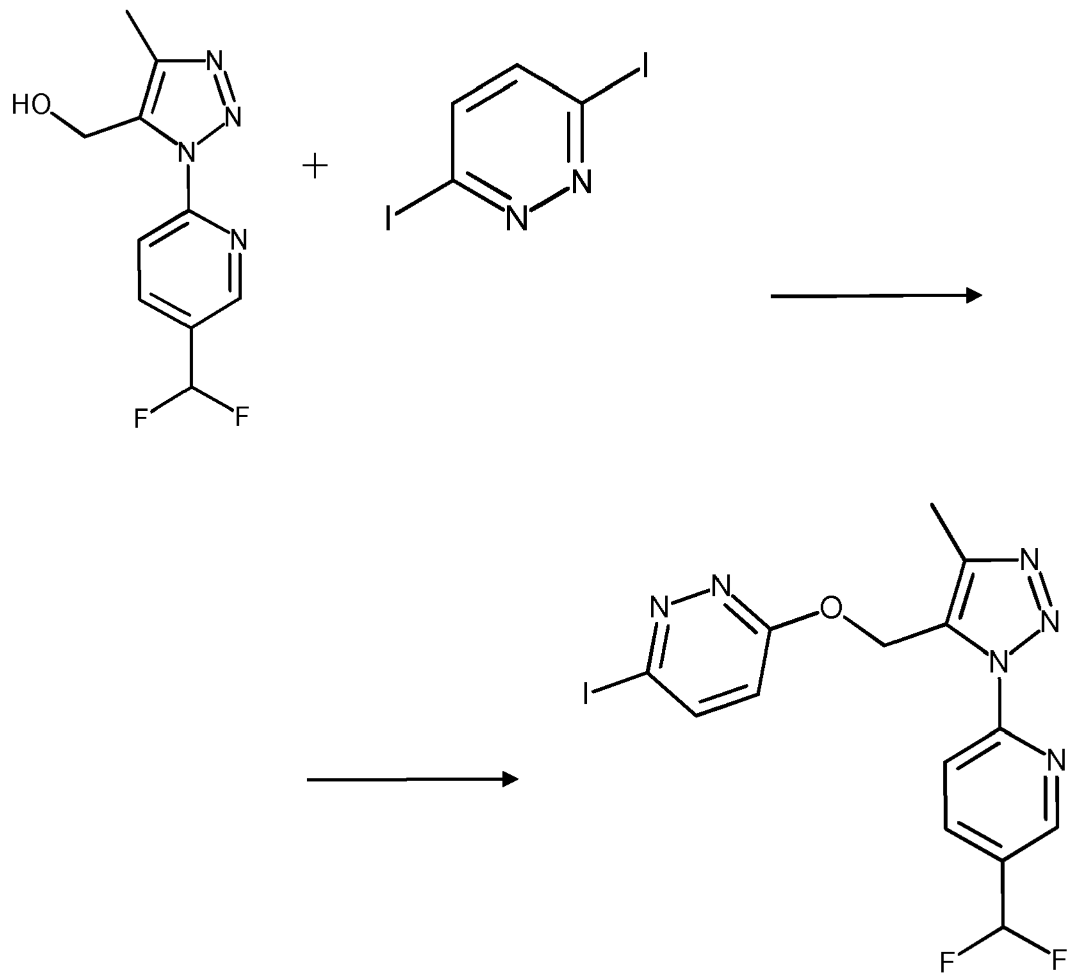

To example I (4.00 g , 16.6 mmol) in 50 mL THF is added sodium hydride (1.10 g, 25.0 mmol) and 3,6-Diiodo-pyridazine (5.50 g, 17.0 mmol) The reaction mixture is stirred at 80°C overnight.. The reaction mixture is evaporated. The residue is quenched with water and the product is extracted with DCM. The organic phases are combined and the organic phase is dried with $MgSO_4$ and is evaporated. The crude product is purified by column chromatography (silica gel, CH/EE (6/4)) to afford 6.30 g of the product.

| | |
|---|---|
| $C_{14}H_{11}F_{21}N_6O$ | (M = 444.2 g/mol) |
| ESI-MS: | 445 $[M+H]^+$ |
| $R_t$ (HPLC): | 0.57 min (Method A) |

$^1H$ NMR (DMSO-d6, 400 MHz): $\delta$ = 8.53-8.80 (m, 1H), 8.33 (dt, J = 8.5, 1.1 Hz, 1H), 7.96 (d, J = 9.1 Hz, 1H), 7.22 (t, J = 52 Hz, 1H), 6.95 (d, J = 9.1 Hz, 1H), 5.95 (s, 2H), 2.36-2.45 ppm (m, 3H)

Examples IX.2 to IX.4 mentioned in the following table of the compounds of examples IX.2 to IX.4 are prepared according to the general procedure of **Example IX.1** described above.

Table of the compounds of examples IX.2 to IX.4:

| Ex. | Starting material | Starting material | Structure |
|---|---|---|---|
| **IX.2** | 1.0 eq<br>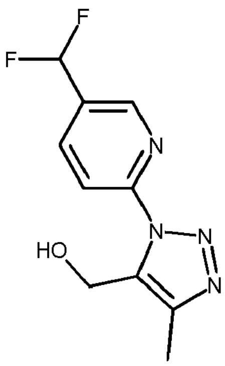<br>**I** | 1.5 eq<br>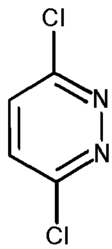 | 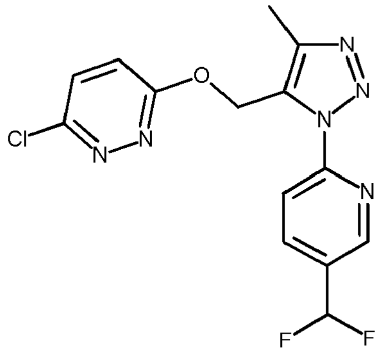 |
| **IX.3** | 1.0 eq<br>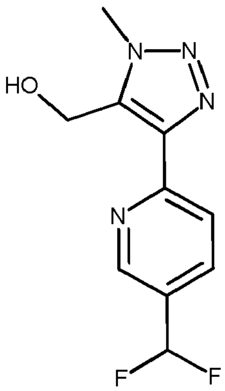<br>**VIII** | 1.5 eq<br>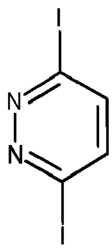 | 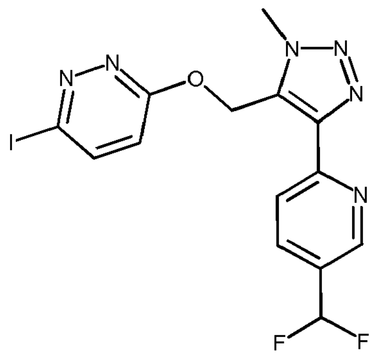 |
| **IX.4** | 1.0 eq<br>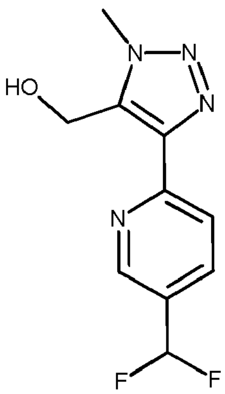<br>**VIII** | 1.5 eq<br>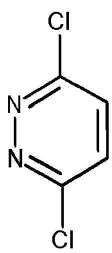 | 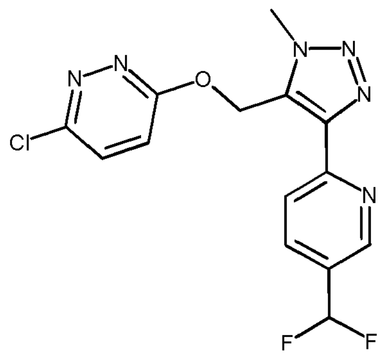 |

The reaction conditions mentioned in the Table below were used for examples IX.2 to IX.4

Table: Reaction conditions used for examples IX.2 to IX.4 above

| Ex. | Reaction conditions | ESI-MS/NMR | HPLC retention time [min] (method) |
|---|---|---|---|
| **IX.2** | 1.5eq pyridazine; 1.3eq NaH; overnight 60°C; purification by preparative HPLC | 353 [M+H]+ | 1.01 (Method A) |
| **IX.3** | | 445 $[M+H]^+$/$^1$H NMR (DMSO-$d_6$, 400 MHz): $\delta$ = 8.78 (d, *J* = 1.0 Hz, 1H), 8.26 (d, *J* = 8.2 Hz, 1H), 8.13 (dt, *J* = 8.2, 1.0 Hz, 1H), 8.01 (d, *J* = 9.1 Hz, 1H), 7.16 (t, J = 56 Hz, 1H), 7.08 (d, *J* = 9.1 Hz, 1H), 6.11 (s, 2H), 4.18 ppm (s, 3H) | 0.54 (Method B) |
| **IX.4** | 1,3eq NaH; 60°C; 4h; purification by preparative HPLC | 353 [M+H]+/$^1$H NMR (DMSO-$d_6$, 400 MHz): $\delta$ = 8.78 (d, *J* = 0.9 Hz, 1H), 8.26 (d, *J* = 8.2 Hz, 1H), 8.10-8.16 (m, 1H), 7.85 (d, *J* = 9.3 Hz, 1H), 7.41 (d, *J* = 9.3 Hz, 1H), 7.16 (t, J = 56 Hz, 1H), 6.14 (s, 2H), 4.19 ppm (s, 3H) | 0.98 (Method G) |

**Example X.1:** 1-(6-chloropyridazin-3-yl)-1 H-pyrazole-4-carbonitrile

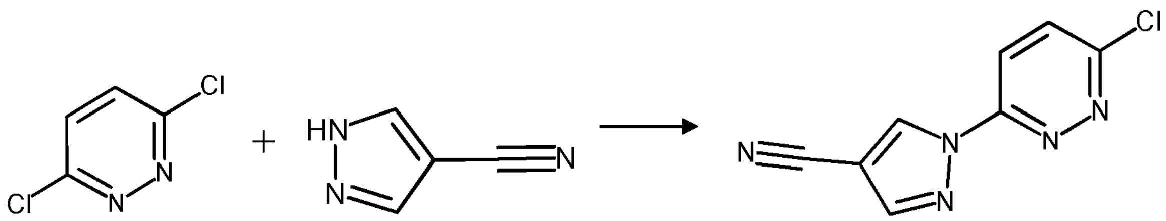

To 4-Cyanopyrazole (312 mg, 3.40 mmol) in 5 mL DMF are added 3,6-dichloropyridazine (500 mg, 3.40 mmol) and potassium carbonate (1.40 g, 10.1 mmol) and the reaction mixture is stirred at RT overnight. The mixture is quenched with iced water and the precipitation is filtered. The residue is washed with water and the isolated solid is dried in the vacuum drying cabinet to get 524 mg of the product.

| | |
|---|---|
| $C_8H_4ClN_5$ | (M = 205.6 g/mol) |
| ESI-MS: | 206 $[M+H]^+$ |
| $R_t$ (HPLC): | 0.80 min (Method C) |

The following compounds are prepared according to the general procedure of **Example X.1** described above:

| Ex. | Starting material | Starting material | Structure | Solvent | ESI-MS/NMR | HPLC retention time (method) [min] |
|---|---|---|---|---|---|---|
| **X.2** | 1.0 eq 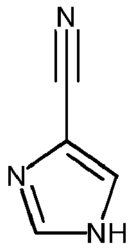 | 1.5 eq 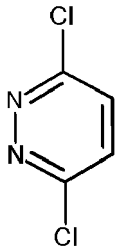 | 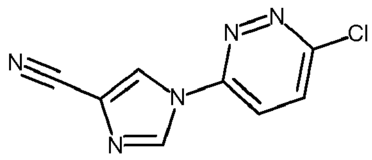 | DMSO | 206 [M+H]+ | 0.46 (Method A) |
| **X.3** | 1.0 eq 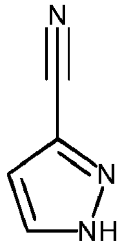 | 1.5 eq 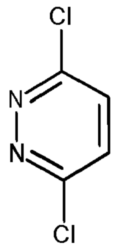 | 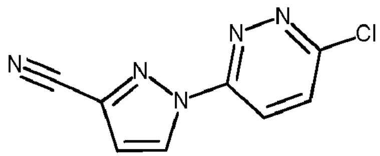 | DMSO | 206 [M+H]+ | 0.43 (Method B) |

**Example XI:** 3-chloro-6-(5-methyl-1H-1,2,4-triazol-1-yl)pyridazine

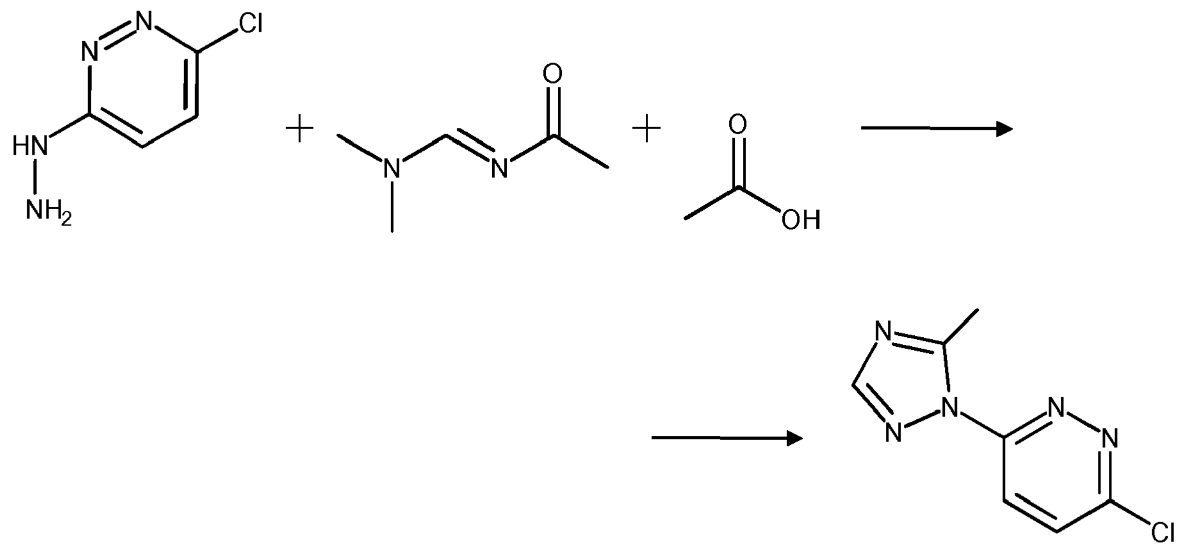

6-Chloro-pyridazin-3-yl)-hydrazine (16.4 g, 113 mmol) and N-[1-Dimethylamino-meth-(E)-ylidene]-acetamide (15.5 g, 136 mmol) are dissolved in 164 mL acetic acid. The mixture is placed in a pre-heated oil bath at 80°C. The reaction is stirred at this temperature for 30 min (TLC monitoring) and acetic acid is evaporated in vacuo. The residue is dissolved in EtOAc and the organic layer is slowly neutralized with $NaHCO_3$ (conc. water colution).. The oranic layer is dried over Na2SO4 and the solvent is evaporated. The product is purified on silica gel utillizing EtOAc/hehane (3: 1) as an eluent. 10.2 g 3Chloro-6-(5-methyl-[1,2,4]triazol-1-yl)-pyridazine is obtained as a creamy solid.

**Example XII:** methyl 1-(6-chloropyridazin-3-yl)-1H-imidazole-4-carboxylate

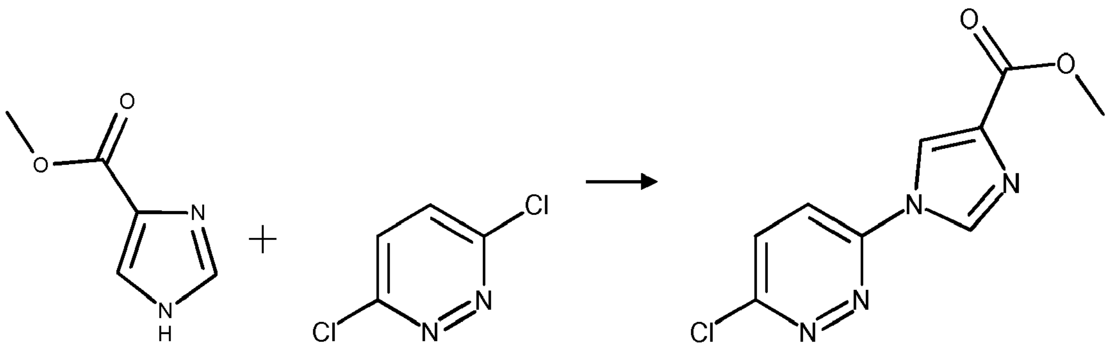

To sodium hydride (1.74 g, 43.6 mmol) in 50 mL DMF is added methyl 1H-imidazole-4-carboxylate (5.00 g, 39.6 mmol) at 0 °C. The reaction mixture is stirred for 30 min. To the reaction mixture is added a solution of 3,6-dichloropyridazine (5.90 g, 39.6 mmol) in 30 mL DMF at 0 °C and the mixture is stirred for 20 h to reach RT. The reaction mixture is quenched with water under ice cooling and the precipitation is filtered, washed and dried to give 3.90 g of the product.

| | |
|---|---|
| $C_9H_7ClN_4O_2$ | (M = 238.6 g/mol) |
| ESI-MS: | 239 $[M+H]^+$ |

$^1H$ NMR (400 MHz, DMSO-$d_6$) $\delta$ = 8.74 (d, J = 1.3 Hz, 1H), 8.72 (d, J = 1.0 Hz, 1H), 8.43 (d, J = 9.3 Hz, 1H), 8.25 (d, J

= 9.3 Hz, 1H), 3.82 (s, 3H)

**Example XIII:** 1-(6-chloropyridazin-3-yl)-1H-imidazole-4-carboxylic acid

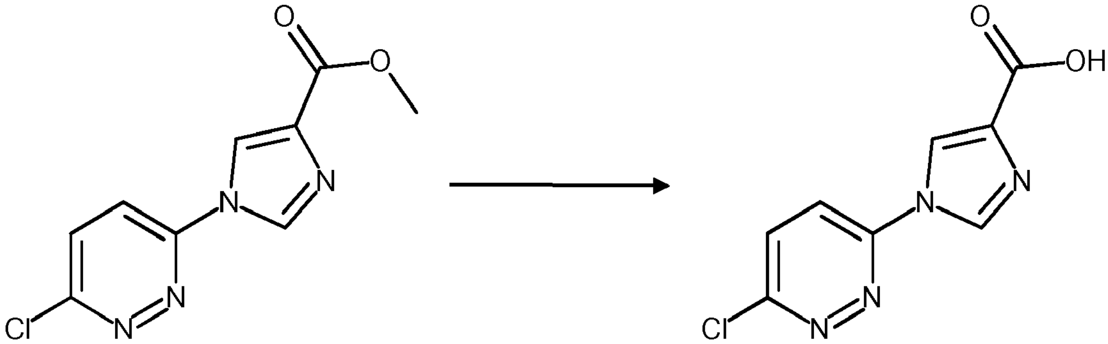

To **example XII** (3.80 g, 15.8 mmol) in 100 mL 1,4-dioxane is added 1M NaOH (16.0 mL, 16.0 mmol) and is stirred for 18 h at RT. The reaction mixture is quenched with ice and 1 M HCl (16.0 mL, 16.0 mmol). The precipitation is filtered, washed and dried to yield 3.30 g of the product.

| | |
|---|---|
| $C_8H_5ClN_4O_2$ | (M = 224.6 g/mol) |
| ESI-MS: | 225 $[M+H]^+$ |
| $R_t$ (HPLC): | 0.58 min (Method C) |

**Example XIV:** 1-(6-chloropyridazin-3-yl)-1H-imidazole-4-carboxamide

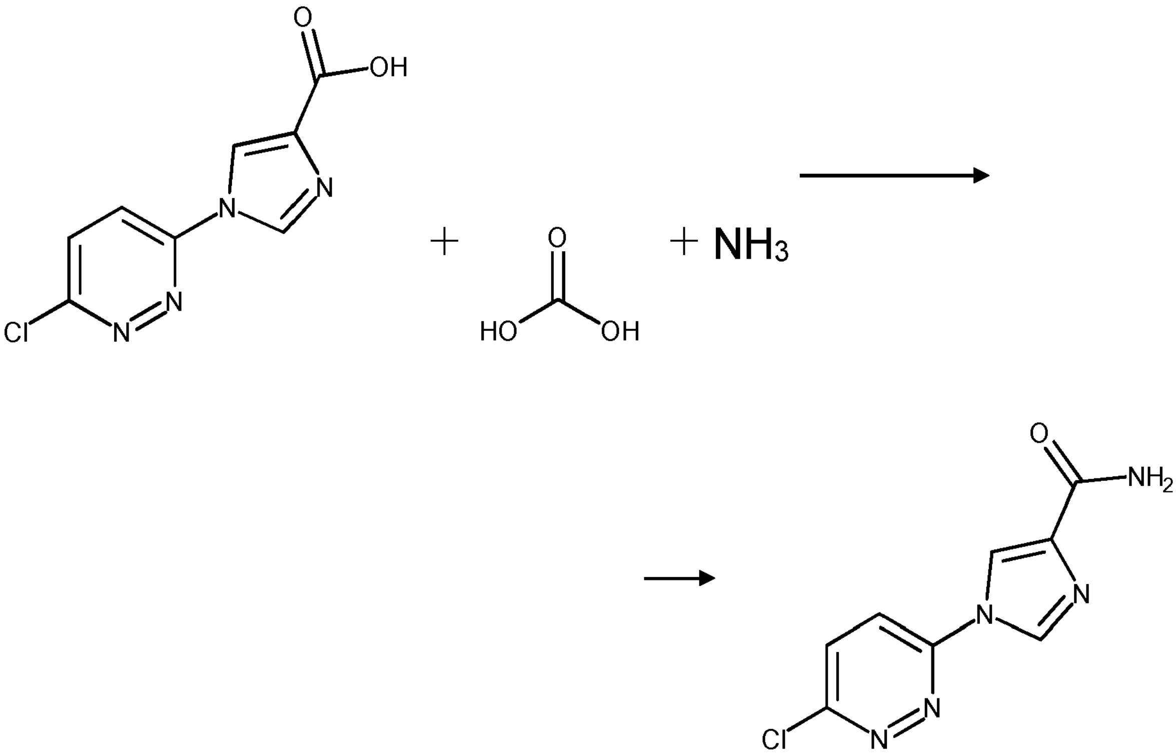

To **example VI** (1.00 g, 4.50 mmol) in 10 mL DMF are added DIPEA (2.30 mL, 13.4 mmol) and TBTU (1.40 g,

4.50 mmol) and is stirred for 10 min at RT. Ammonium bicarbonate (1.10 g, 13.4 mmol) is added and the reaction mixture is stirred for 1 h at RT.The mixture is quenched with ice and the precipitation is filtered, washed and dried to afford 0.80 g of the product.

| | |
|---|---|
| $C_8H_6ClN_5O$ | (M = 223,6 g/mol) |
| ESI-MS: | 224 $[M+H]^+$ |
| $R_t$ (HPLC): | 0,54 min (Method C) |

**Example XV:** 1-[6-(4-cyano-1H-imidazol-1-yl)pyridazin-3-yl]-1H-imidazole-4-carbonitrile

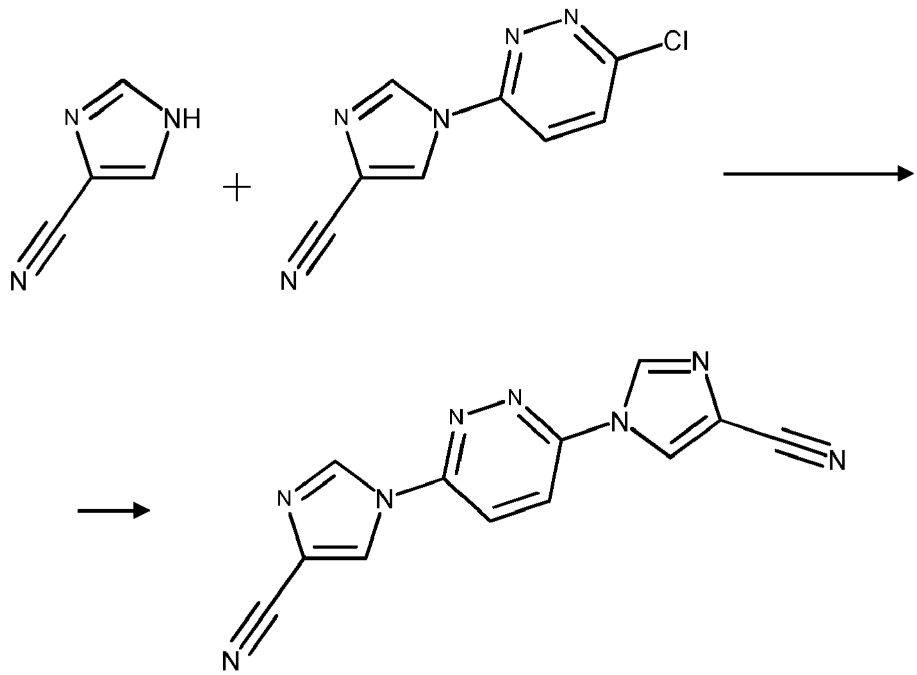

To 1H-imidazole-4-carbonitrile (9.30 g, 99.9 mmol) in 100 mL DMF are added **example III** (20.5 g, 99.9 mmol) and potassium carbonate (41.4 g, 299 mmol) and the reaction mixture is stirred at 50 °C for 18 h. Addition of 1H-imidazole-4-carbonitrile (5.00 g, 53.7 mmol) and the mixture is stirred at 50 °C for 3 days.

The mixture is quenched with water and the precipitate is filtered to give after drying 25.8 g of the product.

| | |
|---|---|
| $C_{12}H_6N_8$ | (M = 262.2 g/mol) |
| ESI-MS: | 263 $[M+H]^+$ |
| $R_t$ (HPLC): | 0.74 min (Method C) |

**Example XVI.1:** 3-chloro-6-(4-chloro-1H-pyrazol-1-yl)pyridazine

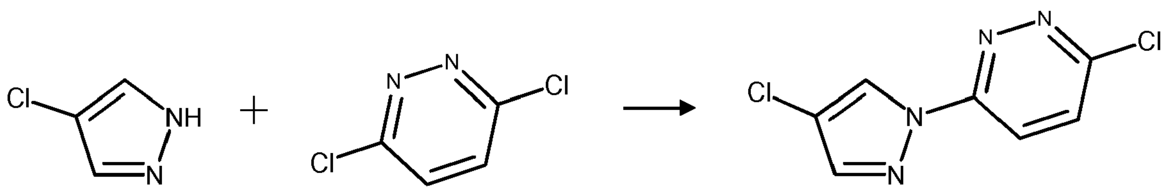

To 4-chloro-1H-pyrazole (688 mg, 7.00 mmol) in 5 mL DMF are added 3,6-Dichloropyridazine (500 mg, 3.36

mmol) and cesium carbonate (2.40 g, 7.38 mmol) and the reaction mixture is stirred at RT overnight. The mixture is quenched with water and the precipitate is filtered to give 686 mg of the product.

| | |
|---|---|
| $C_8H_6N_8$ | (M = 215.0 g/mol) |
| ESI-MS: | 216 $[M+H]^+$ |
| $R_t$ (HPLC): | 0,51 min (Method A) |

The following compounds are prepared according to the general procedure (**Example XVI.1**) described above:

| Ex. | Starting material | Starting material | Structure | Reaction conditions | ESI-MS/ NMR | HPLC retention time (method) [min] |
|---|---|---|---|---|---|---|
| **XVI.2** | 1.0 eq 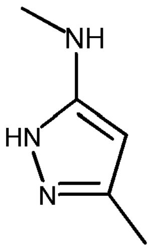 | 1.0 eq 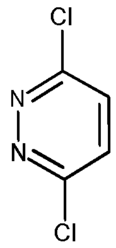 | 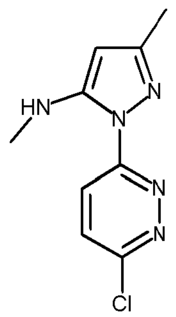 | 2.5 eq base; 60°C overnight; 80°C overnight; workup: extraction with water/EE; purification by silica column (CH/EE 9/1 to 1/9) | 224 [M+H]+ | 0.49 (Method B) |
| **XVI.3** | 1.0 eq 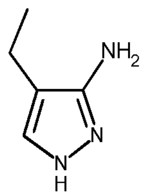 | 1.0 eq 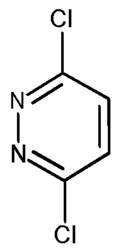 | 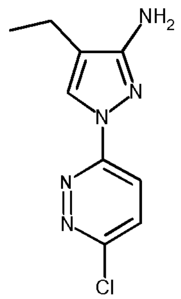 | 2.2 eq base; 60°C; 6h; workup: extraction with water/EE; purification by silica column (CH/EE 9/1 to 1/9) | 224 [M+H]+ | 0.52 (Method I) |
| **XVI.4** | 2.2 eq 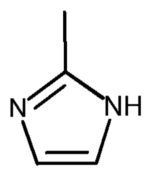 | 1.0 eq 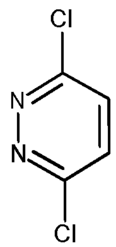 | 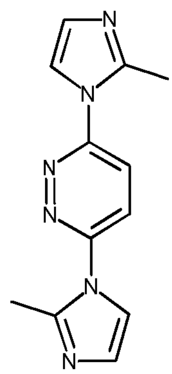 | 2.2 eq base | 241 [M+H]+ | 0.11 (Method A) |

| | | | | | | |
|---|---|---|---|---|---|---|
| **XVI.5** | 2.2 eq 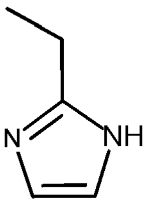 | 1.0 eq 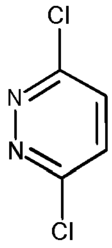 | 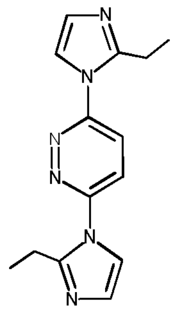 | 2.2 eq base | 269 [M+H]+ | 0.20 (Method A) |
| **XVI.6** | 2.2 eq 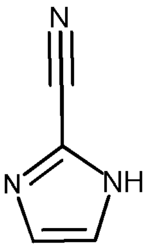 | 1.0 eq 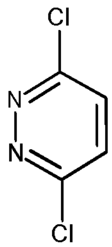 | 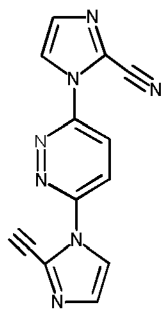 | 2.2 eq base | 263 [M+H]+ | 0.72 (Method C) |
| **XVI.7** | 2.0 eq 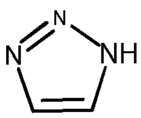 | 1.0 eq 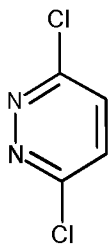 | 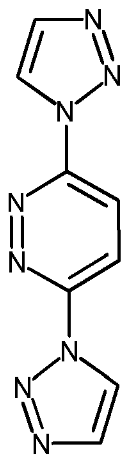 | 2.2 eq base; 60°C; overnight; product: isomer mixture | 215 [M+H]+ | 0.28 (Method B) |

**Example XVII:** 6-(pyrazin-2-yl)-2,3-dihydropyridazin-3 -one

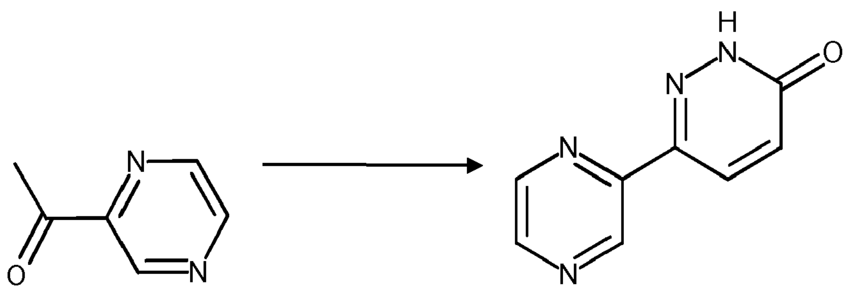

To 2-oxoacetic acid hydrate (2.26 g, 25.0 mmol) in aq. $K_2CO_3$-solution (6.79 g, 49.0 mmol in 30mL water) is added acetylpyrazine (3.00 g, 24.6 mmol). The mixture is stirred at RT for 6 h. Then acetic acid (12.9 mL, 221 mmol)

and hydrazine hydrate (1.42 mL, 29.0 mmol) are added and the reaction mixture is refluxed for 2h. The solution is cooled to RT and basified to pH7 with $K_2CO_3$. The precipitate is filtered, dried in the oven at 40°C to get 1.29 g of the product.

| | |
|---|---|
| $C_8H_6N_4O$ | (M = 174.1 g/mol) |
| ESI-MS: | 175 $[M+H]^+$ |
| $R_t$ (HPLC): | 0.23 min (Method A) |

**Example XVIII**: 3-chloro-6-(pyrazin-2-yl)pyridazine

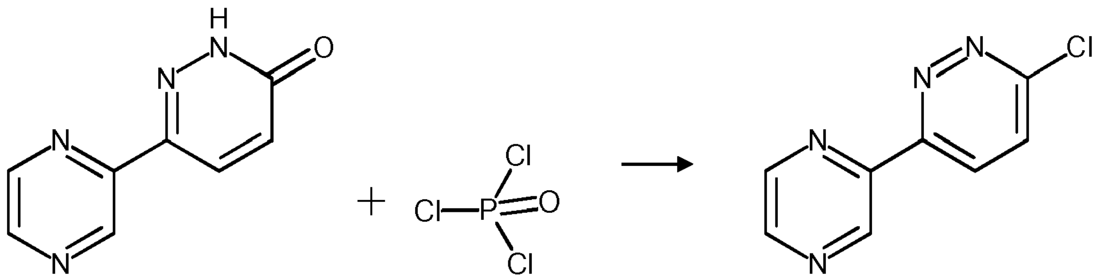

**Exapmle XVII** (1.50 g, 6.03 mmol) in $POCl_3$ (5.00 mL, 53.6 mmol) is stirred at 100°C for 1h. The reaction mixture is evaporated and the residue is diluted with DCM under cooling. 10 mL sat. NaHCO3-solution is added and this solution is added dropwise under stirring to an icy sat. NaHCO3-solution until the solution is neutral. After 30 min the solution is filtered over celite and it is extracted with DCM. The organic layers are collected, dried and the solvent is evaporated. The product is purified by column chromatography (silica gel, CH/EE (1/1)) to afford 570 mg of the product.

| | |
|---|---|
| $C_8H_6ClN_4$ | (M = 192.6 g/mol) |
| ESI-MS: | 193 $[M+H]^+$ |
| $R_t$ (HPLC): | 0.33 min (Method A) |

**Preparation of Final Compounds**

**Example 1:** 5-[6-({1-[5-(difluoromethyl)pyridin-2-yl]-4-methyl-1H-1,2,3-triazol-5-yl}methoxy)pyridazin-3-yl]-1-methyl-1,2-dihydropyridin-2-one

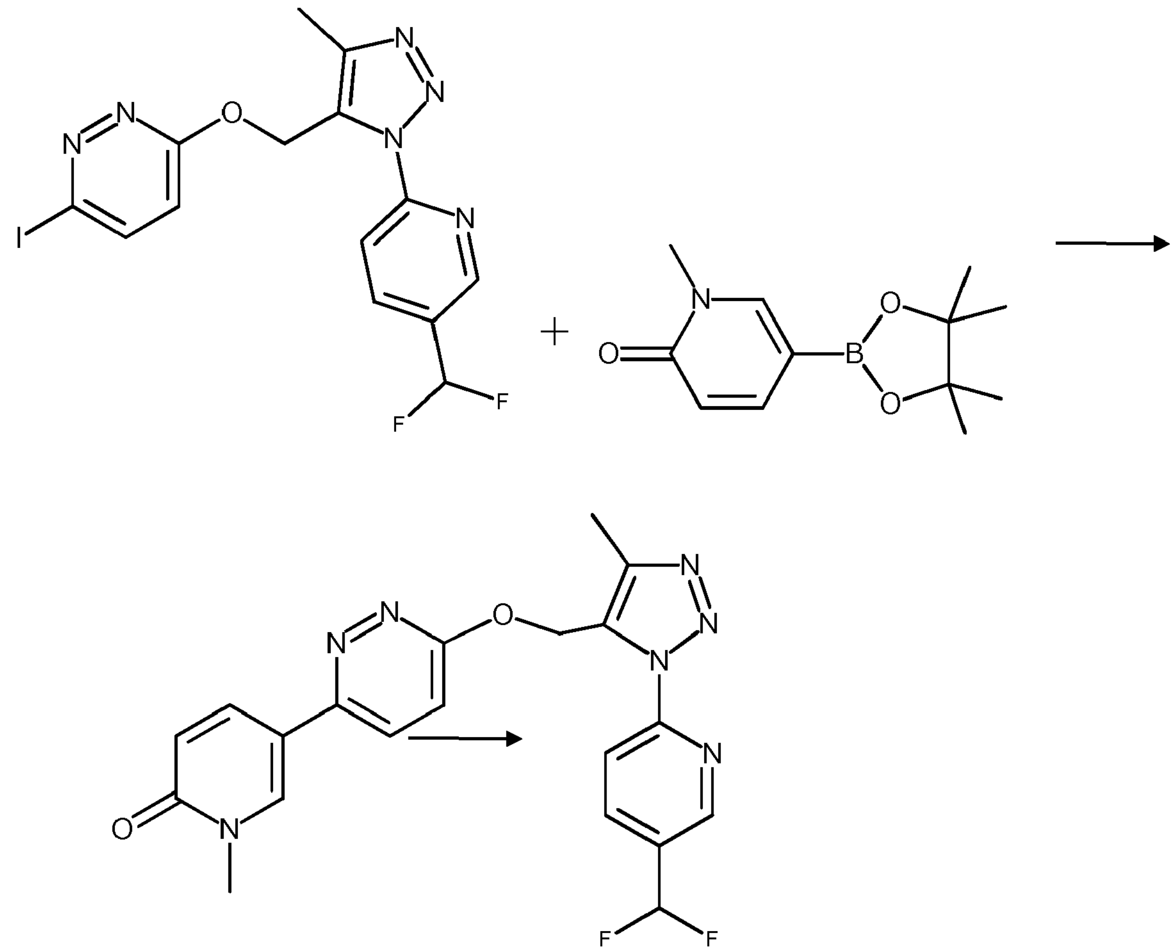

To 1-methyl-5-(4,4,5,5-tetramethyl-1,3,2-dioxaborolan-2-yl)-1,2-dihydropyridin-2-one (159 mg, 0.70 mmol) is added **example IX.1** (250 mg, 0.60 mmol) in 1.5 mL methanol, 3 mL 1,4 dioxane, 2M aq sodium carbonate solution (0.60 mL, 1.10 mmol) and Pd-PEPPSI (9.50 mg 0.01 mmol) under argon and the reaction mixture is stirred for 3 h at 100 °C. The mixture is purified by preparative HPLC to obtain 96.1 mg of the product.

| | |
|---|---|
| $C_{20}H_{17}F_2N_7O_2$ | (M = 425.4 g/mol) |
| ESI-MS: | 426 $[M+H]^+$ |
| $R_t$ (HPLC): | 0.58 min (Method D) |

$^1$H NMR (400 MHz, DMSO-$d_6$) δ ppm 2.41 - 2.46 (m, 3 H) 3.48 - 3.57 (m, 3 H) 5.96 - 6.08 (m, 2 H) 6.47 - 6.58 (m, 1 H) 7.21 (t, J = 56 Hz 1 H) 7.23 (s, 1 H) 7.95 - 8.06 (m, 1 H) 8.13 - 8.22 (m, 2 H) 8.29 - 8.38 (m, 1 H) 8.51 (d, *J*=2.66 Hz, 1 H) 8.65 - 8.71 (m, 1 H)

The following compounds are prepared according to the general procedure of **Example 1** described above:

| Ex. | Starting Material | | Structure |
|---|---|---|---|
| **2** | 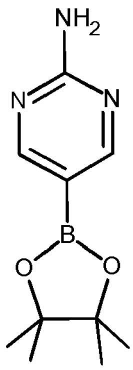 | **IX.1** | 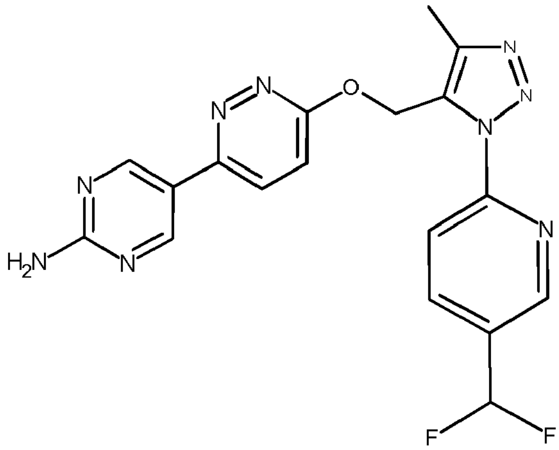 |
| **3** | 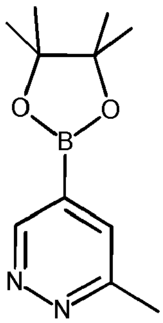 | **IX.1** | 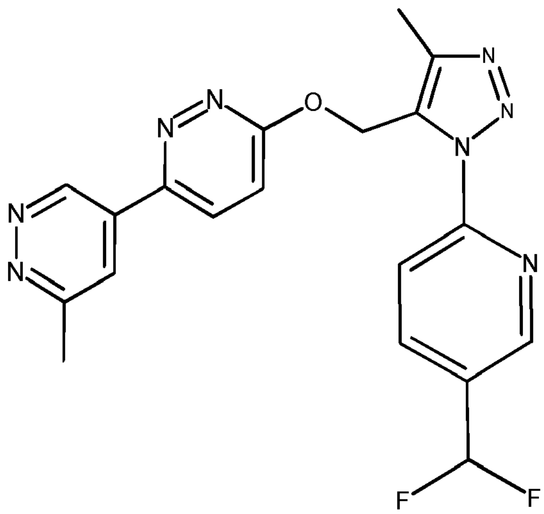 |
| **4** | 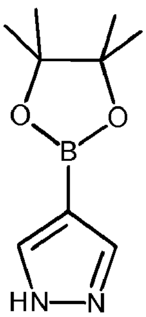 | **IX.1** | 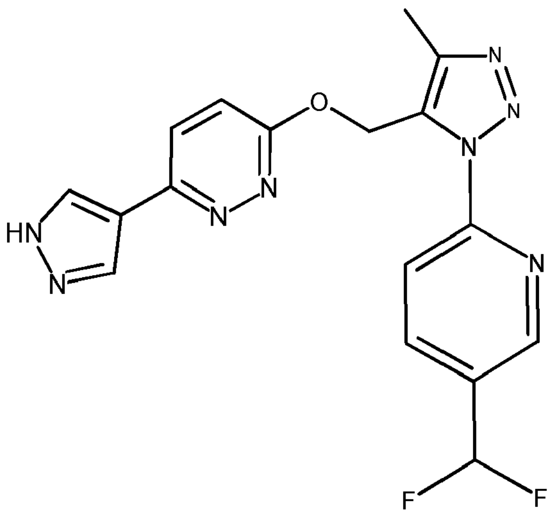 |

(continued)

| Ex. | Starting Material | | Structure |
|---|---|---|---|
| **5** | 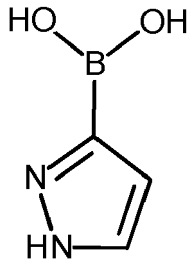 | **IX.1** | 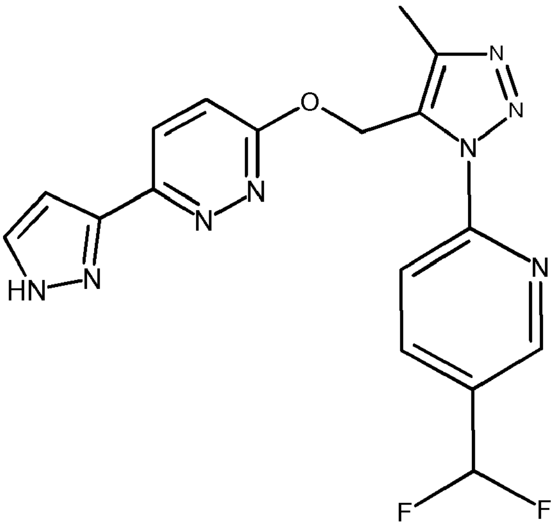 |
| **6** | 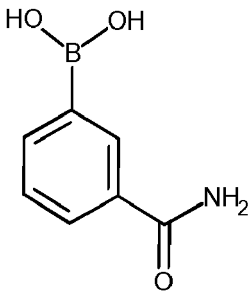 | **IX.1** | 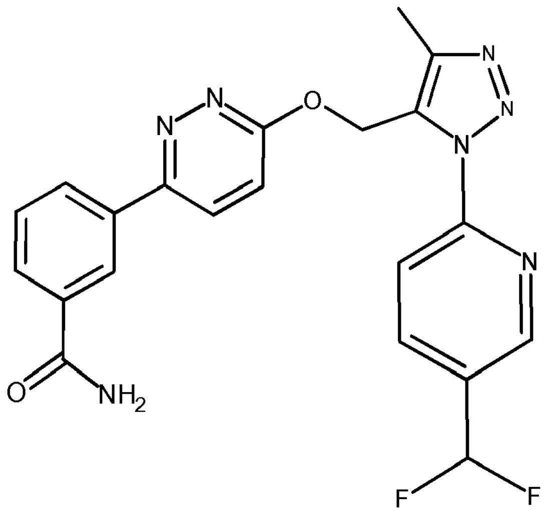 |
| **7** | 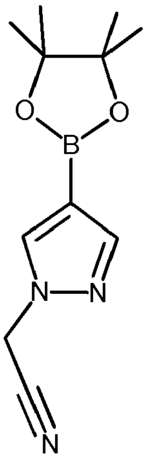 | **IX.1** | 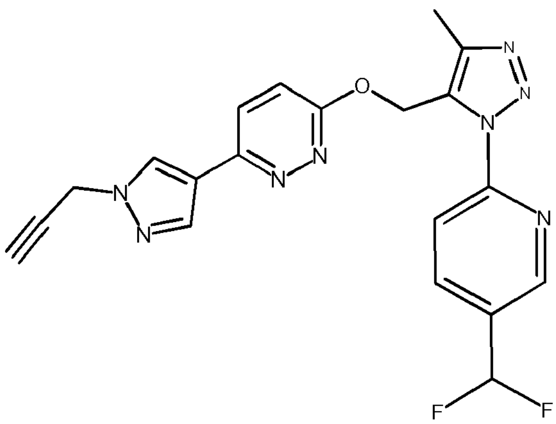 |

(continued)

| Ex. | Starting Material | | Structure |
|---|---|---|---|
| **8** | 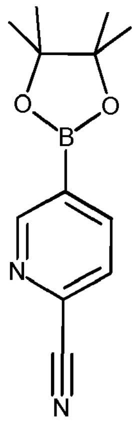 | **IX.1** | 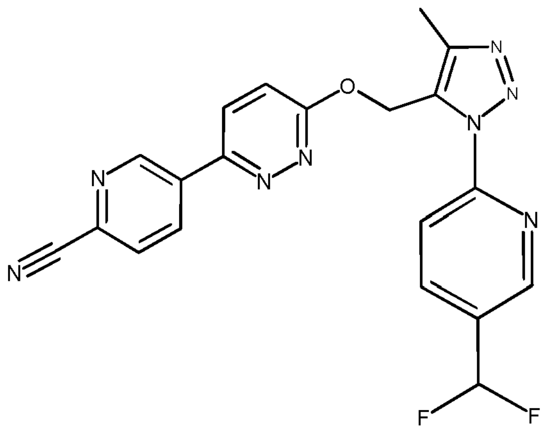 |
| **9** | 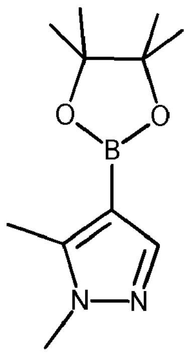 | **IX.1** | 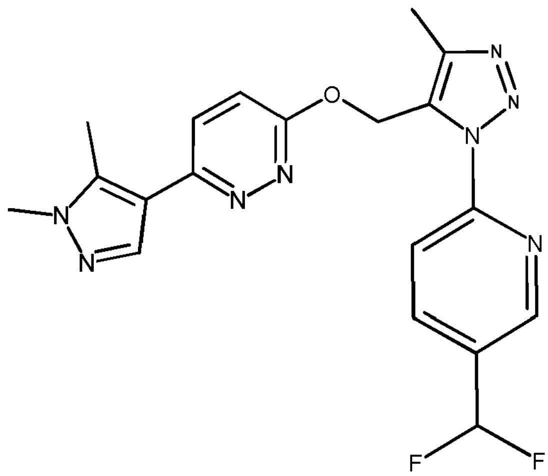 |
| **10** | 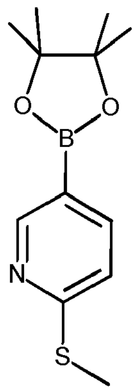 | **IX.1** | 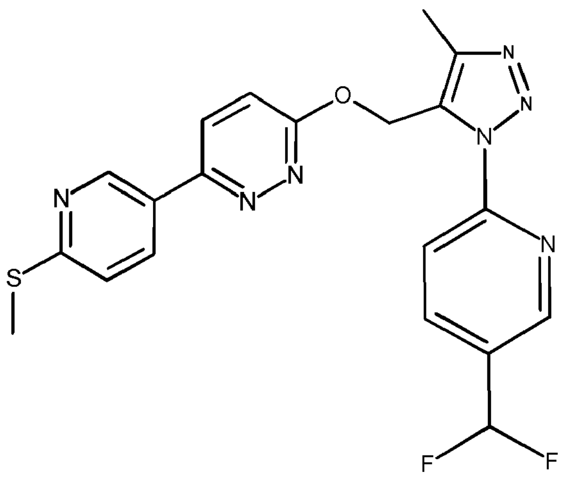 |

(continued)

| Ex. | Starting Material | | Structure |
|---|---|---|---|
| 11 | 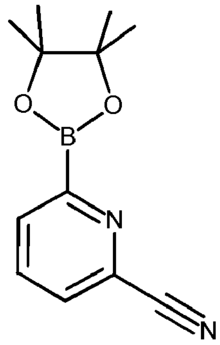 | IX.1 | 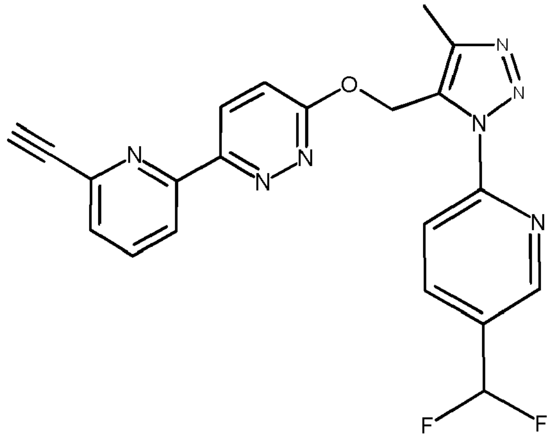 |
| 12 | 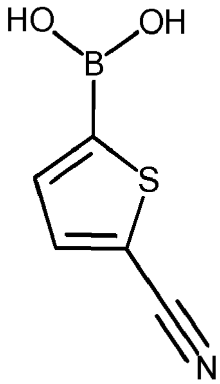 | IX.1 | 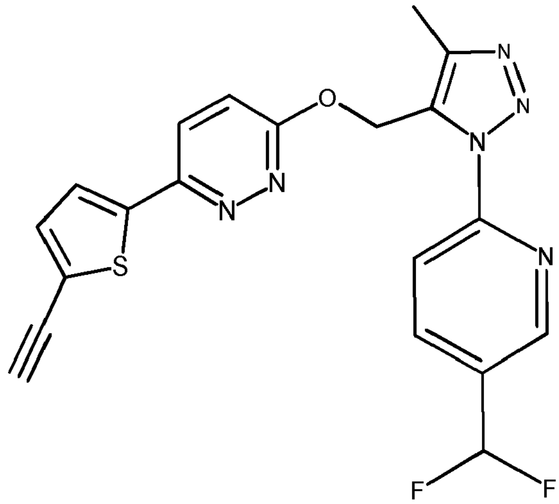 |
| 13 | 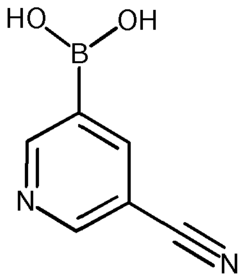 | IX.1 | 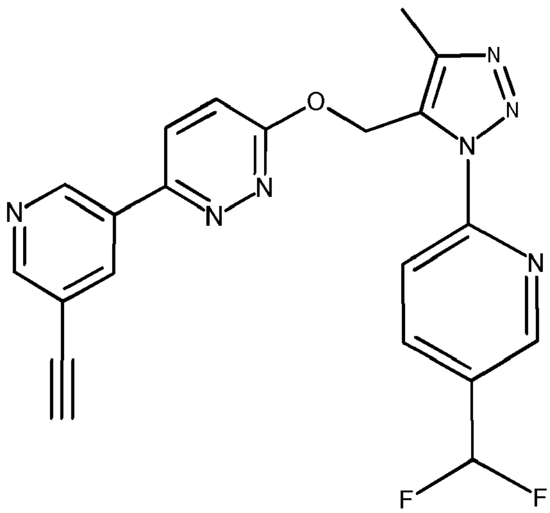 |

(continued)

| Ex. | Starting Material | | Structure |
|---|---|---|---|
| **14** | 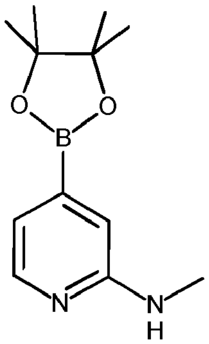 | **IX.1** | 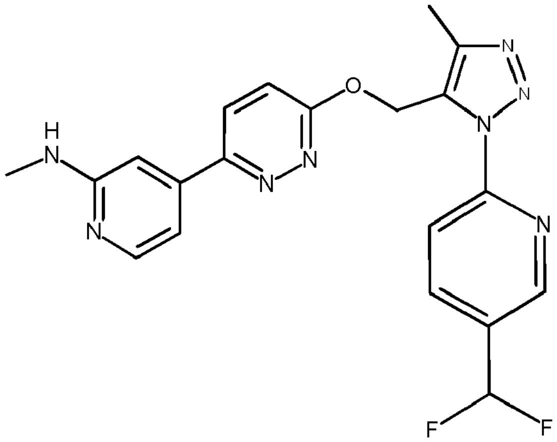 |
| **15** | 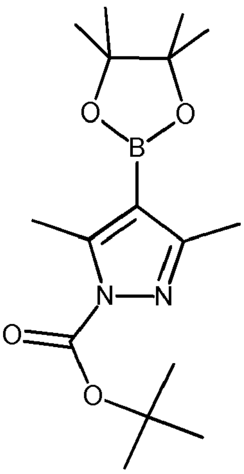 | **IX.1** | 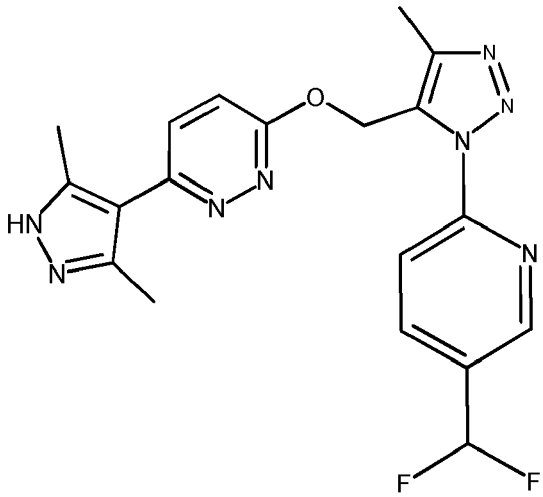 |
| **16** | 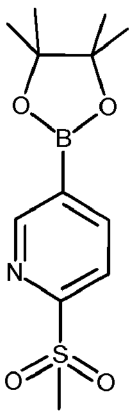 | **IX.1** | 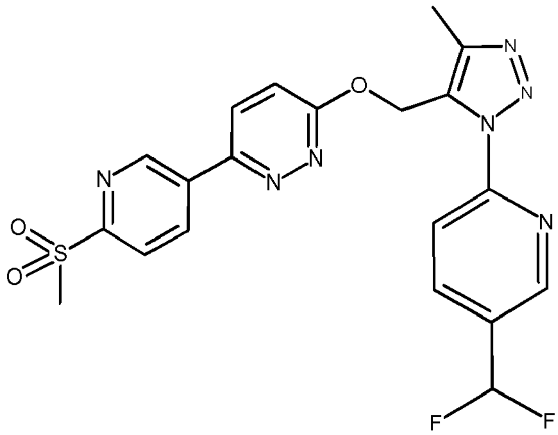 |

(continued)

| Ex. | Starting Material | | Structure |
|---|---|---|---|
| **17** | 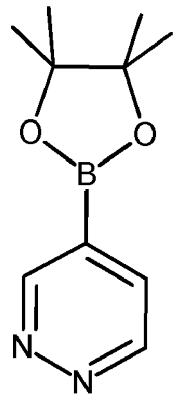 | **IX.1** | 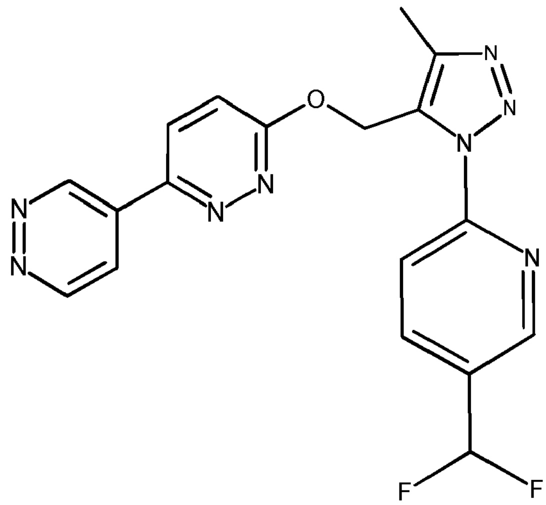 |
| **18** | 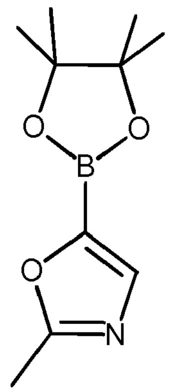 | **IX.1** | 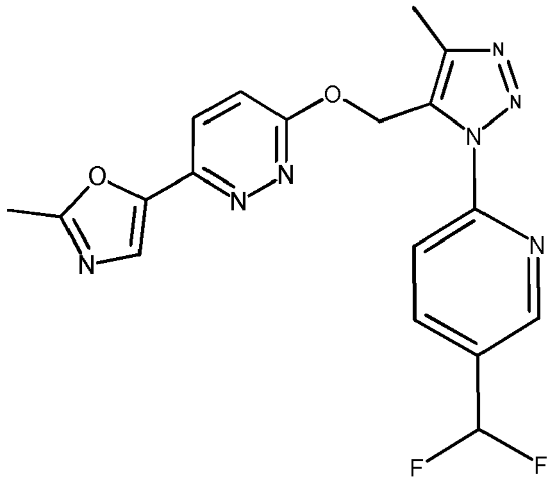 |
| **19** | 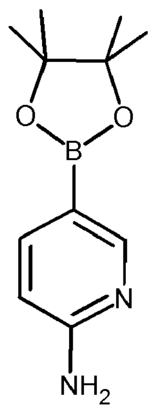 | **IX.1** | 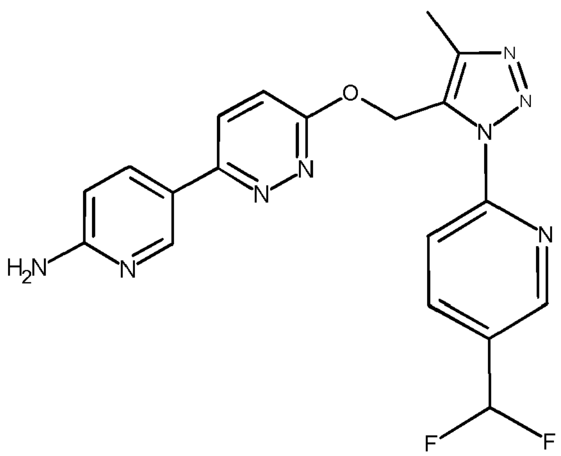 |

(continued)

| Ex. | Starting Material | | Structure |
|---|---|---|---|
| **20** | 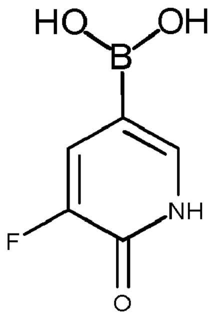 | **IX.1** | 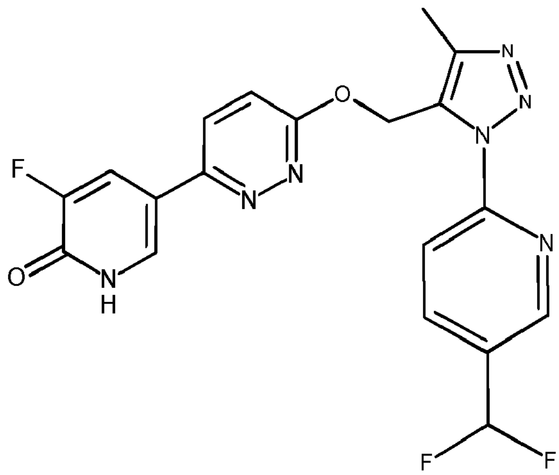 |
| **21** | 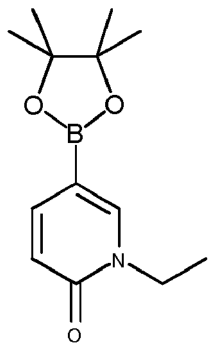 | **IX.1** | 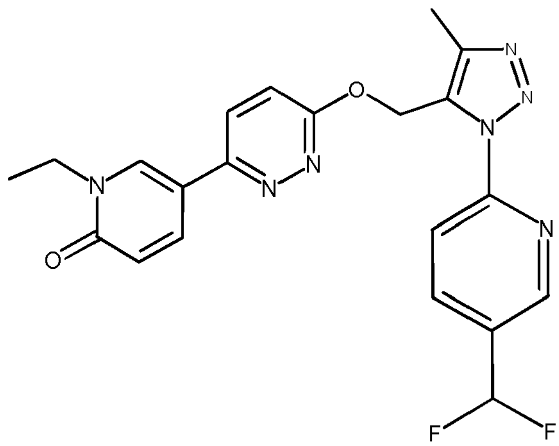 |
| **22** | 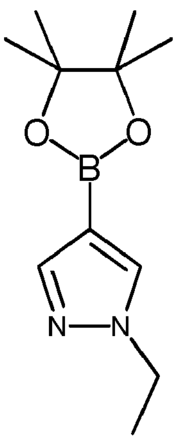 | **IX.3** | 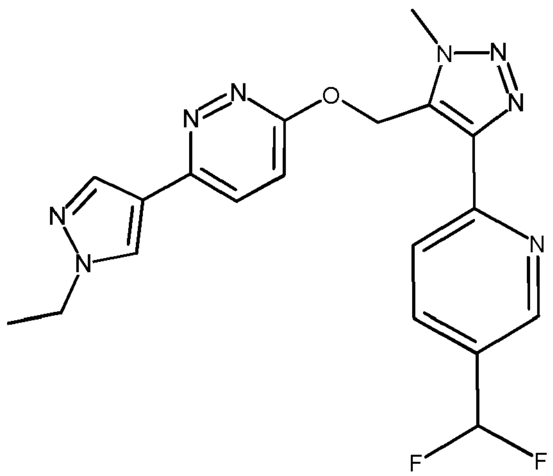 |

(continued)

| Ex. | Starting Material | | Structure |
|---|---|---|---|
| 23 | 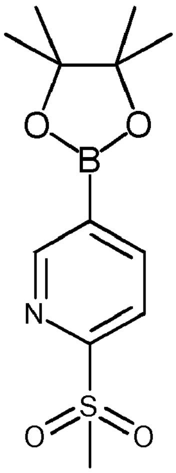 | IX.3 | 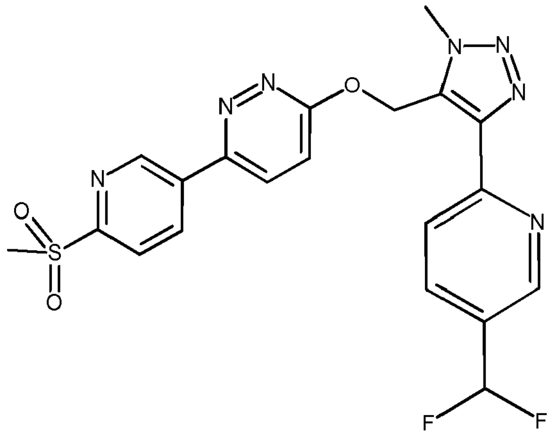 |
| 24 | 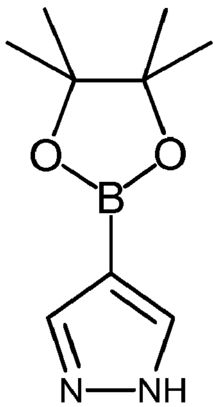 | IX.3 | 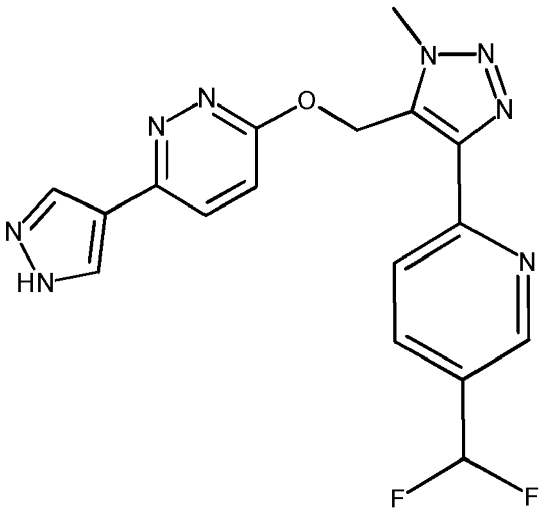 |
| 25 | 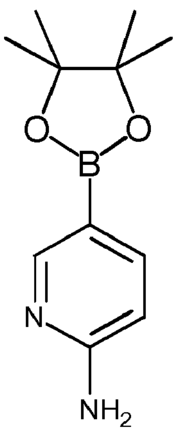 | IX.3 | 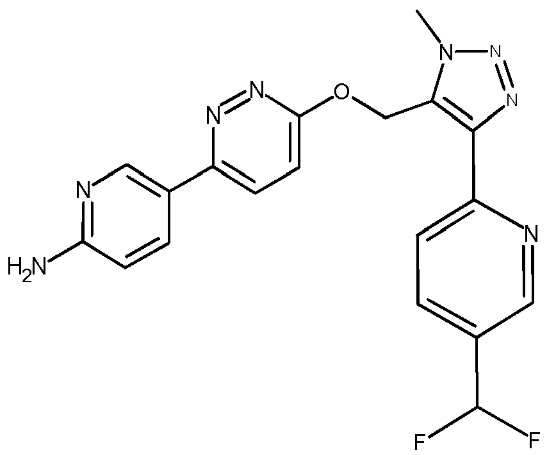 |

(continued)

| Ex. | Starting Material | | Structure |
|---|---|---|---|
| **26** | 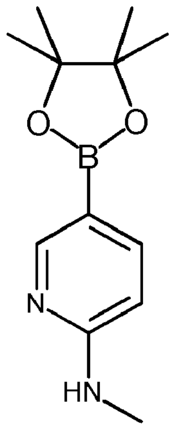 | **IX.3** | 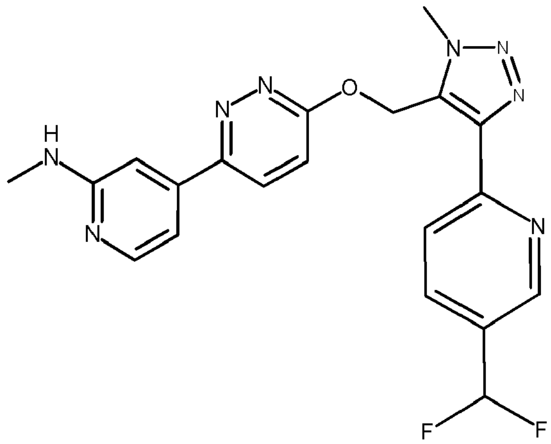 |
| **27** | 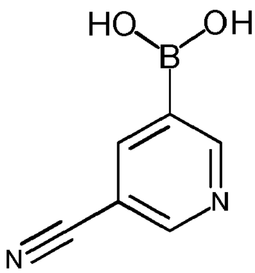 | **IX.3** | 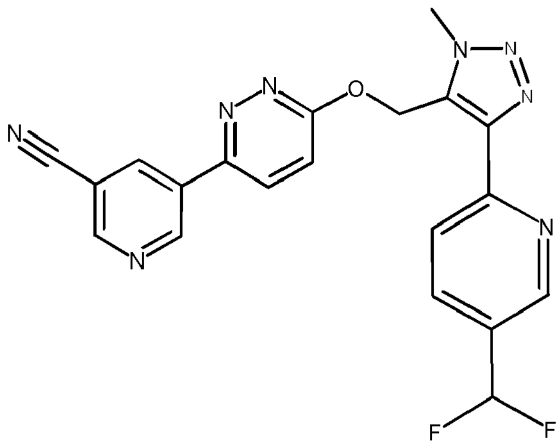 |
| **28** | 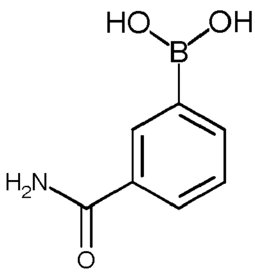 | **IX.3** | 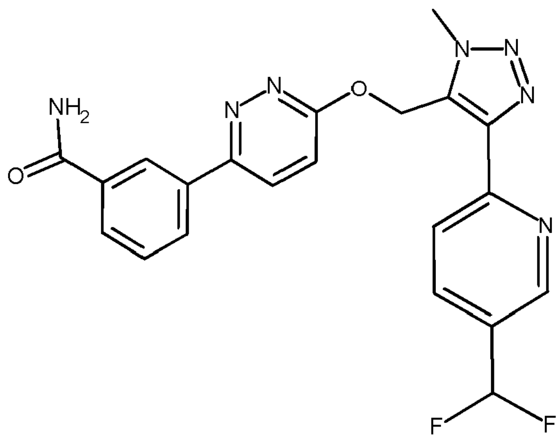 |

(continued)

| Ex. | Starting Material | | Structure |
|---|---|---|---|
| **29** | 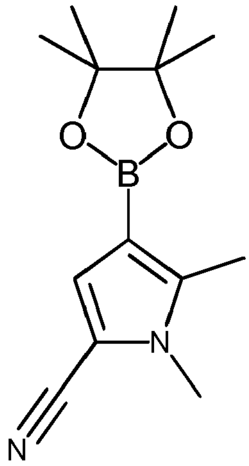 | **IX.4** | 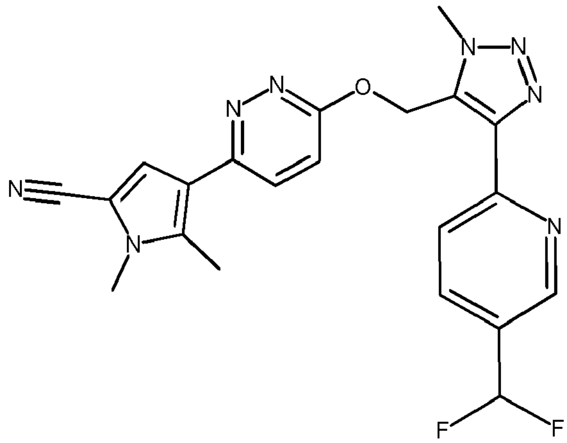 |
| **30** | 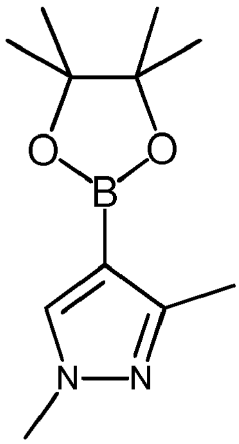 | **IX.4** | 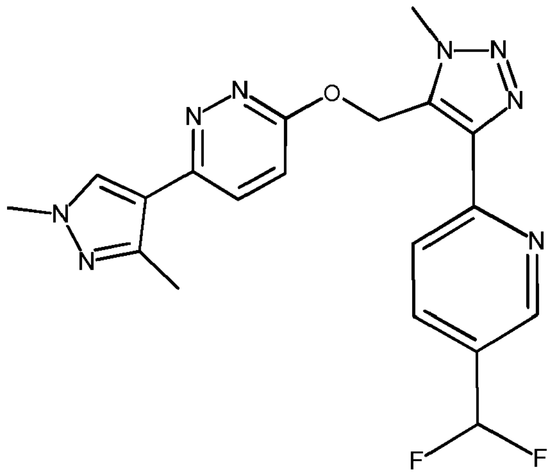 |
| **31** | 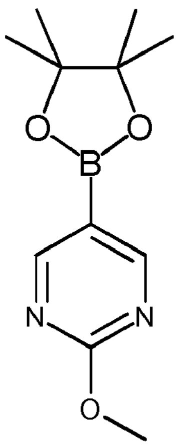 | **IX.4** | 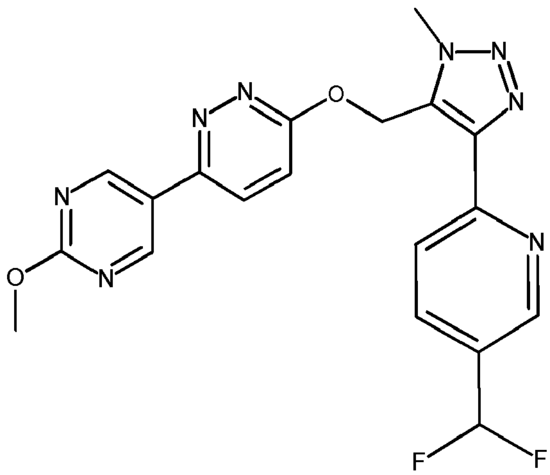 |

(continued)

| Ex. | Starting Material | | Structure |
|---|---|---|---|
| **32** | 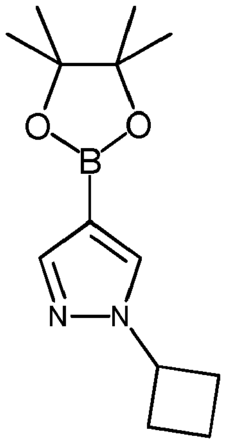 | **IX.4** | 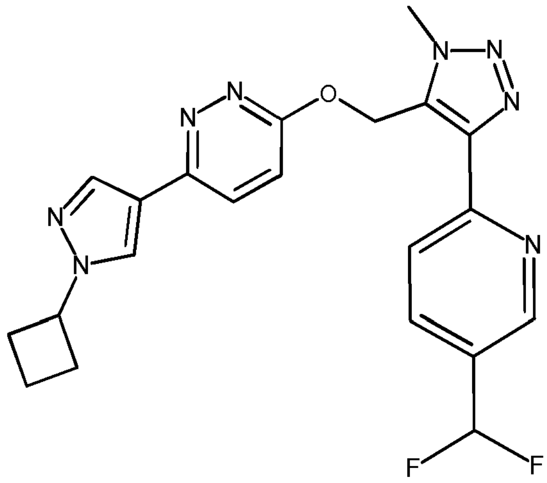 |
| **33** | 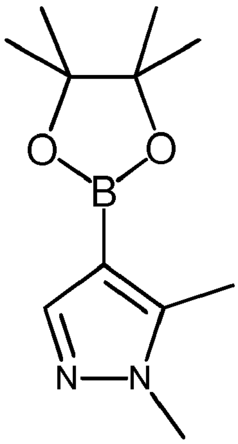 | **IX.4** | 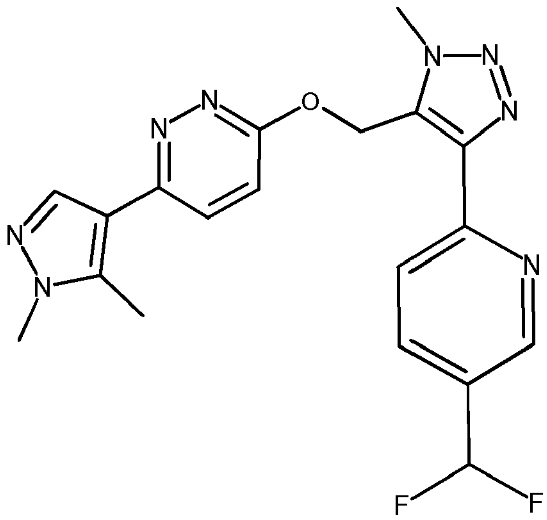 |
| **34** | 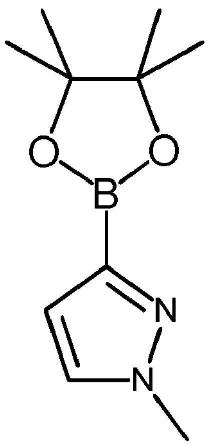 | **IX.4** | 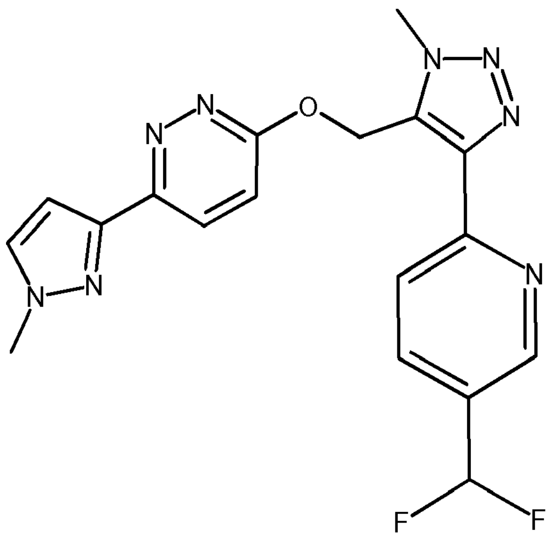 |

(continued)

| Ex. | Starting Material | | Structure |
|---|---|---|---|
| 35 | 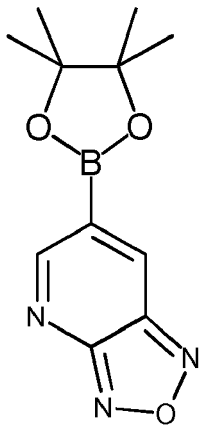 | IX.4 | 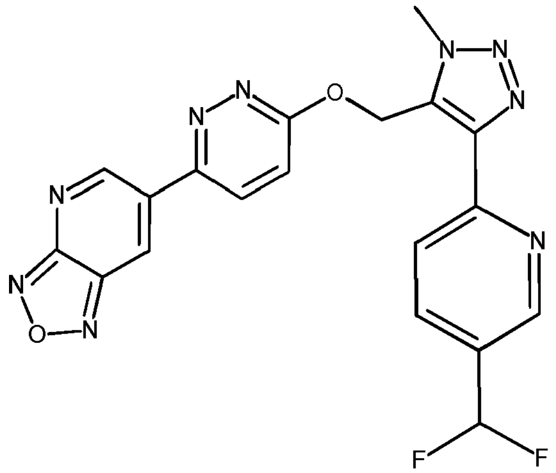 |

For the example compounds the reaction conditions in Table 2 were used.

Table 2: Reaction conditions for examples 1-35

| Example | Reaction conditions |
|---|---|
| 2 | 1.5eq borolane; 0.1eq dppf;2 mL Dioxane; 3eq base; work-up: filtrate over SPE-thiol cartridge and alox cartridge; purification by preparative HPLC |
| 3 | overnight 100 °C |
| 4 | 2.7eq base; 1.3eq borolane;<br>2 h 100°C |
| 5 | 2.7eq base; 1.3eq boronic acid;<br>2 h 100°C |
| 6 | 2.7eq base; 1.3eq boronic acid;<br>2 h 100°C |
| 7 | 2.7eq base; 1.3eq borolane;<br>2 h 100°C |
| 8 | 2.7eq base; 1,3eq borolane;<br>2 h 100°C |
| 9 | 2.7eq base; 1.3eq borolane;<br>2 h 100°C |
| 10 | 1.5eq borolane; 0.1eq dppf;2 mL Dioxane; 3eq base; work-up: filtrate over SPE-thiol cartridge and alox cartridge; purification by preparative HPLC |
| 11 | 1.5eq borolane; 0.1eq dppf;2 mL Dioxane; 3eq base; work-up: filtrate over SPE-thiol cartridge and alox cartridge; purification by preparative HPLC |
| 12 | 1.5eq boronic acid; 0.1eq dppf;2 mL Dioxane; 3eq base; work-up: filtrate over SPE-thiol cartridge and alox cartridge; purification by preparative HPLC |
| 13 | 1.5eq boronic acid; 0.1eq dppf;2 mL Dioxane; 3eq base; work-up: filtrate over SPE-thiol cartridge and alox cartridge; purification by preparative HPLC |
| 14 | 1.5eq borolane; 0.1eq dppf;2 mL Dioxane; 3eq base; work-up: filtrate over SPE-thiol cartridge and alox cartridge; purification by preparative HPLC |

(continued)

| Example | Reaction conditions |
|---|---|
| 15 | 1.5eq borolane; 0.1eq dppf;2 mL Dioxane; 3eq base; work-up: filtrate over SPE-thiol cartridge and alox cartridge; purification by preparative HPLC;<br>194eq TFA/DCM 1/1 +5%$H_2O$; 1h; RT; workup: evaporation; purification by preparative HPLC |
| 16 | Work up: evaporation; extracion DCM/$H_2O$; org. layer evaporation; recrystallization in EE |
| 17 | overnight 100°C |
| 18 | overnight 100°C |
| 19 | Workup: reaction mixture is diluted with MeOH, precipitatio; filtrate the crystalls; drying |
| 20 | Workup: reaction mixture is diluted with water, precipitatio; filtrate the crystalls; drying |
| 21 | 1.5eq borolane; 0.1eq dppf;2 mL Dioxane; 3eq base; work-up: filtrate over SPE-thiol cartridge and alox cartridge; purification by preparative HPLC |
| 22 | ligand: (dppf)PdCl2 |
| 23 | ligand:Pd-PEPPSI; workup: filtration of the precipitated crystalls |
| 24 | 3.5h; 110°C;<br>workup:evaporation;extraction DCM/water; purification by HPLC |
| 25 | ligand:Pd-PEPPSI; workup: filtration of the precipitated crystalls |
| 26 | 1,5eq borolane; 0,1eq dppf;2 mL Dioxane; 3eq base; work-up: filtrate over SPE-thiol cartridge and alox cartridge; purification by preparative HPLC |
| 27 | 1,5eq borolane; 0,1eq dppf;2 mL Dioxane; 3eq base; work-up: filtrate over SPE-thiol cartridge and alox cartridge; purification by preparative HPLC |
| 28 | ligand:Pd-PEPPSI; workup: filtration of the precipitated crystalls |
| 29 | 2.5 eq base; 1,2 eq borolane;<br>100 °C; overnight |
| 30 | 2.5 eq base; 1,2 eq borolane;<br>100 °C; overnight |
| 31 | 2.5 eq base; 1,2 eq borolane;<br>100 °C; overnight |
| 32 | 2.5 eq base; 1,2 eq borolane;<br>100 °C; overnight |
| 33 | 2.5 eq base; 1,2 eq borolane;<br>100 °C; overnight |
| 34 | 2.5 eq base; 1,2 eq borolane;<br>100 °C; overnight |
| 35 | 2.5 eq base; 1,2 eq borolane;<br>100 °C; overnight |

Example 36

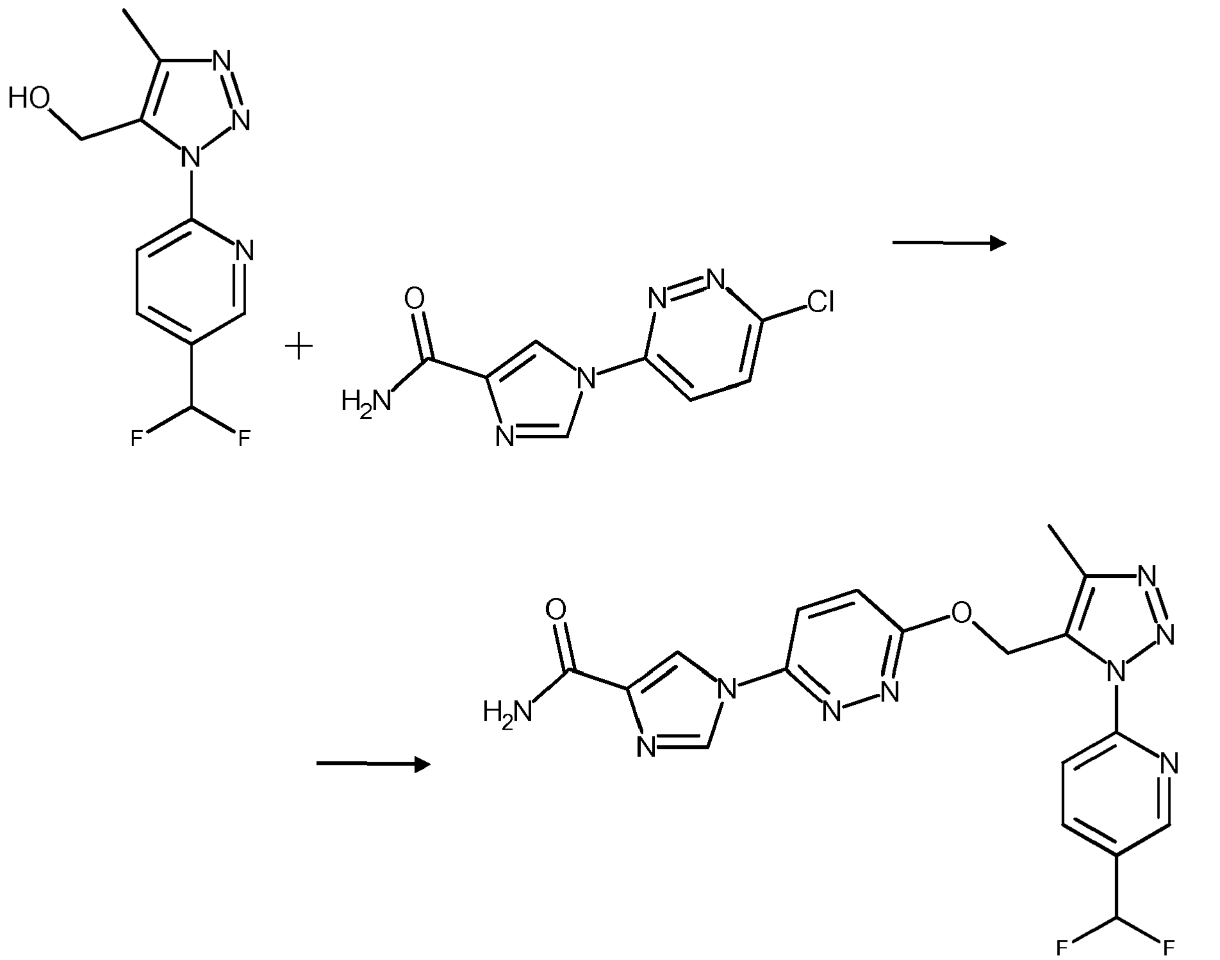

To **example I** (50.0 mg, 0.20 mmol) and example XIV (46.5 mg, 0.20 mmol) in 2 mL DMSO is added dropwise at 0 °C a solution of sodium *tert*-pentoxide 2 mol/L in Me-THF (83.9 µL, 0.20 mmol). The reaction mixture is stirred at RT overnight, then at 50°C overnight and then at 75°C for 3 days. The mixture is purified by preparative HPLC to obtain 3.20 mg of the product.

| | |
|---|---|
| $C_{18}H_{15}F_2N_9O_2$ | (M = 427.4 g/mol) |
| ESI-MS: | 428 $[M+H]^+$ |
| $R_t$ (HPLC): | 0.77 min (Method C) |

The following compounds are prepared according to the general procedure of **example 36** described above:

| | | | |
|---|---|---|---|
| 37 | 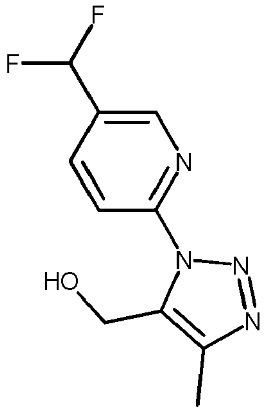 I | 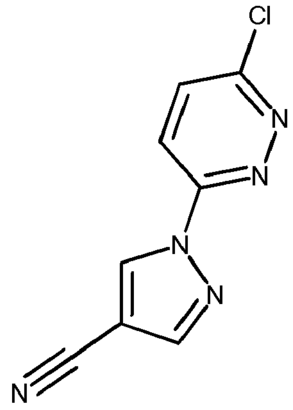 X | 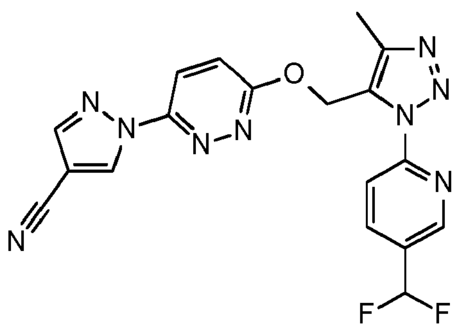 |
| 38 | 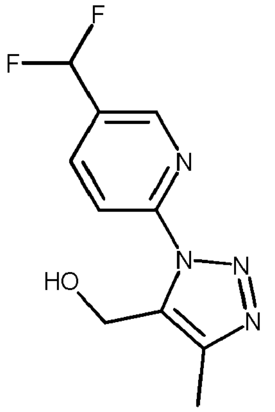 I | 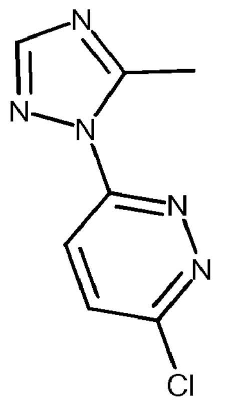 XI | 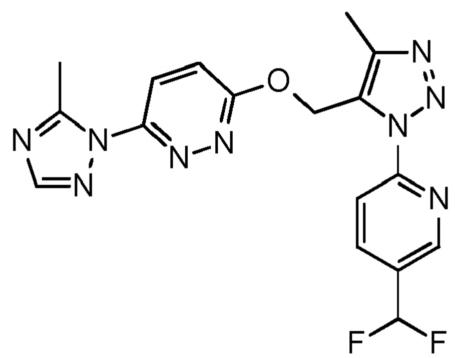 |
| 39 | 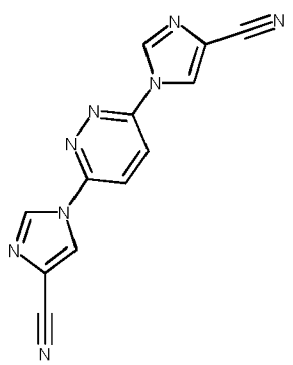 XV | 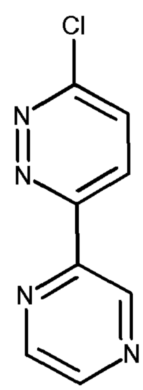 XVIII | 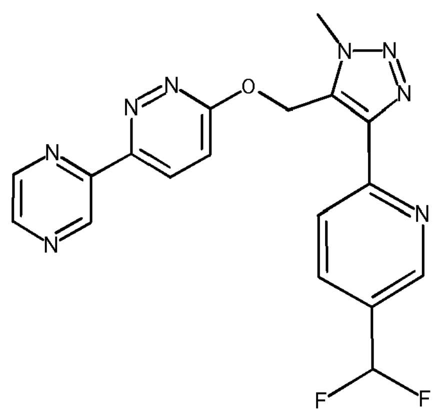 |

(continued)

| | | | |
|---|---|---|---|
| **40** | 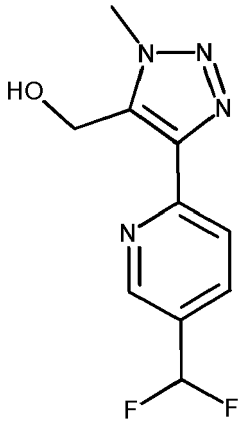 **VIII** | XII.3 | 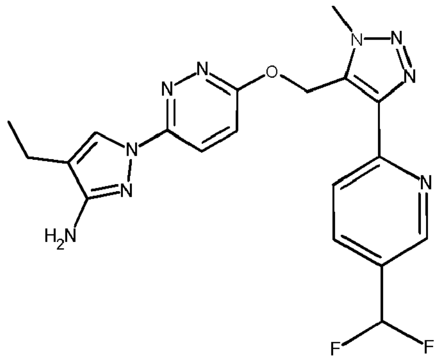 |
| **41** | 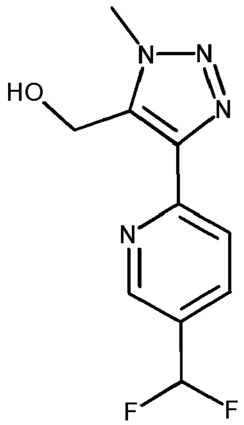 **VIII** | XII.2 | 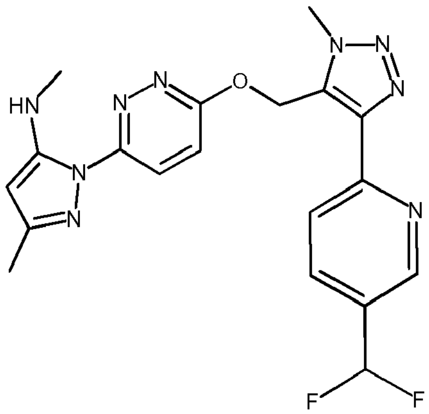 |

For the example compounds 37-41 the reaction conditions in the Table below were used.

Table of reaction conditions of example compounds 37-41

| # | Reaction conditions | ESI-MS | HPLC retention time [min] (method) / NMR |
|---|---|---|---|
| 37 | NMP; 1.3eq sodium-tert.-amylat, 30%ige Lösung in THF; 0°C 1h, RT overnight; Workup: adding water; filtration of the crystalls; drying in vacuum drying cabinet | 410 $[M+H]^+$ | 0.56 (method A) |
| 38 | Dioxane; 1.1eq sodium-tert-pentoxide; 90°C overnight;RT over weekend; Workup: adding water; filtration of the crystalls; drying in vacuum drying cabinet | 400 $[M+H]^+$ | 0.48 (method A) $^1$H NMR (400 MHz, DMSO-3939$d_6$) δ ppm 2.4406 (s, 3 H) 2.7341 (s, 3 H) 6.03 - 6.09 (m, 2 H) 7.22 (t, J = 56 Hz, 1H) 7.40 - 7.46 (m, 1 H) 7.96 - 8.07 (m, 1 H) 8.13 - 8.15 (m, 1 H) 8.16 - 8.23 (m, 1 H) 8.27 - 8.40 (m, 1 H) 8.62 - 8.73 (m, 1 H) |

(continued)

| # | Reaction conditions | ESI-MS | HPLC retention time [min] (method) / NMR |
|---|---|---|---|
| 39 | NMP; 1.3eq sodium-tert.-amylat, 30%ige Lösung in THF; 0°C 1h, RT overnight; Workup: adding water; filtration of the crystalls; drying in vacuum drying cabinet | 397 $[M+H]^+$ | 0.50 (method A) $^1H$ NMR (DMSO-$d_6$, 400 MHz): $\delta$ = 9.63 (d, $J$ = 1.4 Hz, 2H), 8.75-8.83 (m, 1H), 8.45 (d, $J$ = 9.3 Hz, 1H), 8.28 (d, $J$ = 7.9 Hz, 1H), 8.14 (dt, $J$ = 8.4, 1.0 Hz, 1H), 7.48 (d, $J$ = 9.3 Hz, 1H), 7.17 (t, J = 56 Hz, 1H), 6.86-9.72 (m, 1H), 6.28 (s, 2H), 4.23 ppm (s, 3H) |
| 40 | workup: directly purification by HPLC | 428 $[M+H]^+$ | 0.51 (method B) |
| 41 | workup: directly purification by HPLC | 428 $[M+H]^+$ | 0.84 (method D) |

**Example 42:** 1-[6-({1-[5-(difluoromethyl)pyridin-2-yl]-4-methyl-1H-1,2,3-triazol-5-yl}methoxy)pyridazin-3-yl]-1H-imidazole-4-carbonitrile

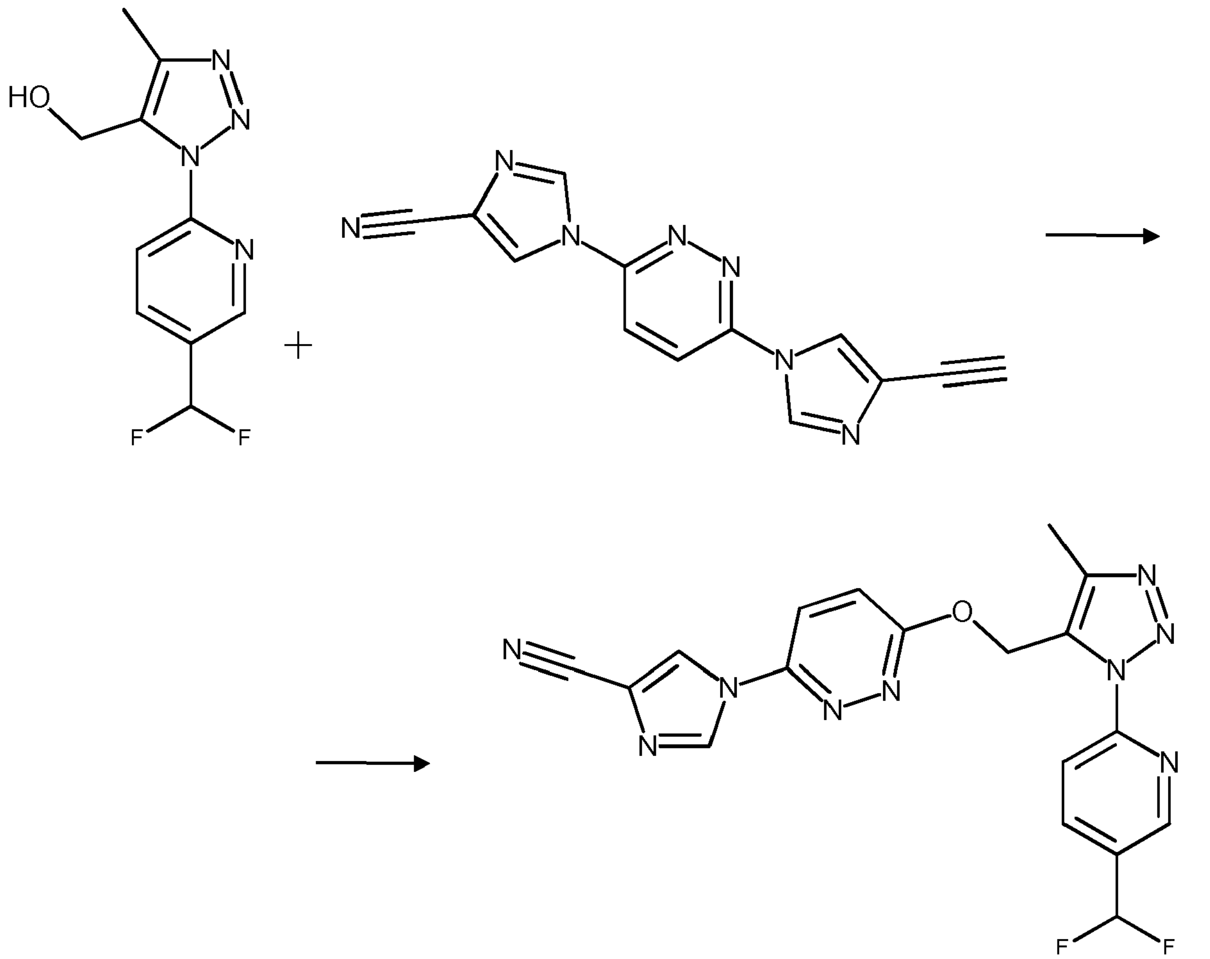

To **example I** (200 mg, 1.00 mmol) in 5 mL ACN are added cesium carbonate (814 mg, 2.00 mmol) and **example XV** (273 mg, 1.00 mmol) and the mixture is stirred overnight at 90 °C. The reaction mixture is quenched with water and the precipitation is filtered to get the crude product. The crude product is purified by silica gel column (CH/EE), recrystallization in MeOH/EE/Ether at the end give 151 mg of the product.

| | |
|---|---|
| $C_{18}H_{13}F_2N_9O$ | (M = 409.3 g/mol) |
| ESI-MS: | 410 $[M+H]^+$ |
| $R_t$ (HPLC): | 0.50 min (Method A) |

$^1H$ NMR (400 MHz, DMSO-$d_6$) $\delta$ ppm 2.34 - 2.48 (m, 3 H) 5.97 - 6.14 (m, 2 H) 7.22 (t, J = 56 Hz, 1H) 7.50 - 7.64 (m, 1 H) 8.16 - 8.19 (m, 1 H) 8.19 - 8.21 (m, 1 H) 8.31 - 8.43 (m, 1H) 8.67 (d, *J*=1.14 Hz, 1 H) 8.73 (d, *J*=1.27 Hz, 1 H) 8.90 - 8.95 (m, 1 H)

The following compounds 43-47 of the Table below are prepared according to the general procedure of **Example 42** described above:

Table of compounds 43-47

| Ex. | Starting materials | | Structure |
|---|---|---|---|
| **43** | 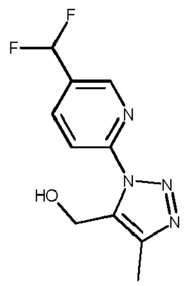 I | XVI.7 | 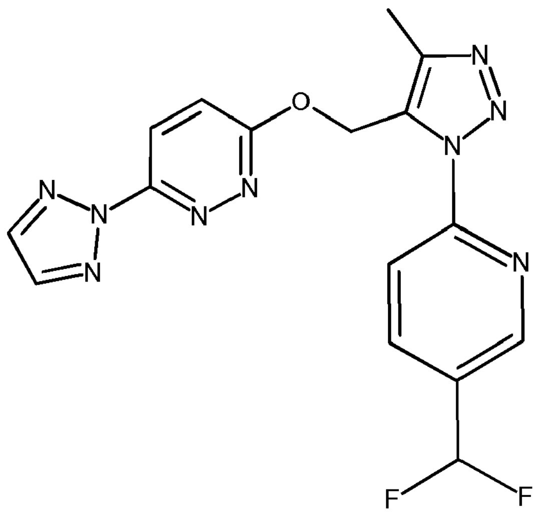 |
| **44** | 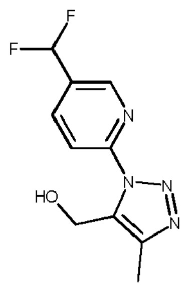 I | XVI.7 | 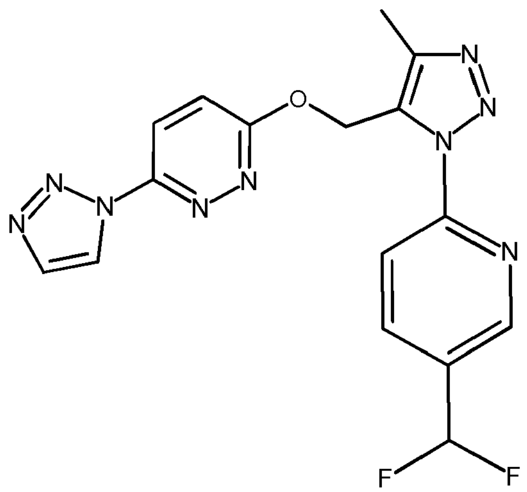 |

(continued)

| Ex. | Starting materials | | Structure |
|---|---|---|---|
| **45** | 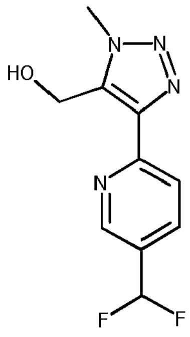<br>**VIII** | **XVI.5** | 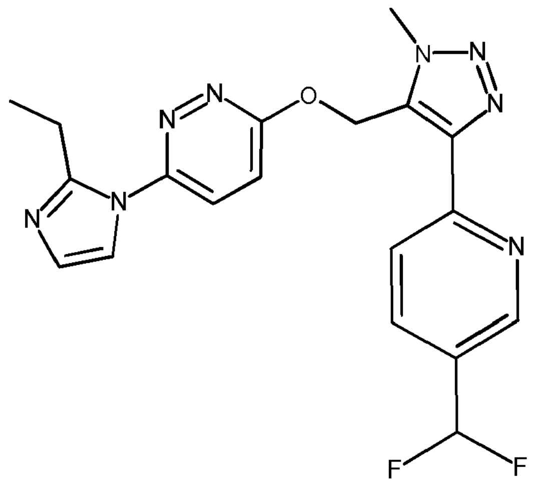 |
| **46** | 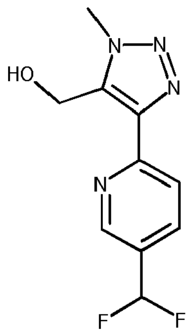<br>**VIII** | **XVI.6** | 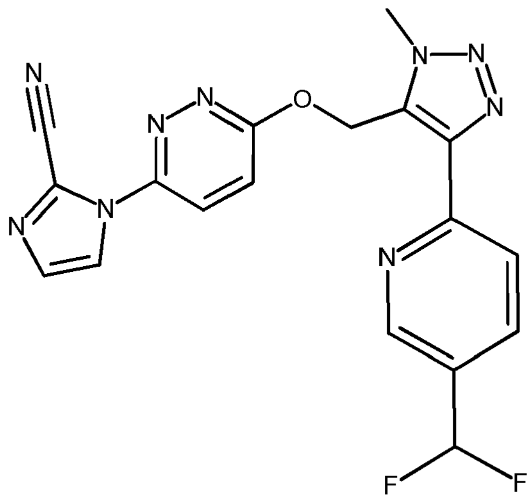 |
| **47** | 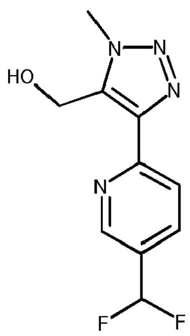<br>**VIII** | **XVI.4** | 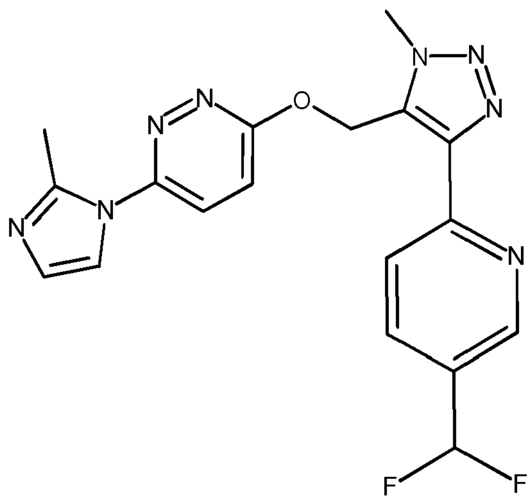 |

For the example compounds 43-47 the reaction conditions in the table below were used.

Table of reaction conditions of example compounds 43-47

| Ex. | Reaction conditions | ESI-MS | HPLC retention time [min] (method) / NMR |
|---|---|---|---|
| **43** | Workup: evaporation; extraction DCM/water; purification by silica gel column (CH/EE); chiral separation; purification by preparative HPLC | 386 $[M+H]^+$ | 0.73 (method A) $^1$H NMR (400 MHz, DMSO-$d_6$) δ ppm 2.47 (s, 3 H) 6.08 (s, 2 H) 7.22 (t, J = 56 Hz, 1H) 7.44 (d, *J*=9.38 Hz, 1 H) 8.19 (s, 1 H) 8.21 - 8.22 (m, 1 H) 8.25 (s, 2 H) 8.34 (dt, *J*=8.43, 1.17 Hz, 1 H) 8.68 (d, *J*=1.14 Hz, 1 H) |
| **44** | Workup: evaporation; BI01815149extraction DCM/water; purification by silica gel column (CH/EE); chiral separation | 386 $[M+H]^+$ | 0.73 (method A) $^1$H NMR (400 MHz, DMSO-$d_6$) δ ppm 2.46 (s, 3 H) 6.01 - 6.13 (m, 2 H) 7.22 (t, J = 56 Hz, 1H) 7.46 - 7.56 (m, 1 H) 8.06 (d, *J*=1.14 Hz, 1 H) 8.20 (d, *J*=8.49 Hz, 1 H) 8.34 (d, *J*=9.38 Hz, 2 H) 8.60 - 8.72 (m, 1 H) 8.92 - 9.02 (m, 1 H) |
| **45** | | 413 $[M+H]^+$ | 0.50 (method K) |
| **46** | Workup: purification by silica gel column (CH/EE); recrystallization in ether/MeOH 9/1 | 410 $[M+H]^+$ | 0.49 (method A) |
| **47** | Workup: evaporation; purification by HPLC | 399 $[M+H]^+$ | 0.47 (method K) |

**Example 48:** 3-({4-[5-(difluoromethyl)pyridin-2-yl]-1-methyl-1H-1,2,3-triazol-5-yl}methoxy)-6-(5-methyl-1H-1,2,4-triazol-1-yl)pyridazine

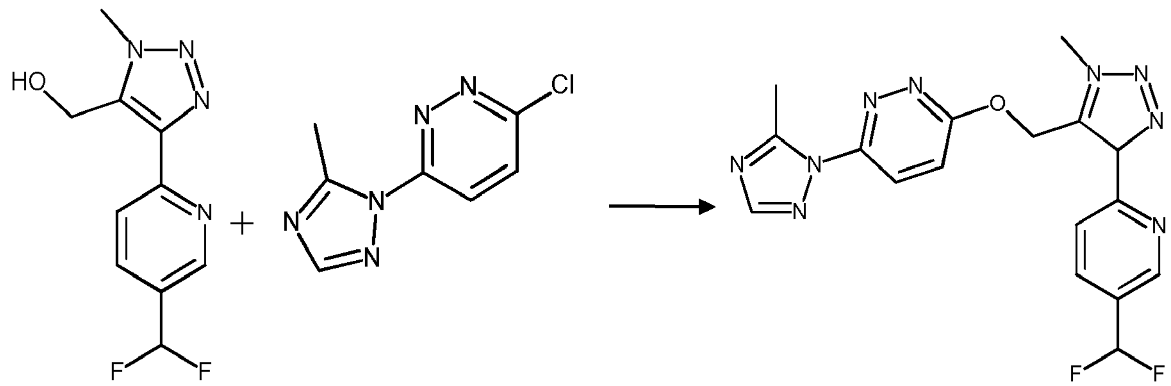

To **example VIII** (200 mg, 0.83 mmol) and **example XI** (163 mg, 0.83 mmol) in 10 mL Dioxan is added sodium *tert*-pentoxide (101 mg, 0.92 mmol). The reaction mixture is stirred at 90°C overnight, then at RT over the weekend. The reaction mixture is diluted with water and the precipitate is filtered. The crude solid is recrystallized in MeOH to to obtain 212 mg of the product.

| | |
|---|---|
| $C_{17}H_{15}F_2N_9O$ | (M = 399.4 g/mol) |
| ESI-MS: | 400 $[M+H]^+$ |
| $R_t$ (HPLC): | 0.46 min (Method A) |

The following compounds 49 and 50 are prepared according to the general procedure of **example 48** described above:

| Ex. | Starting materials | | Structure |
|---|---|---|---|
| **49** | 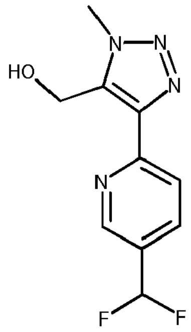 VIII | 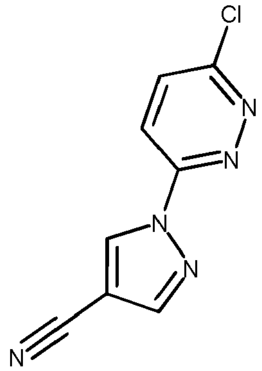 X.1 | 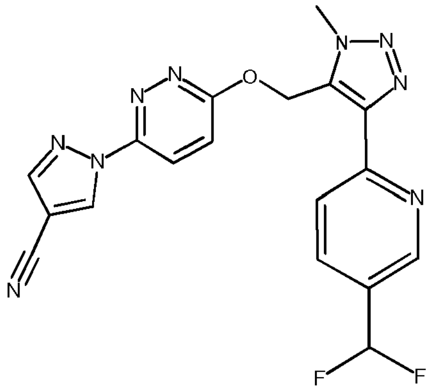 |
| **50** | 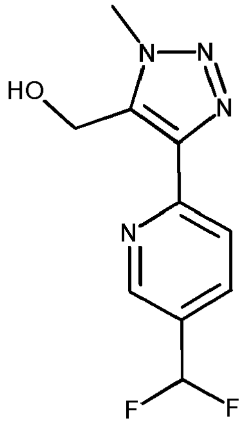 VIII | 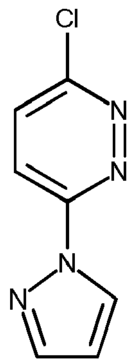 | 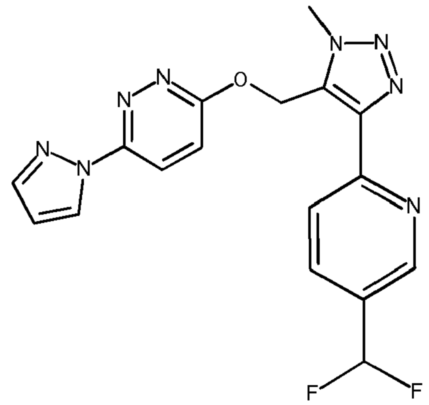 |

For the example compounds 49-50 the reaction conditions in the Table below were used.

Table of reaction conditions of example compounds 49-50

| Ex. | Reaction conditions | ESI-MS | HPLC retention time [min] (method) |
|---|---|---|---|
| **49** | RT; overnight; workup: direct purification by HPLC | 410 $[M+H]^+$ | 0.96 (method G) |
| **50** | RT; 2h; workup: direct purification by HPLC | 385 $[M+H]^+$ | 0.73 (method H) |

**Example 51:** 3-(4-chloro-1H-pyrazol-1-yl)-6-({4-[5-(difluoromethyl)pyridin-2-yl]-1-methyl-1H-1,2,3-triazol-5-yl}methoxy)pyridazine

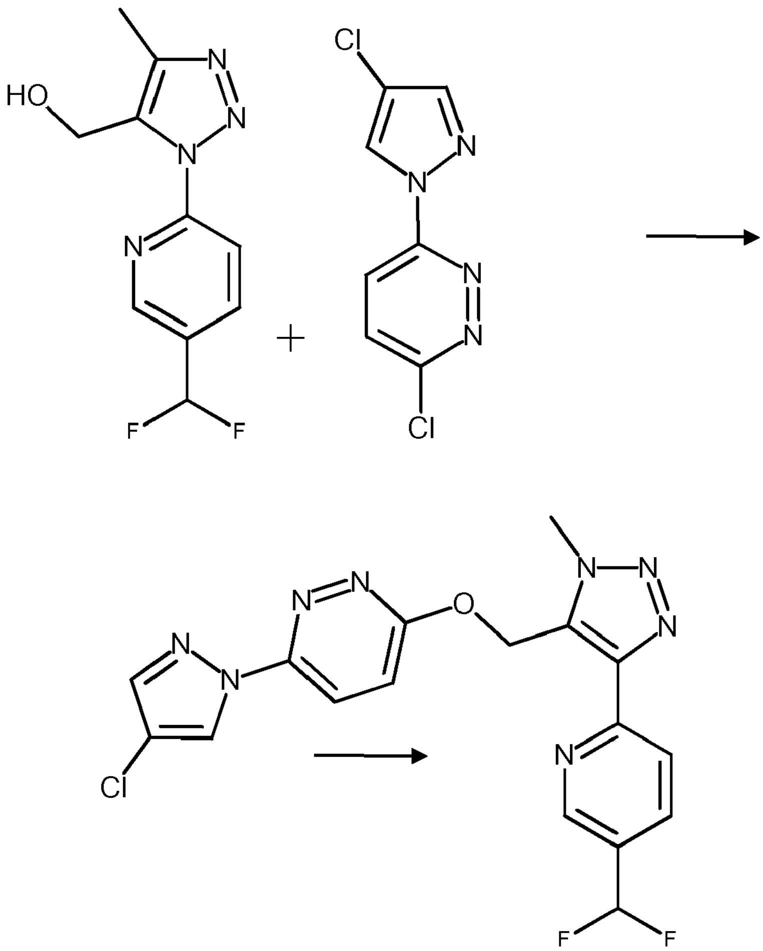

To **example VIII** (100 mg, 0.42 mmol) and **example XVI.1** (89.5 mg, 0.42 mmol) in 2 mL DCM is added sodium hydride (21.8 mg, 0.50 mmol). The reaction mixture is stirred at RT overnight. The reaction mixture is directly purified by preparative HPLC to obtain 41.6 mg of the product.

| | |
|---|---|
| $C_{17}H_{13}ClF_2N_8O$ | (M = 418.8 g/mol) |
| ESI-MS: | 397 $[M+H]^+$ |
| $R_t$ (HPLC): | 0.86 min (Method F) |

The following compounds are prepared according to the general procedure **(example 51)** described above:

| Ex. | Starting materials | | Structure |
|---|---|---|---|
| **52** | 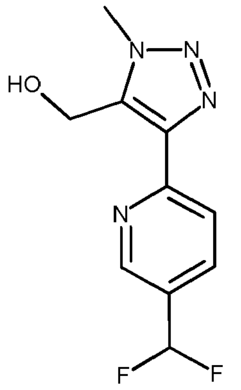 **VIII** | 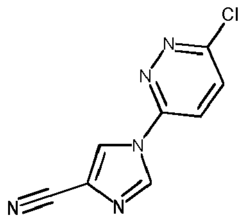 **X.2** | 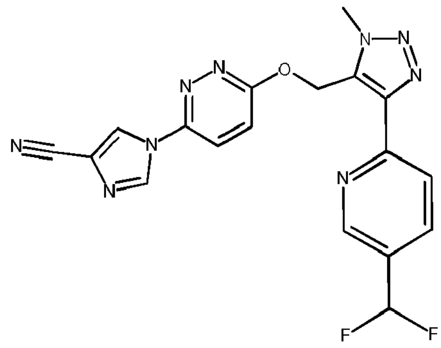 |
| **53** | 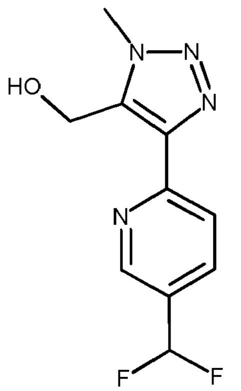 **VIII** | 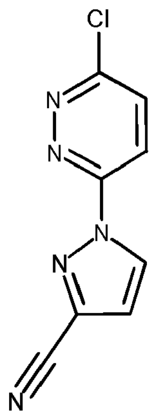 **X.3** | 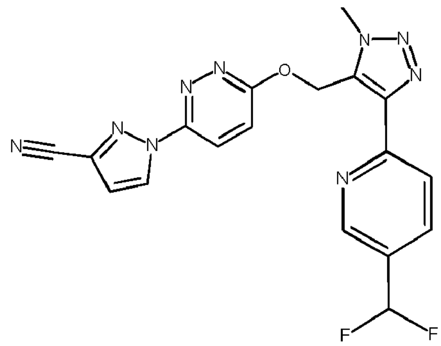 |

For the example compounds 52-53 the reaction conditions in the Table below were used.

Table of reaction conditions of example compounds 52-53

| Ex. | Reaction conditions | ESI-MS | HPLC retention time [min] (method) NMR |
|---|---|---|---|
| **52** | workup: directly purification by column chromatography (silica gel, CH/EE (1/1)) | 410 $[M+H]^+$ | 0.70 (method B) $^1$H NMR (DMSO-$d_6$, 400 MHz): $\delta$ = 8.95 (d, *J* = 1.3 Hz, 1H), 8.79 (d, *J* = 1.0 Hz, 1H), 8.76 (d, *J* = 1.3 Hz, 1H), 8.27 (d, *J* = 8.2 Hz, 1H), 8.23 (d, *J* = 9.4 Hz, 1H), 8.14 (dd, *J* = 8.3, 1.1 Hz, 1H), 7.65 (d, *J* = 9.4 Hz, 1H), 7.16 (t, J = 56 Hz, 1H), 6.22 (s, 2H), 4.21 ppm (s, 3H) |
| **53** | workup: extraction DCM/water; purification by column chromatography (silica gel, DCM/MeOH (100/0 to 93/7)); purification by preparative HPLC | 410 $[M+H]^+$ | 0.86 (method A) $^1$H NMR (DMSO-$d_6$, 400 MHz): $\delta$ = 8.97 (d, *J* = 2.8 Hz, 1H), 8.79 (s, 1H), 8.26-8.31 (m, 1H), 8.25 (s, 1H), 8.14 (br d, *J* = 8.0 Hz, 1H), 7.59 (d, *J* = 9.4 Hz, 1H), 7.35 (d, *J* = 2.7 Hz, 1H), ), 7.16 (t, J = 56 Hz, 1H) 6.23 (s, 2H), 4.21 ppm (s, 3H) |

**Example 54:** 3-({1-[5-(difluoromethyl)pyridin-2-yl]-4-methyl-1H-1,2,3-triazol-5-yl}methoxy)-6-(4-fluoro-1H-pyrazol-1-yl)pyridazine

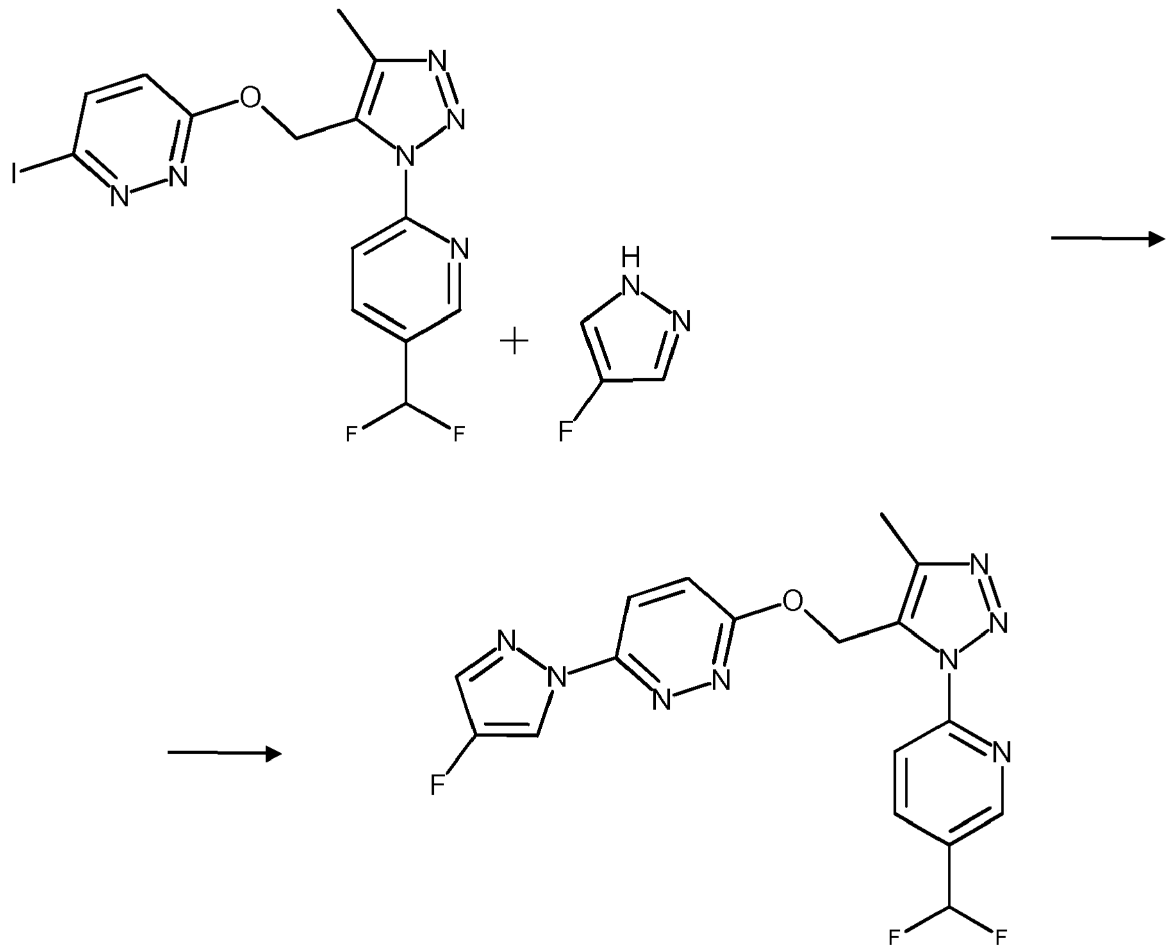

To 4-Fluoro-1H-pyrazole (6.00 mg, 0.10 mmol) in 1 mL dioxane is added **example IX.1** (30.0 mg, 0.10 mmol), copper(I)iodide (5.10 mg, 0.03 mmol), potassium phosphate (57.3 mg, 0.30 mmol) and (1R,2R)-N,N'-Dimethyl-1,2-cyclohexanediamine (8.50 µL, 0.05 mmol) under argon. Then 100 µL 25% ammonia is added and the mixture is stirred another 15 min.The reaction mixture is filtered over alox cartridge and SPE-thiol cartridge and then it is purified by preparative HPLC to obtain 20.2 mg of the product.

| | |
|---|---|
| $C_{17}H_{13}F_3N_8O$ | (M = 402.3 g/mol) |
| ESI-MS: | 403 $[M+H]^+$ |
| $R_t$ (HPLC): | 0,84 min (Method F) |

The following compound 55 is prepared according to the general procedure of **Example 54** described above:

| Ex. | Starting materials | | Structure |
|---|---|---|---|
| **55** | 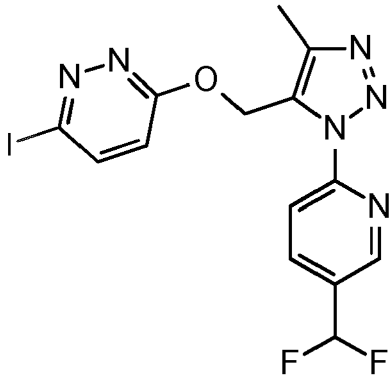 **IX.1** | 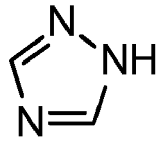 | 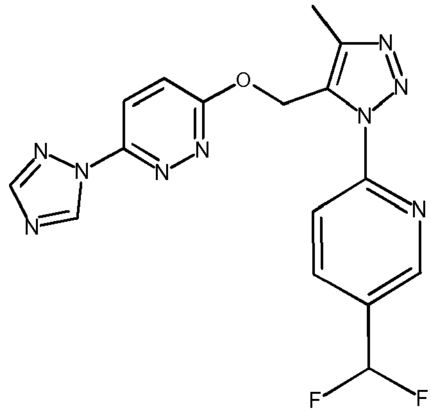 |

For the example compound 55 the following reaction conditions were used.

| Ex. | Reaction conditions | ESI-MS | HPLC retention time [min] (method) |
|---|---|---|---|
| **55** | overnight 120°C | 386 $[M+H]^+$ | 0.94 (method A) |

**Example 56:** 3-({1-[5-(difluoromethyl)pyridin-2-yl]-4-methyl-1H-1,2,3-triazol-5-yl}methoxy)-6-(pyrazin-2-yl)pyridazine

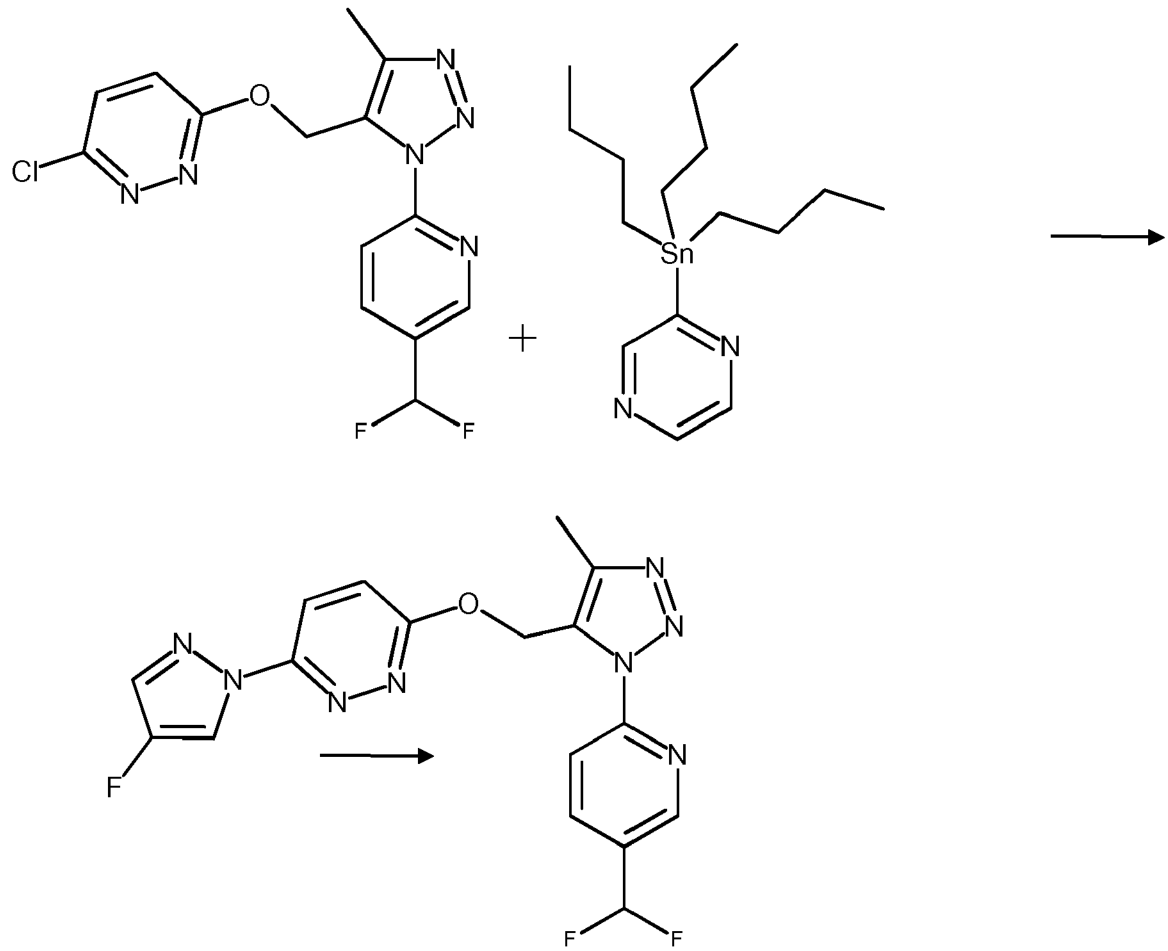

To 2-Tributylstannylpyrazine (83.0 mg, 0.23 mmol) in 2 mL dioxane is added to **example IX.2** (53.0 mg, 0.15 mmol), cesium fluoride (46.0 mg, 0.30 mmol) and xphos (26.0 mg, 0.03 mmol) under argon. The reaction mixture is purified by preparative HPLC to obtain 17.0 mg of the product.

| | |
|---|---|
| $C_{18}H_{14}F_2N_8O$ | (M = 396.3 g/mol) |
| ESI-MS: | 397 $[M+H]^+$ |
| $R_t$ (HPLC): | 0,92 min (Method G) |

$^1$H NMR (400 MHz, DMSO-$d_6$) δ ppm 2.47 (s, 3 H) 6.11 (s, 2 H) 7.22 (t, J = 56 Hz, 1H) 7.35 (d, *J*=9.25 Hz, 1 H) 8.20 (d, *J*=8.49 Hz, 1 H) 8.32 - 8.37 (m, 1 H) 8.39 (d, *J*=9.25 Hz, 1 H) 8.67 (d, *J*=1.14 Hz, 1 H) 8.74 - 8.84 (m, 2 H) 9.55 - 9.66 (m, 1 H)

**Biological Examples**

**Assay A: In vitro inhibition of $^3$H-flumazenil ($^3$H-Ro 15-1788) binding HEK cells expressing the human $GABA_A$ $\alpha_5\beta_3\gamma_{2s}$ receptor**

The benzodiazepine modulator unit can selectively be labelled with the antagonist $^3$H-flumazenil.

The affinity of $^3$H-flumazenil for different subunit combinations have been reported to be 1.0nM, 1.1nM, 1.5nM and 0.4nM for $\alpha_1\beta_2\gamma_2$; $\alpha_2B_2\gamma_2$; $\alpha_3\beta_2\gamma_2$ and $\alpha_5\beta_2\gamma_{2s}$ receptors, respectively, and 107 nM and 90 nM for $\alpha_4\beta_2\gamma_2$ and $\alpha_6\beta_2\gamma_2$ receptors (see Sieghart; Pharmacol. Rev. 1995 47 181-234).

The pharmacology of the mutated $\alpha_5\beta_3\gamma_{2s}$ $GABA_A$ receptor is similar to that of the wild type receptor with respect 3H-flumazenil binding.

## Cell cultures and membrane preparation

HEK-293 cell lines with stable expression of recombinant human $GABA_A$ $\alpha_5\beta_3\gamma_{2s}$ receptors (plasmid H46/E9/B10) are seeded in T175 polystyrene flasks or roller bottles (1700 $cm^2$, Fisher Scientific CCI-431191), and cultured (37°C, 5% $CO_2$) in Dulbecco's Modified Eagle Medium (DMEM) with GlutaMAX™ supplemented with 10% fetal bovine serum and one or both of the following antibiotics: hygromycin B (50 pg/ml; $\gamma_2$ subunit) or G418 (0.5 mg/ml; $\Omega$5 subunit).

When the cultures reach confluency, the DMEM is removed and the cells are washed (10 ml for T175 flasks; 50 ml for roller bottles) once in Dulbecco's Phosphate Buffered Saline (DPBS). Following addition of DPBS to the cultures (10 ml for T175 flasks; 100 ml for roller bottles) for approximately 5 min cells are easily detached from the surface by shaking or tapping the flask gently. The cell suspension is transferred to Falcon tubes and centrifuged at 23,500 x g for 10 min at 2°C. The pellet is washed once in 15 ml Tris-citrate buffer (50 mM, pH 7.1) using an Ultra-Turrax homogenizer and centrifuged at 2°C for 10 min at 27,000 x g. The washed pellet is re-suspended in 15 ml Tris-citrate buffer and frozen at -80°C until the day of the binding experiment.

## Assay

On the day of the experiment the cell membrane preparation is thawed and centrifuged at 2°C for 10 min at 27,000 x g. The pellet is re-suspended, using an Ultra-Turrax homogenizer in Tris-citrate buffer, to 15-50 pg protein per assay and then used for binding assays.

Aliquots of 500 µl cell suspension are added to 25 µl of test compound solution and 25 µl of $^3$H-flumazenil (1 nM, final concentration), mixed and incubated for 40 min at 2°C. Non-specific binding is determined using clonazepam (1 µM, final concentration).

All dilutions of test compounds and incubation of assay are performed in glass vials / 96-vial plates. Solutions of test compounds and $^3$H-flumazenil are prepared 22x the desired final concentration. Compounds are dissolved in 100% DMSO (10 mM stock), diluted in 48% ethanol-water, and tested in triplicate in serial 1:3 or 1:10 dilutions. When screening large numbers of compounds only one concentration of each compound is tested in single wells. Reference compounds are not included routinely, but for each experiment performed total and nonspecific binding is compared to data obtained during validation of the assay.

Binding is either terminated by rapid filtration onto

1) Whatman GF/C glass fibre filters using a Brandel Cell harvester, followed by 5 washes with 1 ml ice-cold buffer or onto 2) UniFilter GF/C glass fibre filter plates using a Tomtec cell harvester, followed by washing with approximately 5 ml ice-cold buffer.

The amount of radioactivity on the filters is determined by conventional liquid scintillation counting using a

1) Tri-Garb™counter (PerkinElmer Life and Analytical Sciences) for separate large filters or
2) Topcount™counter (PerkinElmer Life and Analytical Sciences) for 96-well filter plates. Specific binding is total binding minus non-specific binding.

## Calculations

25-75% inhibition of specific binding must be obtained before calculation of an $IC_{50}$ (the concentration (µM) of the test compound which inhibits the specific binding of $^3$H-flumazenil by 50%).

The $IC_{50}$ value for a test compound is determined based on the equation:

$$B = 100—(100 * C^n/(IC_{50}{}^n + C^n))$$

where B is the binding in percentage of total specific binding; C is the concentration of test compound; and n is the Hill coefficient. For screening purposes n is set to 1. The $IC_{50}$ value is calculated from the concentration response curves by the non-linear regression method using the curve-fitting program GraphPad Prism.

The Ki value for a test compound can be calculated from the $IC_{50}$ value using the equation by Cheng and Prusoff:

$$K = IC_{50}/(1 + L/K_d)$$

where the $K_d$ for $^3$H-flumazenil is 0.36 nM, and L is the measured concentration of $^3$H-flumazenil in the inhibition assay.

**Assay B: In vitro evaluation of modulation of $\alpha_5\beta_2\gamma_2$ GABA$_A$ receptor.**

Modulatory efficacy of compounds of formular (I) is determined electrophysiological recordings in oocytes using the two-electrode voltage clamp (TEVC) technique. Oocytes are injected with cRNA for human GABA$_A$ receptor subunits $\alpha_5$, $\beta_2$ and $\gamma_2$ in a 3:1:3 ratio and modulatory efficacy is evaluated by co-applications with a submaximal $EC_{5-20}$ GABA concentration (0.5 $\mu$M) termed GABA control. As a standard, the compounds are tested in five concentrations (3.16, 0.316, 0.0316, 0.00316 and 0.000316 $\mu$M) on each oocyte starting with the lowest concentration. Background subtracted peak current amplitudes are normalized to the respective GABAcontrol current, converted to % change and depicted +/- S.E.M. as a function of increasing compound concentrations. Plotted datapoints are fitted to the empirical Hill equation using non-linear regression. 95 % confidence intervals for maximal efficacy (Bottom) and potency (Log $EC_{50}$) are derived from this fitting routine.

| Example | Assay A: GABA$_A$ $\alpha_5$ $\beta_3$ $\gamma_{2s}$ Ki [nM] | Assay B: GABA$_A$ $\alpha_5\beta_3\gamma_2$ $EC_{50}$ [nM] | Assay B: GABA$_A$ $\alpha_5\beta_3\gamma_2$ Emax vs GABA [nM] |
|---|---|---|---|
| 1 | 10 | 41.3 | -24.9 |
| 2 | 15 | 92.5 | -25.3 |
| 3 | 13 | 6.6 | -24.7 |
| 4 | 17 | 93 | -22.3 |
| 5 | 16 | 11.6 | -32.3 |
| 6 | 15 | 13.9 | -8.8 |
| 7 | 26 | 12.7 | -6.3 |
| 8 | 18 | | 2.7 |
| 9 | 13 | | |
| 10 | 9 | 75 | -8 |
| 11 | 9 | 37.5 | -13.5 |
| 12 | 19 | 146.5 | -12.3 |
| 13 | 6 | 44.5 | -9 |
| 14 | 33 | 89 | -21 |
| 15 | 40 | 34.7 | -24.7 |
| 16 | 21 | 85.9 | -20.3 |
| 17 | 16 | 11 | -33.2 |
| 18 | 14 | 23.3 | -28.7 |
| 19 | 17 | 64 | -28.5 |
| 20 | 16 | 11.6 | -32.3 |
| 21 | 10 | | -4 |
| 22 | 19 | 26.9 | -14.9 |
| 23 | 49 | 3.1 | -8 |
| 24 | 32 | 9.8 | -14.7 |
| 25 | 23 | 8.2 | -11 |
| 26 | 14 | | -5.3 |

(continued)

| Example | Assay A: $GABA_A\ \alpha_5\ \beta_3\ \gamma_{2s}$ Ki [nM] | Assay B: $GABA_A\ \alpha_5\ \beta_3\ \gamma_2$ $EC_{50}$ [nM] | Assay B: $GABA_A\ \alpha_5\ \beta_3\ \gamma_2$ Emax vs GABA [nM] |
|---|---|---|---|
| 27 | 12 | | -6.7 |
| 28 | 24 | 14.6 | -20 |
| 29 | 19 | 11.5 | -29 |
| 30 | 24 | 4.6 | -4.7 |
| 31 | 23 | 20 | -12.3 |
| 32 | 18 | | -3.3 |
| 33 | 24 | 29.5 | -21 |
| 34 | 25 | 4.2 | -17 |
| 35 | 49 | 63 | -9.6 |
| 36 | >8891 | | |
| 37 | 11 | 37.3 | -25.3 |
| 38 | 12 | 22.8 | -22.5 |
| 39 | 39 | 353.7 | -24.6 |
| 40 | 8 | 32.7 | -10.3 |
| 41 | 2 | 43.7 | -21.3 |
| 42 | 14 | 15.8 | -25 |
| 43 | 28 | 24.8 | -20.2 |
| 44 | 14 | 14.1 | -17.7 |
| 45 | 150 | 23 | -17.3 |
| 46 | 220 | 10.4 | -26.3 |
| 47 | 120 | 15.5 | -7 |
| 48 | 82 | 78.9 | -13.2 |
| 49 | 25 | | 5.7 |
| 50 | 22 | 12 | -13.7 |
| 51 | 15 | 29 | -27.3 |
| 52 | 35 | 2 | -16 |
| 53 | 19 | 3.9 | -16 |
| 54 | 14 | 15 | -34.7 |
| 55 | 32 | 24.3 | -20.3 |
| 56 | 10 | 14.5 | -17.7 |

These data show that the compounds of the present invention show target engagement and a strong negative modulation of the GABA receptor function. The data also show that the compounds have improved properties with respect to $GABA_A$5R binding, which translates in lower efficacious doses of the compounds for disease treatment (see also: Ballard, T.M., et al. (2009). RO4938581, a novel cognitive enhancer acting at GABAA $\alpha$5 subunit-containing receptors. Psychopharmacology (2009) 202: 207-223; J. Pharmacol. Exp. Ther. (2006) 316: 1335-1345.)

**Assessment of efflux in Madin-Darby canine kidney (MDCK) cells transfected with the human MDR1 gene to assess brain penetration** (Drug Metabolism and Disposition February 2008, 36 (2) 268-275; DOI: https://doi.org/10.1124/dmd.107.017434)

Apparent permeability coefficients (PE) of the compounds across the MDCK-MDR1 cell monolayers are measured (pH 7.4, 37°C) in apical-to-basal (AB) and basal-to-apical (BA) transport direction. AB permeability (PEAB) represents drug absorption from the blood into the brain and BA permeability (PEBA) drug efflux from the brain back into the blood via both passive permeability as well as active transport mechanisms mediated by efflux and uptake transporters that are expressed on the MDCK-MDR1 cells, predominantly by the overexpressed human MDR1 P-gp. The compounds are assigned to permeability/absorption classes by comparison of the AB permeabilities with the AB permeabilities of reference compounds with known in vitro permeability and oral absorption in the human. Identical or similar permeabilities in both transport directions indicate passive permeation, vectorial permeability points to additional active transport mechanisms. Higher PEBA than PEAB indicates the involvement of active efflux mediated by MDR1 P-gp. Active transport is concentration- dependently saturable.

MDCK-MDR1 cells (1-2 x 10e5 cells/1 cm2 area) are seeded on filter inserts (Costar transwell polycarbonate or PET filters, 0.4 μm pore size) and cultured (DMEM) for 7 days. Subsequently, the MDR1 expression is boosted by culturing the cells with 5 mM sodium butyrate in full medium for 2 days. Compounds are dissolved in appropriate solvent (like DMSO, 1 -20 mM stock solutions). Stock solutions are diluted with HTP-4 buffer (128.13 mM NaCl, 5.36 mM KCl, 1 mM $MgSO_4$, 1.8 mM $CaCl_2$, 4.17 mM $NaHCO_3$, 1.19 mM $Na_2HPO_4$ x $7H_2O$, 0.41 mM $NaH_2PO_4xH_2O$, 15 mM HEPES, 20 mM glucose, 0.25 % BSA, pH 7.4) to prepare the transport solutions (0.1 - 300 μM compound, final DMSO <= 0.5 %). The transport solution (TL) is applied to the apical or basolateral donor side for measuring A-B or B-A permeability (3 filter replicates), respectively. The receiver side contains the same buffer as the donor side. Samples are collected at the start and end of experiment from the donor and at various time intervals for up to 2 hours also from the receiver side for concentration measurement by HPLC-MS/MS or scintillation counting. Sampled receiver volumes are replaced with fresh receiver solution.

| Example | MDCK-PGP A-B [10-6 cm/s] | MDCK-PGP Efflux ratio [PEBA/PEAB] |
|---|---|---|
| 1 | 31 | 1.8 |
| 2 | 34 | 1.9 |
| 3 | 41 | 1.4 |
| 4 | 57 | 0.8 |
| 12 | 51 | 0.3 |
| 14 | 55 | 0.7 |
| 15 | 17 | 3.5 |
| 16 | 59 | 0.6 |
| 17 | 51 | 1 |
| 18 | 71 | 0.6 |
| 19 | 41 | 1.2 |
| 20 | 15 | 2 |
| 22 | 40 | 1.1 |
| 24 | 10 | 6.6 |
| 28 | 3 | 17.2 |
| 37 | 59 | 0.4 |
| 38 | 86 | 0.5 |
| 39 | 59 | 0.6 |
| 42 | 24 | 1.4 |
| 43 | 54 | 0.7 |
| 48 | 69 | 0.8 |

(continued)

| Example | MDCK-PGP A-B [10-6 cm/s] | MDCK-PGP Efflux ratio [PEBA/PEAB] |
|---|---|---|
| 52 | 18 | 2.7 |
| 56 | 58 | 0.6 |

These data shows that the compounds of the present invention have excellent brain penetration properties with low efflux ratio from the brain compartment.

**Assessment of metabolic stability in human liver microsomes (human MST)**

The metabolic stability of the compounds according to the invention may be investigated as follows:
The metabolic degradation of the test compound is assayed at 37°C with pooled human liver microsomes. The final incubation volume of 100 μL per time point contains TRIS buffer pH 7.6 at room temperature (0.1 M), $MgCl_2$ (5 mM), microsomal protein (1 mg/mL) and the test compound at a final concentration of 1 μM. Following a short pre-incubation period at 37°C, the reactions are initiated by addition of beta-nicotinamide adenine dinucleotide phosphate, reduced form (NADPH, 1 mM), and terminated by transferring an aliquot into solvent after different time points. After centrifugation (10000 g, 5 min), an aliquot of the supernatant is assayed by LCMS/MS for the amount of parent compound. The half-life (t1/2) is determined by the slope of the semi-logarithmic plot of the concentration-time profile.

| Example | Human MST t1/2 [min] | Example | Human MST t1/2 > [min] |
|---|---|---|---|
| 1 | >130 | 28 | >130 |
| 3 | >130 | 29 | 97 |
| 4 | 40 | 30 | >130 |
| 5 | 83 | 31 | 127 |
| 6 | 116 | 32 | 78 |
| 7 | >130 | 33 | 98 |
| 8 | >130 | 35 | >130 |
| 9 | 101 | 37 | 129 |
| 17 | >130 | 39 | >130 |
| 18 | >130 | 48 | >130 |
| 20 | >130 | 49 | >130 |
| 22 | >130 | 50 | 130 |
| 23 | >130 | 52 | >130 |
| 24 | 62 | 53 | >130 |
| 25 | >130 | 56 | >130 |

In view of their ability to modulate the activity of $GABA_A$ receptors containing the α5 subunit and their advantaneouges pharmacokinetic properties the compounds of general formula (I) according to the invention, or the physiologically acceptable salts thereof, are suitable for the treatment and/or preventative treatment of all those diseases or conditions which can be influenced by modulation of $GABA_A$ receptors containing the α5 subunit. Therefore, compounds according to the invention, including the physiologically acceptable salts thereof, are particularly suitable for the prevention or treatment of diseases, particularly acute neurological disorders, chronic neurological disorders, cognitive disorders, Alzheimer's disease, memory deficits, schizophrenia, positive, negative and/or cognitive symptoms associated with schizophrenia, cognitive impairment associated with schizophrenia, bipolar disorders, autism, Down syndrome, neurofibromatosis type I, post operative cognitive decline, sleep disorders, disorders of circadian rhythms, amyotrophic lateral sclerosis, dementia caused by AIDS, psychotic disorders, substance-induced psychotic disorder, anxiety disorders, generalized anxiety disorder, panic disorder, delusional disorder, obsessive compulsive disorders, acute stress disorder, drug addictions, movement disorders, Parkinson's disease, restless leg syndrome, cognition deficiency disorders, multi-infarct dementia, mood disorders, depression, major depressive disorder, neuropsychiatric conditions, psychosis, attention-deficit hyperactivity disorder, neuropathic pain, stroke, attentional disorders, eating disorders, anorexia, anorexia nervosa, cachexia, weight loss, muscle atrophy, pain conditions, chronic pain, nociceptive pain, post-operative pain, osteoarthritis pain, rheumatoid arthritis pain, musculoskeletal pain, burn pain, ocular pain, pain due to inflammation, pain due to bone fracture, hyperalgesia, neuropathic pain, herpes-related pain, HIV-related neuropathic pain, traumatic nerve injury, recovery after traumatic brain injury, post-stroke pain, post-ischemia pain, fibromyalgia, chronic headache, migraine, tension-type headache, diabetic neuropathic pain, phantom limb pain, visceral pain and cutaneous pain.

The compounds according to the invention, including the physiologically acceptable salts thereof, are even more suitable for the treatment of i.a. cognitive disorders, post operative cognitive decline, Alzheimer's disease, schizophrenia, positive, negative and/or cognitive symptoms associated with schizophrenia, cognitive impairment associated with schizophrenia, cognitive deficits associated with Down syndrome, cognitive deficits associated with autism, cognitive deficits associated with neurofibromatosis type I, or cognitive deficits after stroke.

In a further aspect of the present invention the present invention relates to methods for the treatment or prevention of above mentioned diseases and conditions, which method comprises the administration of an effective amount of a compound of general formula (I), or the pharmaceutically acceptable salts thereof, to a human being.

The dose range of the compounds of general formula (I) applicable per day is usually from 0.1 to 1000 mg, preferably from 1 to 500 mg by oral route, in each case administered 1 to 4 times a day.

Each dosage unit may conveniently contain from 0.1 to 500 mg, preferably 1 to 100 mg.

The actual pharmaceutically effective amount or therapeutic dosage will of course depend on factors known by those skilled in the art such as age and weight of the patient, route of administration and severity of disease. In any case the combination will be administered at dosages and in a manner which allows a pharmaceutically effective amount to be delivered based upon patient's unique condition.

Suitable preparations for administering the compounds of formula I, including the pharmaceutically acceptable salts thereof, will be apparent to those with ordinary skill in the art and include for example tablets, pills, capsules, suppositories, lozenges, troches, solutions, syrups, elixirs, sachets, injectables, inhalatives, powders, etc.. The content of the pharmaceutically active compound(s) should be in the range from 0.1 to 95 wt.-%, preferably 5.0 to 90 wt.-% of the composition as a whole.

Suitable tablets may be obtained, for example, by mixing one or more compounds according to formula I with known excipients, for example inert diluents, carriers, disintegrants, adjuvants, surfactants, binders and/or lubricants. The tablets may also consist of several layers.

For this purpose, the compounds of formula I prepared according to the invention may be formulated, optionally together with other active substances, together with one or more inert conventional carriers and/or diluents, e.g. with corn starch, lactose, glucose, microcrystalline cellulose, magnesium stearate, citric acid, tartaric acid, water, polyvinylpyrrolidone, water/ethanol, water/glycerol, water/sorbitol, water/polyethylene glycol, propylene glycol, cetylstearyl alcohol, carboxymethylcellulose or fatty substances such as hard fat or suitable mixtures thereof.

The compounds according to the invention may also be used in conjunction with other active substances, particularly for the treatment and/or prevention of the diseases and conditions mentioned above. A list of example is: Donepezil, Memantine, Acetazolamide, Carbamazepine, Eslicarbazepine acetate, Ethosuximide, Gabapentin, Lacosamide, Lamotrigine, Levetiracetam, Brivaracetam, Nitrazepam, Oxcarbazepine, Perampanel, Piracetam, Phenobarbital, Phenytoin, Pregabalin, Primidone, Rufinamide, Sodium valproate, Stiripentol, Tiagabine, Topiramate, Vigabatrin, Zonisamide, Levodopa, Carbidopa, Haloperidol, Loxapine, Thioridazine, Molindone, Thiothixene, Fluphenazine, Mesoridazine, Trifluoperazine, Perphenazine, Chlorpromazine, Aripiprazole, Asenapine Maleate, Clozapine, Iloperidone, Lurasidone, Olanzapine, Paliperidone, Quetiapine, Risperidone, Ziprasidone and Zolpidem.

The dosage for the combination partners mentioned above is usefully 1/5 of the lowest dose normally recommended up to 1/1 of the normally recommended dose.

Therefore, in another aspect, this invention relates to the use of a compound according to the invention or a pharmaceutically acceptable salt thereof combined with at least one of the active substances described above as a combination partner, for preparing a pharmaceutical composition which is suitable for the treatment or prevention of diseases or conditions described above.

The use of the compound according to the invention in combination with another active substance may take place simultaneously or at staggered times, but particularly within a short space of time. If they are administered simultaneously, the two active substances are given to the patient together; while if they are used at staggered times the two active substances are given to the patient within a period of less than or equal to 12 hours, but particularly less than or equal to 6 hours.

Consequently, in another aspect, this invention relates to a pharmaceutical composition which comprises a compound according to the invention or a pharmaceutically acceptable salt thereof and at least one of the active substances described above as combination partners, optionally together with one or more inert carriers and/or diluents.

The compound according to the invention may both be present together in one formulation, for example a tablet

or capsule, or separately in two identical or different formulations, for example as a so-called kit-of-parts.

## Claims

1. A compound having formula (I) or a salt thereof

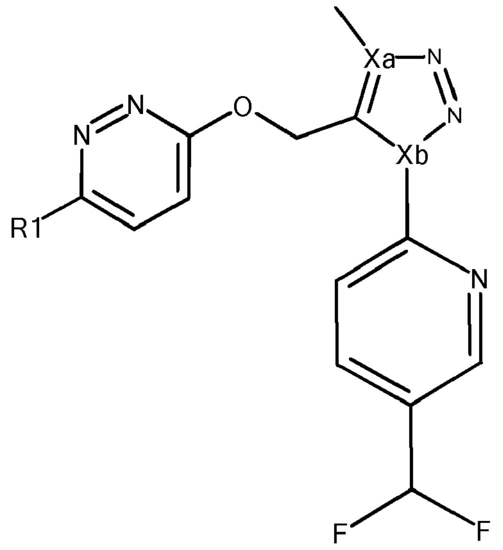

(I),

wherein:

**Xa and Xb** are different from each other and represent C or N and
**R1** is substituted phenyl or a 5- or 6- membered substituted heterocyclyl ring containing 1 or 2 or 3 heteroatoms.

2. The compound of claim 1 or a salt thereof, wherein either **Xa** or **Xb** is C.

3. The compound of claim 1 or a salt thereof, wherein **R1** is selected from the group consisting of

• phenyl substituted with carbamoyl

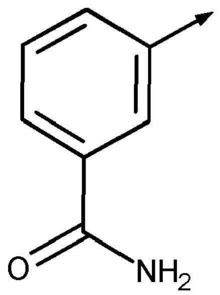
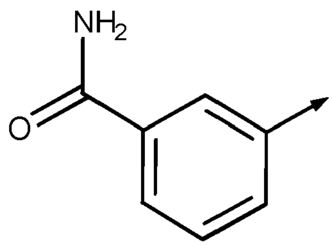

and
• unsubstituted 2-pyridon or 2-pyridon substituted with halogen such as fluorine or substituted on the nitrogen with methyl or ethyl

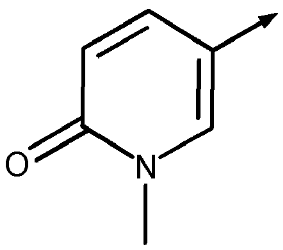
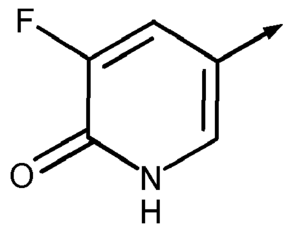
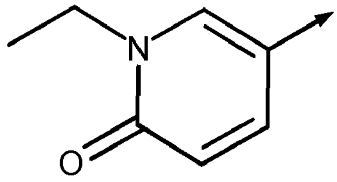

**4.** The compound of claim 1 or a salt thereof, wherein **R1** is
3-pyridyl substituted with NC- or -thiomethylate-, amino-, methyl-amino- or - methylsulfonyl or halogen,

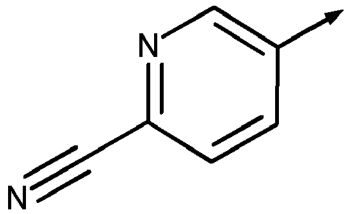
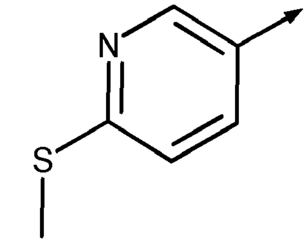
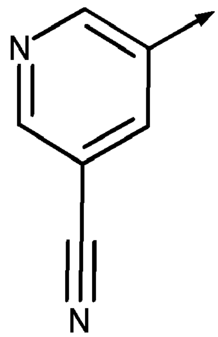

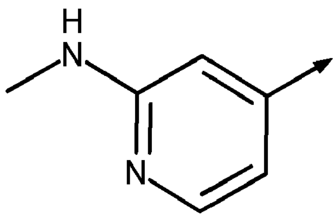
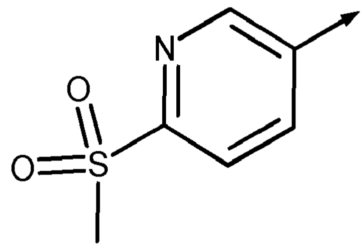

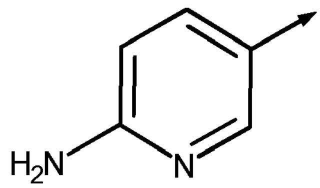

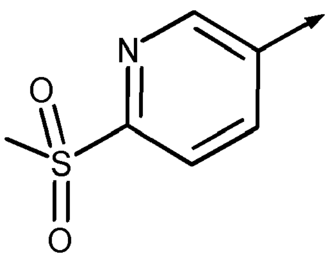
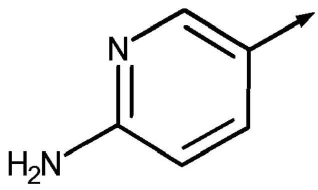
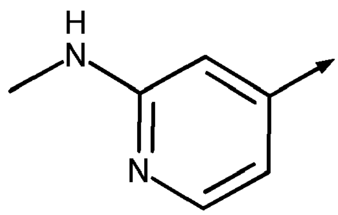

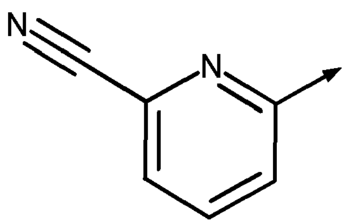
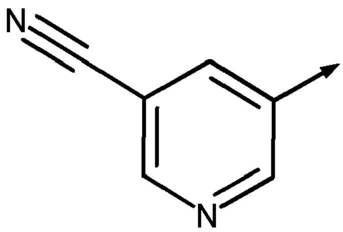

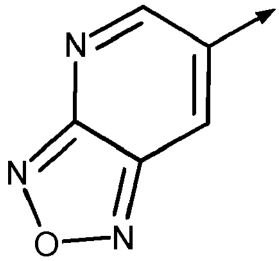

5. The compound of claim 1 or a salt thereof, wherein **R1** is substituted or unsubstituted pyrimidinyl- (substituted with $C_{1-6}$-alkyl-, -amino, - hydroxymethyl) or pyrazinyl-,

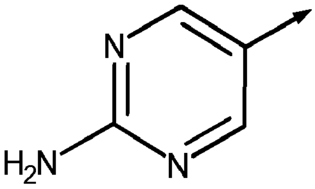
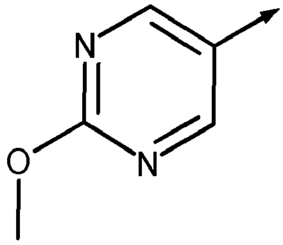
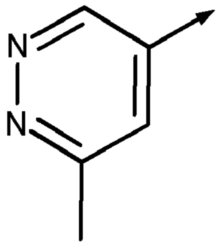

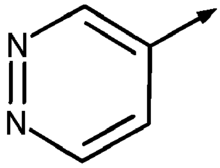
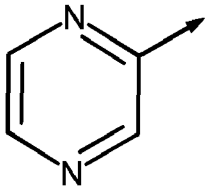

6. The compound of claim 1 or a salt thereof, wherein **R1** is pyrrolyl- substituted with $C_{1-6}$-alkyl- or NC-$CH_2$-$CH_2$- and pyrazolyl- substituted with $C_{1-6}$-alkyl-, $C_{3-5}$-cycloalkyl-, NC-$CH_2$-$CH_2$-, -amino, -methyl-amino, or -halogen

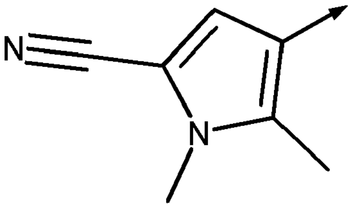

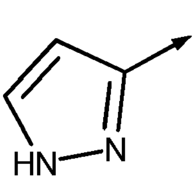
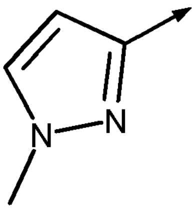
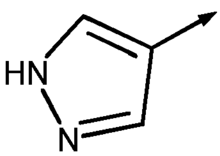
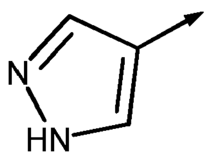

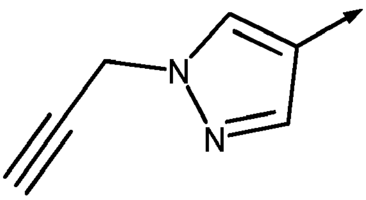
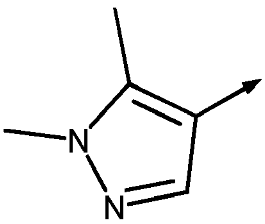
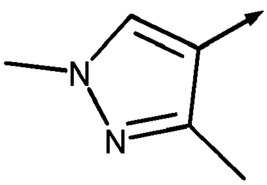

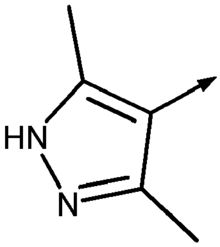
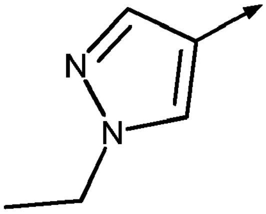
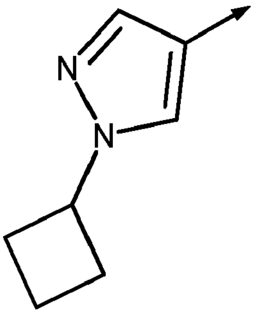

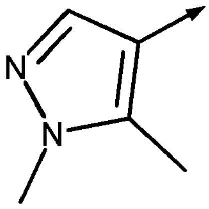

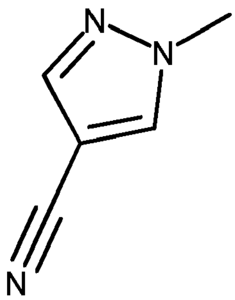
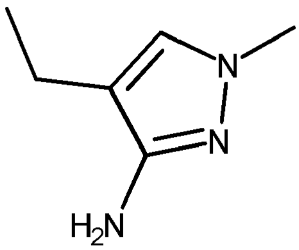
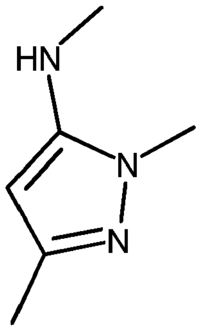

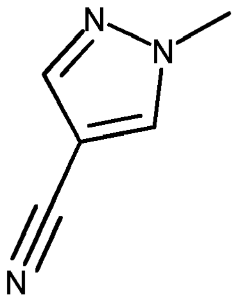
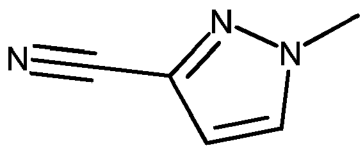
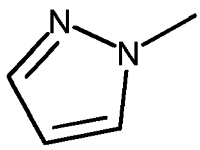

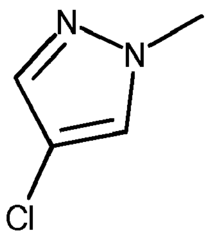
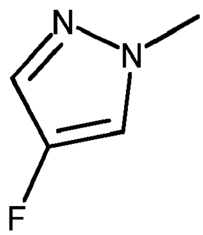

**7.** The compound of claim 1 or a salt thereof, wherein **R1** is imidazoly- substituted with $C_{1-6}$-alkyl-, carbamoyl, NC-$CH_2$-$CH_2$-, amino- or - methylamino-

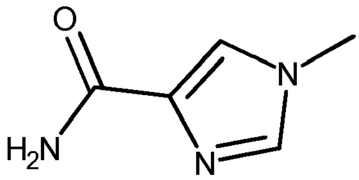
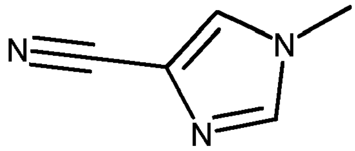

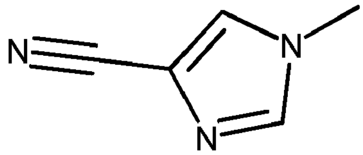

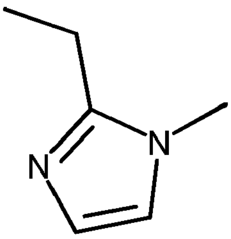
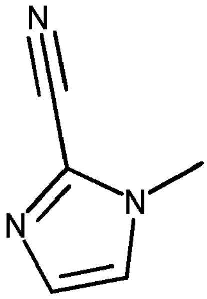
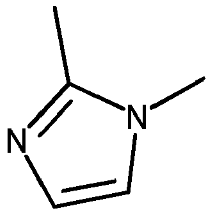

**8.** The compound of claim 1 or a salt thereof, wherein **R1** is unsubstituted triazolyl- or triazolyl- substituted with $C_{1-3}$-alkyl- such as methyl

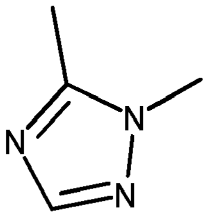
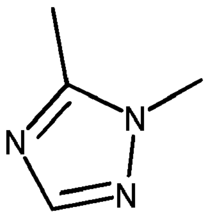
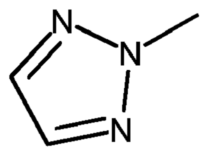
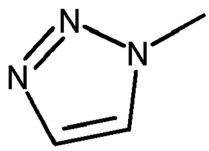

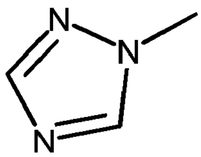

**9.** The compound of claim 1 or a salt thereof, wherein **R1** is oxazolyl- substituted with methyl or thiophenyl- substituted with NC-$CH_2$-$CH_2$-,

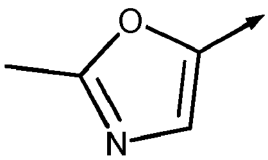
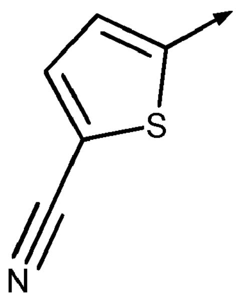

**10.** The compound of claim 1 or a salt thereof, wherein **R1** is selected from the group consisting of

• phenyl substituted with carbamoyl;

- unsubstituted 2-pyridon or 2-pyridon substituted with halogen such as fluorine or substituted on the nitrogen with methyl or ethyl;
- 3-pyridyl substituted with NC- or thiomethylate-, amino-, methyl-amino-, methylsulfonyl- or halogen;
- unsubstituted pyrimidinyl- or pyrimidinyl- substituted with $C_{1-6}$-alkyl-, amino-,-hydroxymethyl- or pyrazinyl-;
- pyrrolyl- substituted with $C_{1-6}$-alkyl- or NC-$CH_2$-$CH_2$- and pyrazolyl- substituted with $C_{1-6}$-alkyl-, $C_{3-5}$-cycloalkyl-, NC-$CH_2$-$CH_2$-, amino-, methyl-amino-, or halogen;
- imidazoly- substituted with $C_{1-6}$-alkyl-, carbamoyl-, NC-$CH_2$-$CH_2$-, amino- or-methylamino-;
- unsubstituted triazolyl- or triazolyl- substituted with $C_{1-3}$-alkyl- such as methyl;
- oxazolyl- substituted with methyl and thiophenyl- substituted with NC-$CH_2$-$CH_2$-.

**11.** The compound of claim 1 or a salt thereof selected from the group of compounds consisting of

| | | | |
|---|---|---|---|
| **1** | 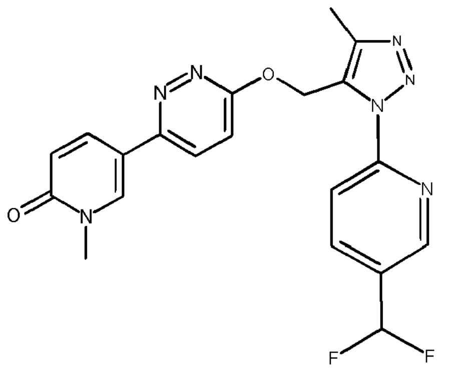 | **29** | 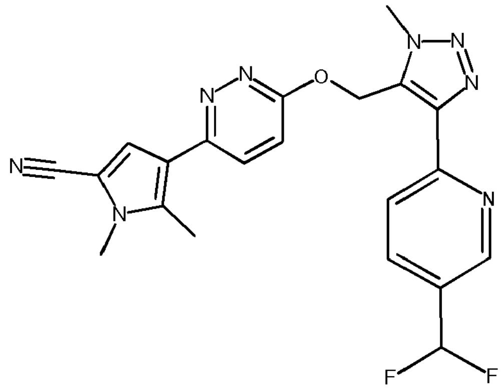 |
| **2** | 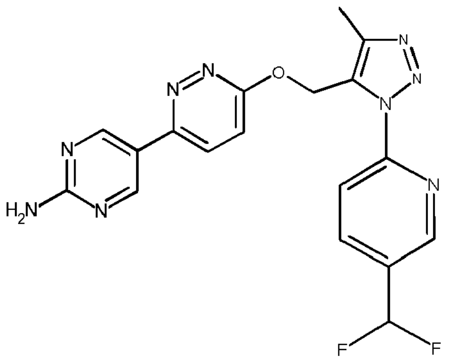 | **30** | 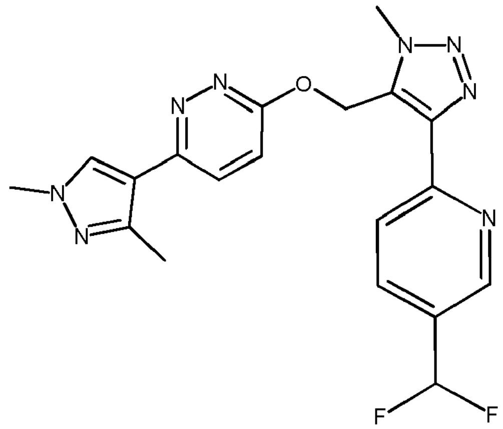 |
| **3** | 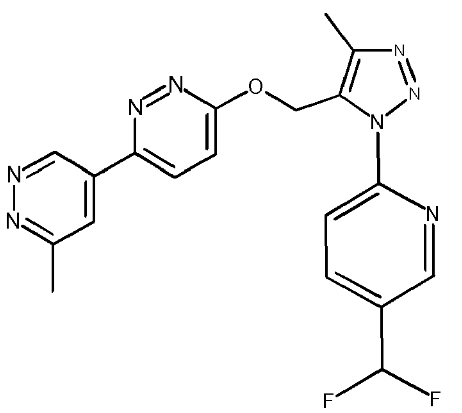 | **31** | 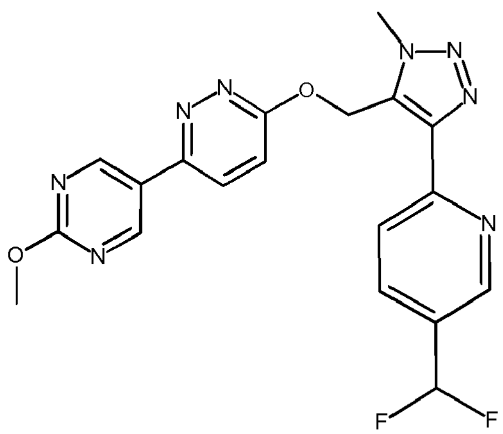 |

(continued)

| | | | |
|---|---|---|---|
| **4** | 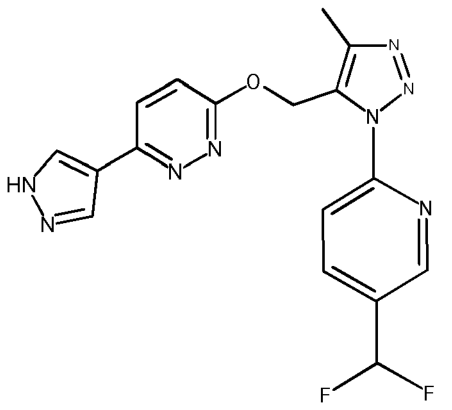 | **32** | 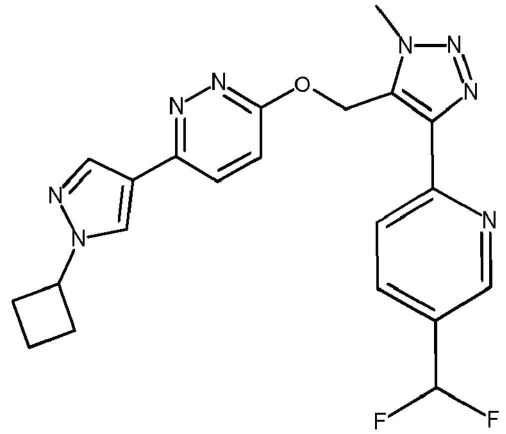 |
| **5** | 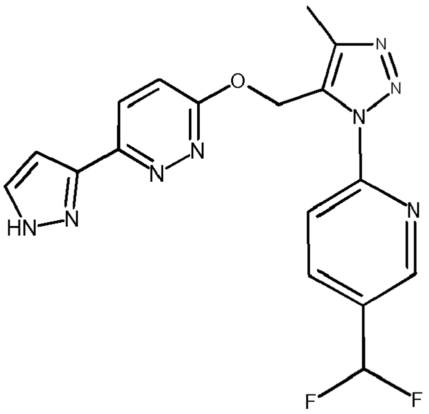 | **33** | 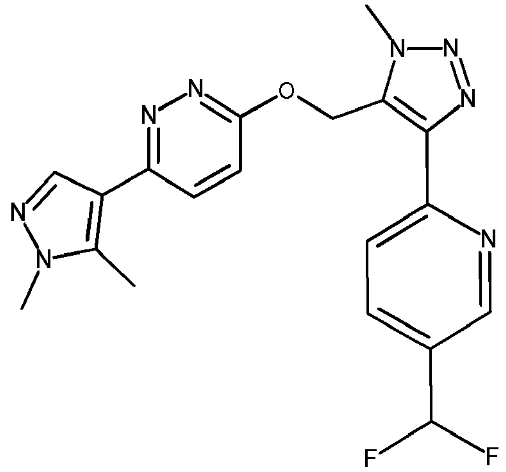 |
| **6** | 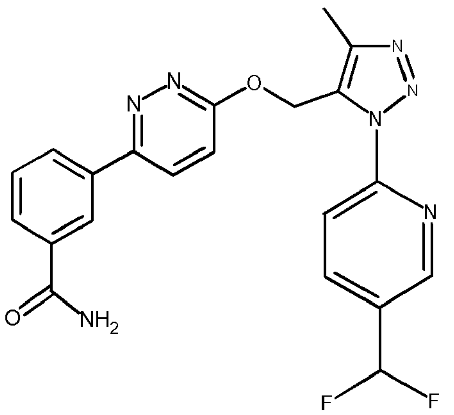 | **34** | 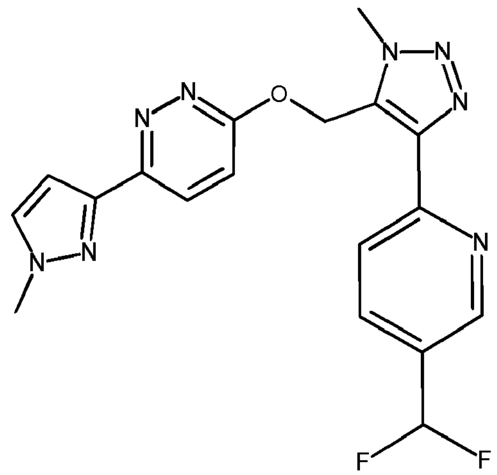 |

(continued)

| | | | |
|---|---|---|---|
| **7** | 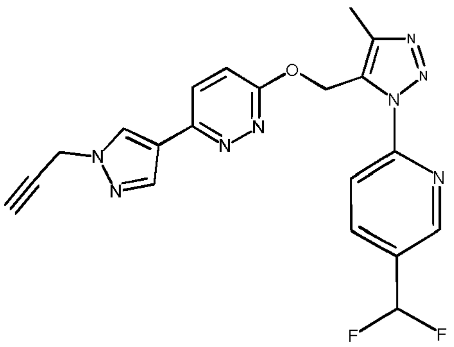 | **35** | 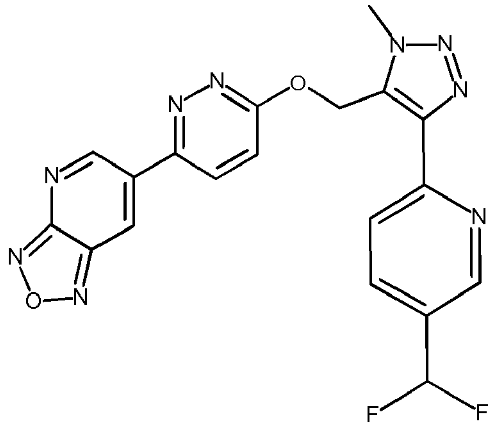 |
| **8** | 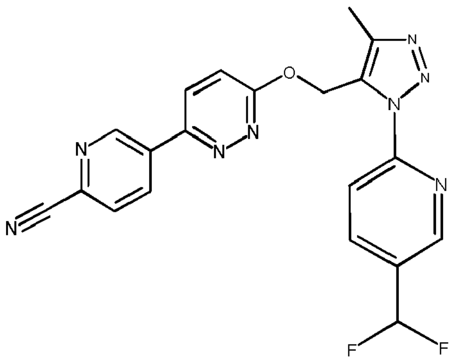 | **36** | 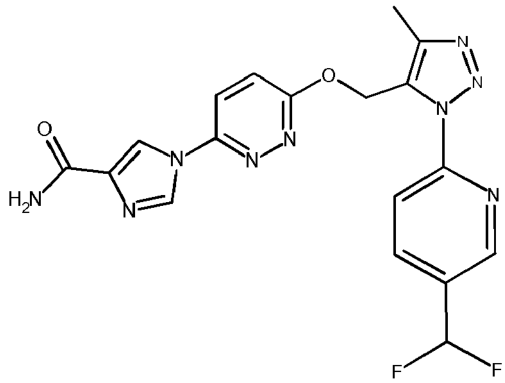 |
| **9** | 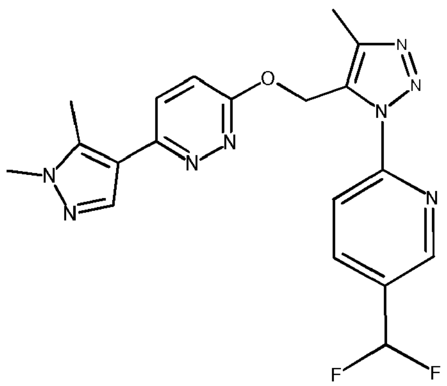 | **37** | 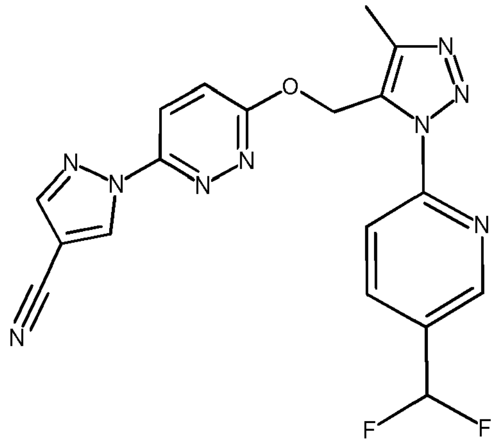 |

(continued)

| | | | |
|---|---|---|---|
| **10** | 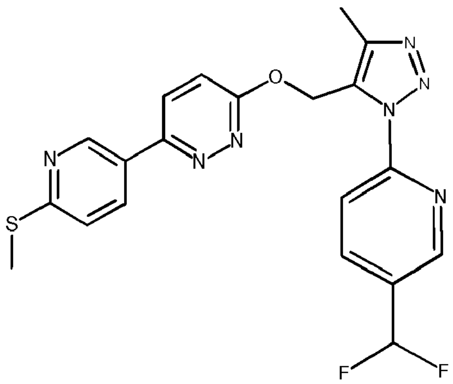 | **38** | 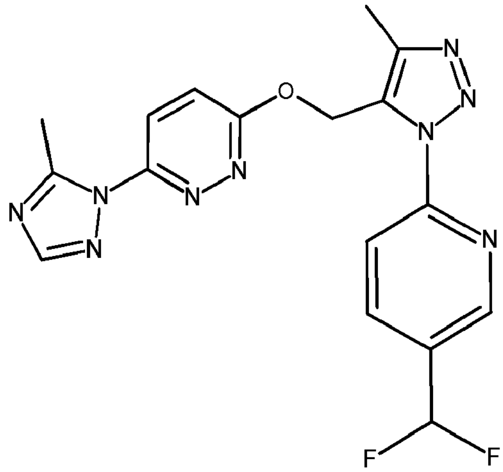 |
| **11** | 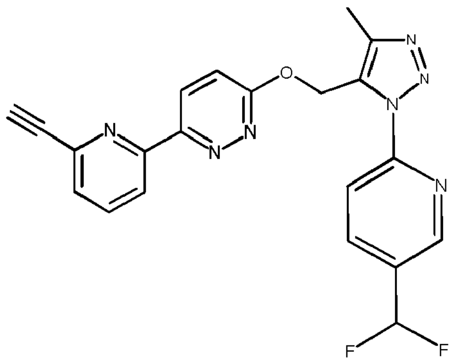 | **39** | 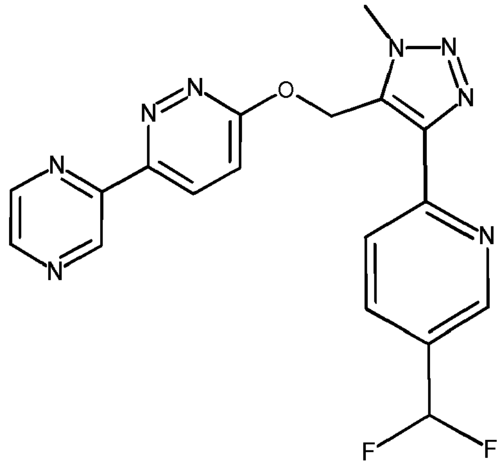 |
| **12** | 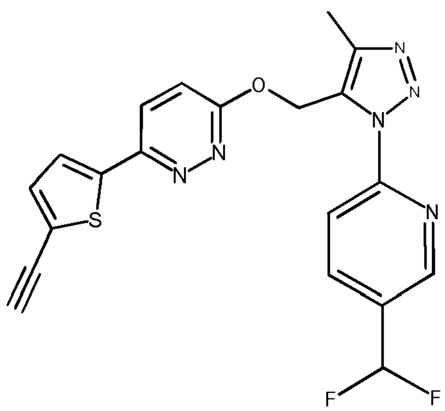 | **40** | 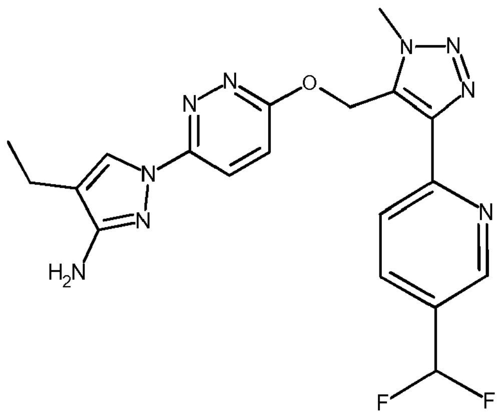 |

(continued)

| | | | |
|---|---|---|---|
| 13 | 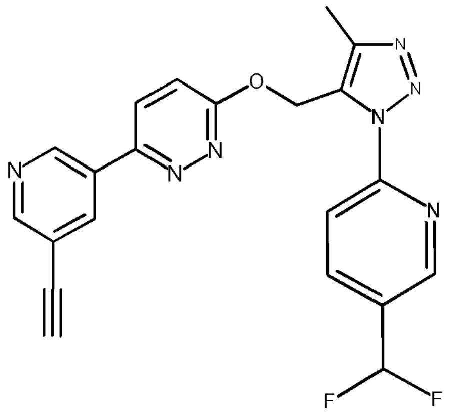 | 41 | 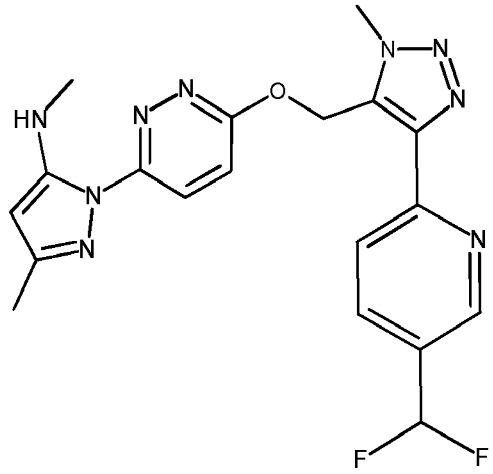 |
| 14 | 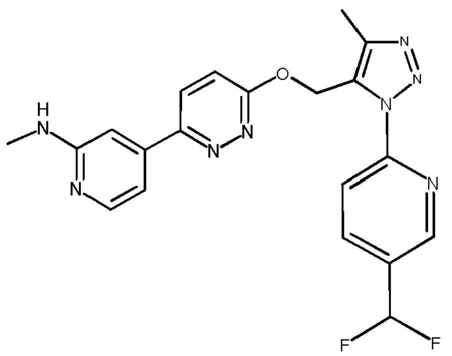 | 42 | 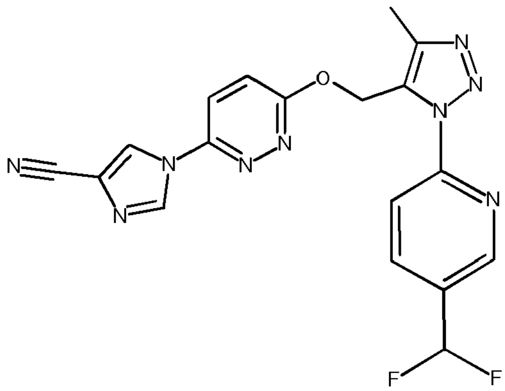 |
| 15 | 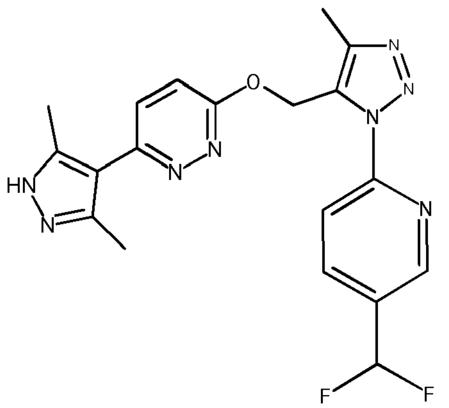 | 43 | 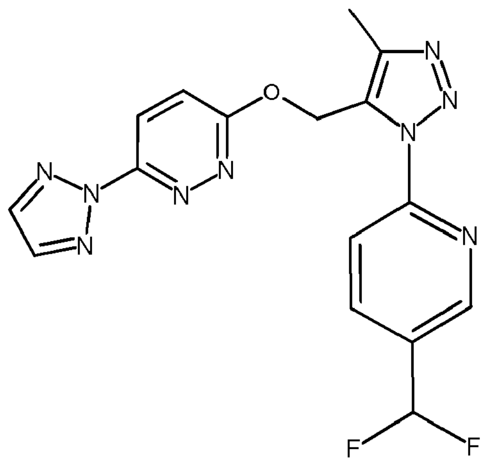 |

(continued)

| | | | |
|---|---|---|---|
| 16 | 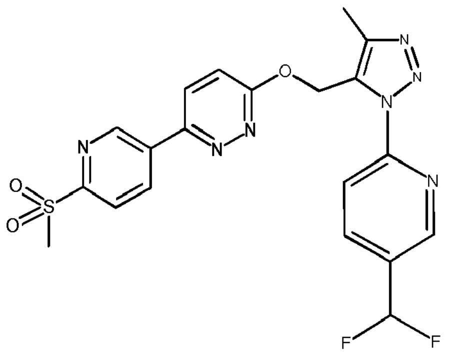 | 44 | 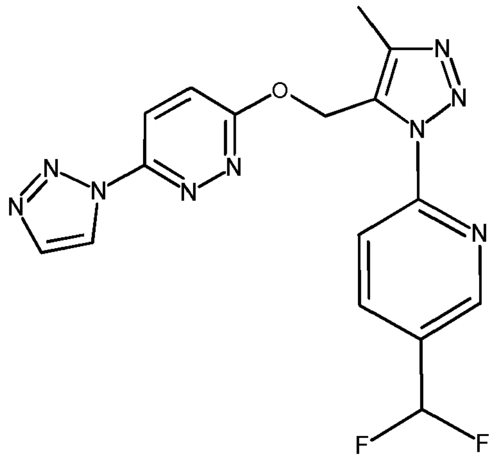 |
| 17 | 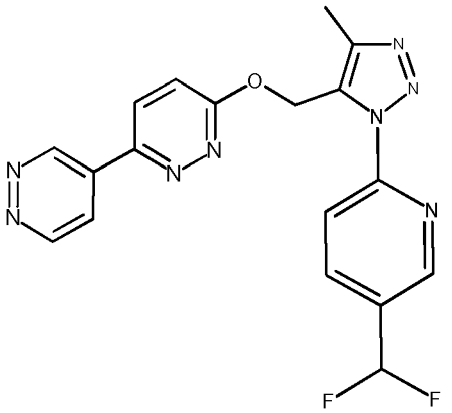 | 45 | 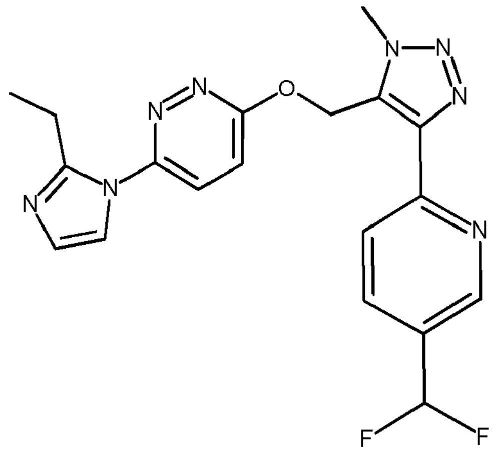 |
| 18 | 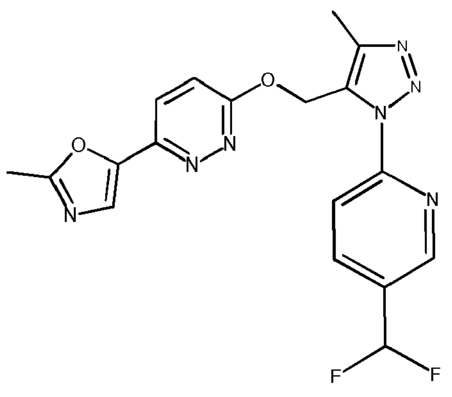 | 46 | 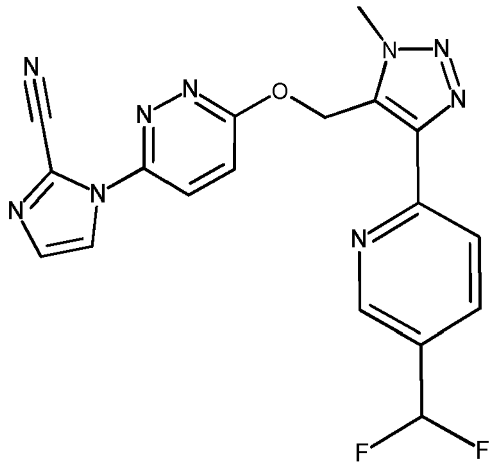 |

(continued)

| | | | |
|---|---|---|---|
| **19** | 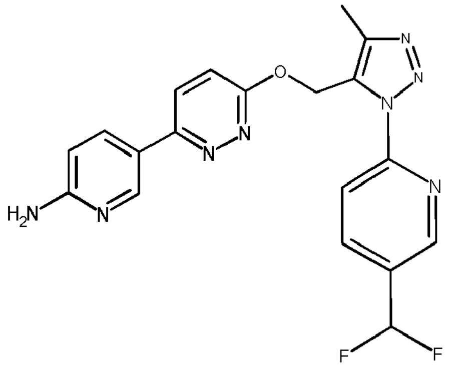 | **47** | 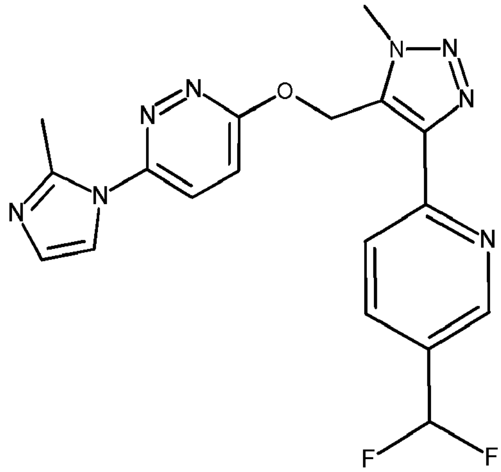 |
| **20** | 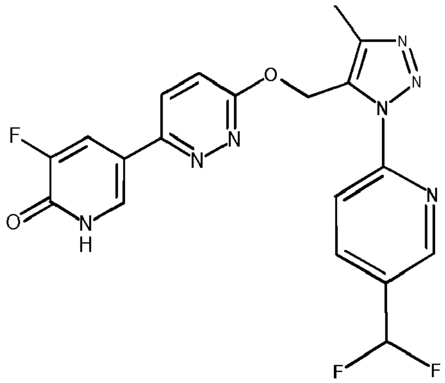 | **48** | 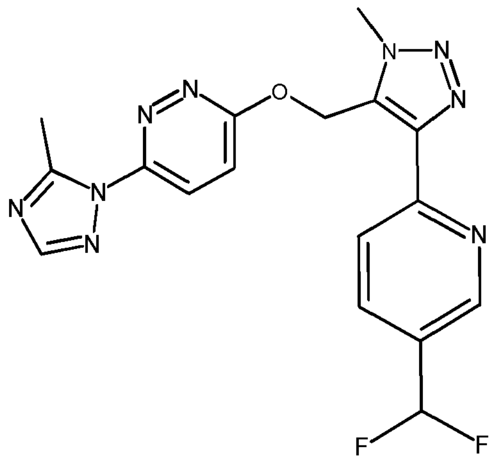 |
| **21** | 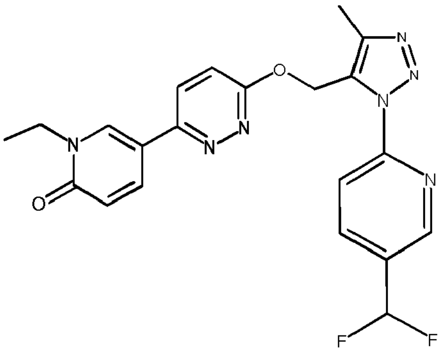 | **49** | 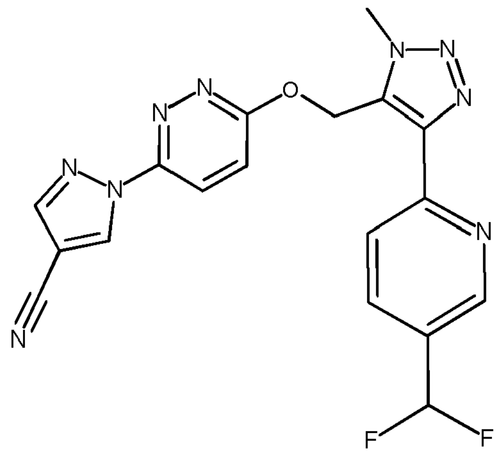 |

(continued)

| | | | |
|---|---|---|---|
| 22 | 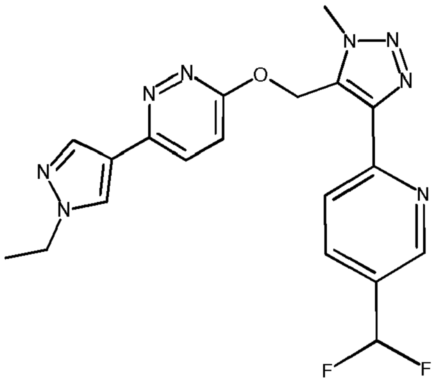 | 50 | 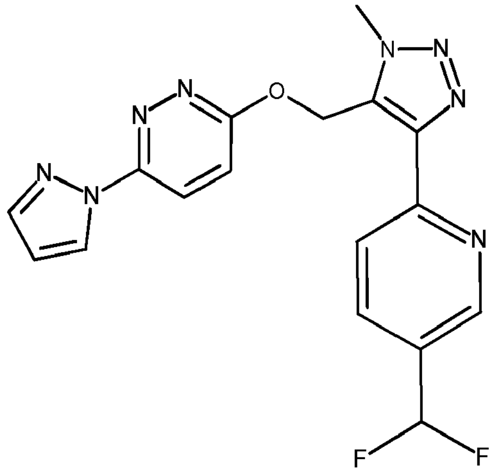 |
| 23 | 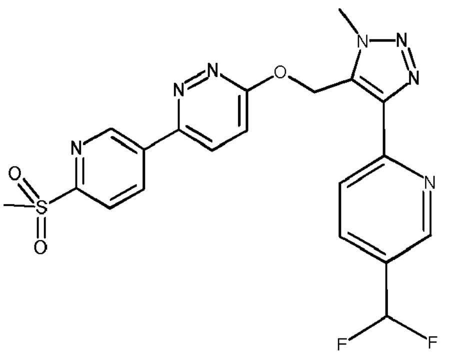 | 51 | 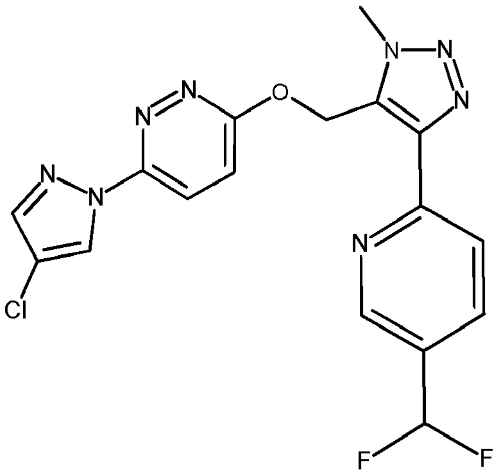 |
| 24 | 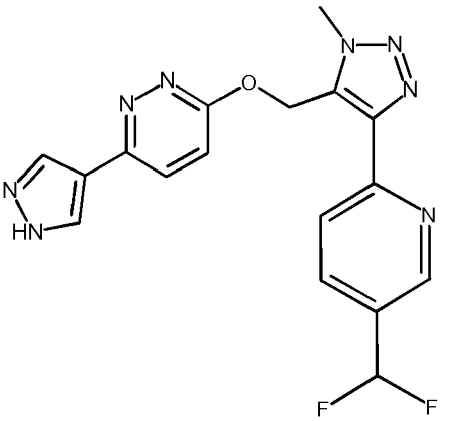 | 52 | 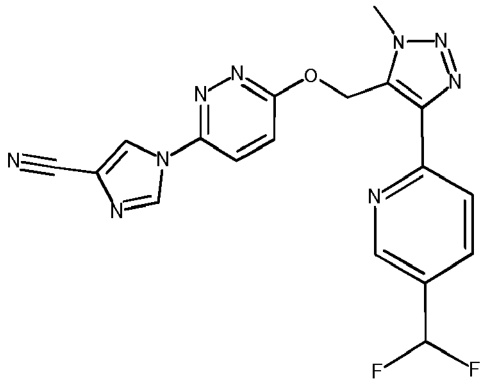 |

(continued)

| | | | |
|---|---|---|---|
| **25** | 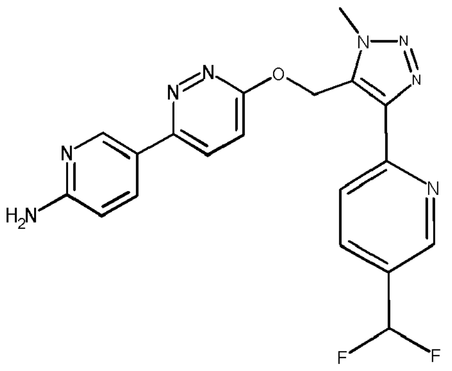 | **53** | 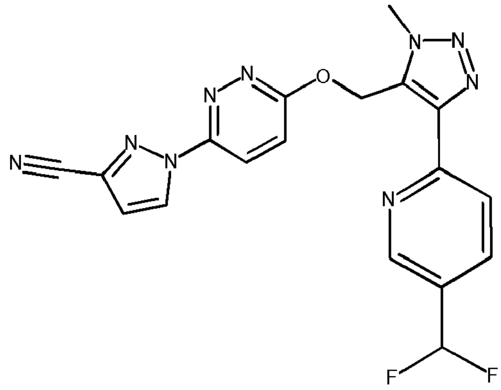 |
| **26** | 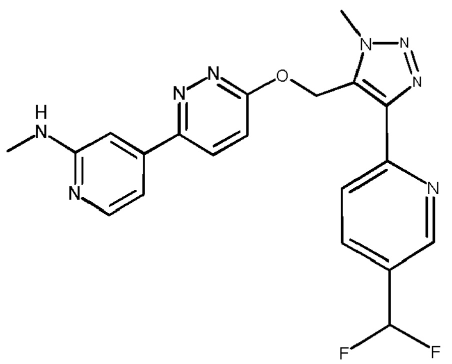 | **54** | 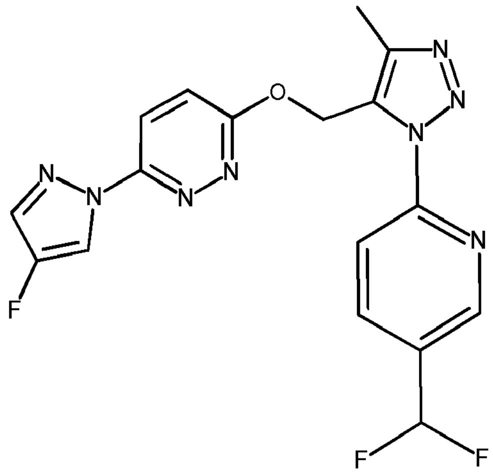 |
| **27** | 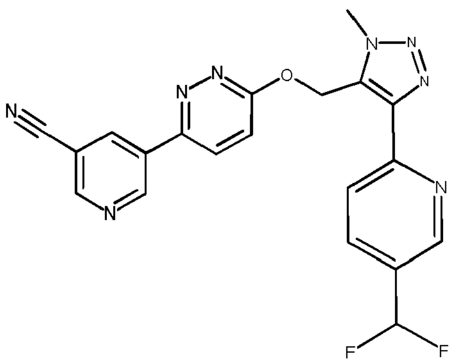 | **55** | 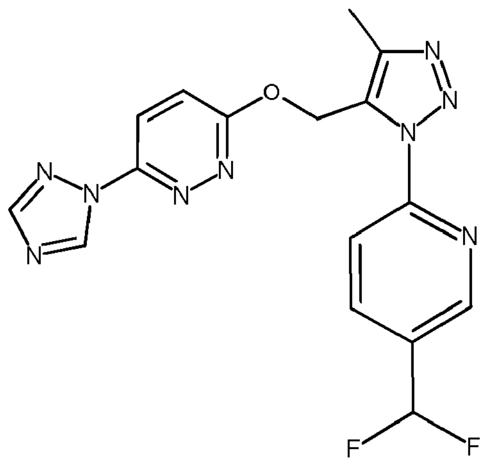 |

(continued)

| | | | |
|---|---|---|---|
| **28** | 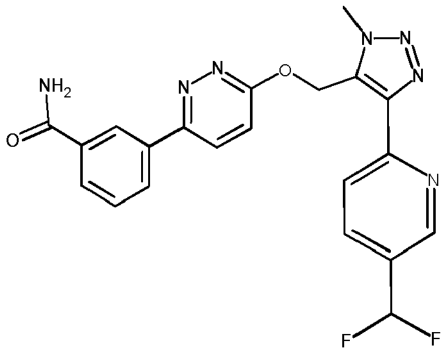 | **56** | 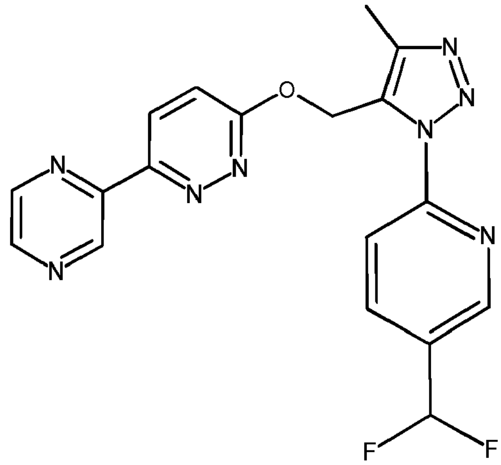 |

**12.** The salt of a compound of any one of claims 1-11 for use as a medicament.

**13.** A medicament prepared with a compound or salt thereof according to any one of claims 1 to 11.

**14.** The compound of claim 1 or a salt thereof wherein formula (I) is the formula of any one of compounds 22 to 56 and 39.

**15.** A method for the preparation of a compound of claim 1, comprising the following chemical reaction route

(a)

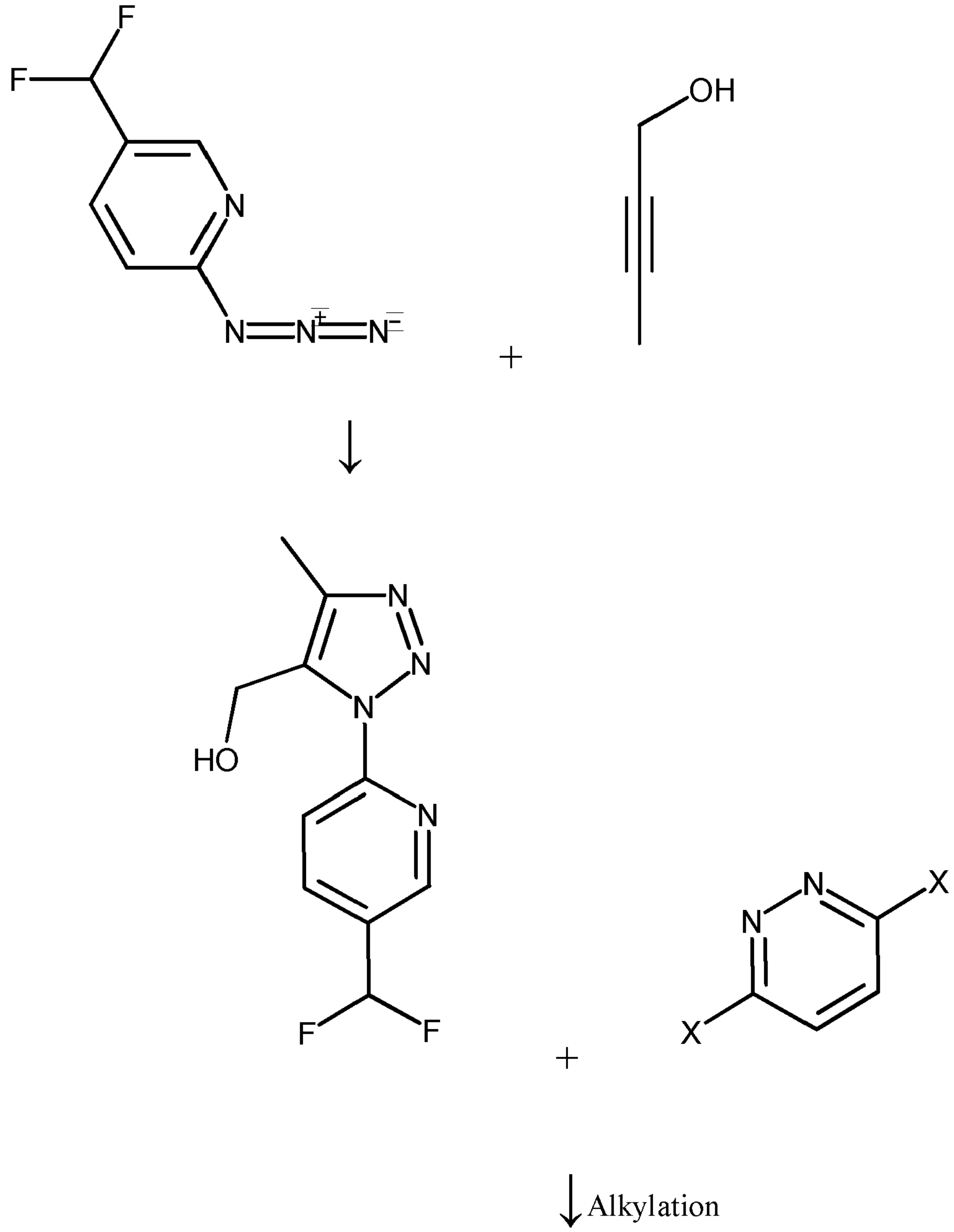

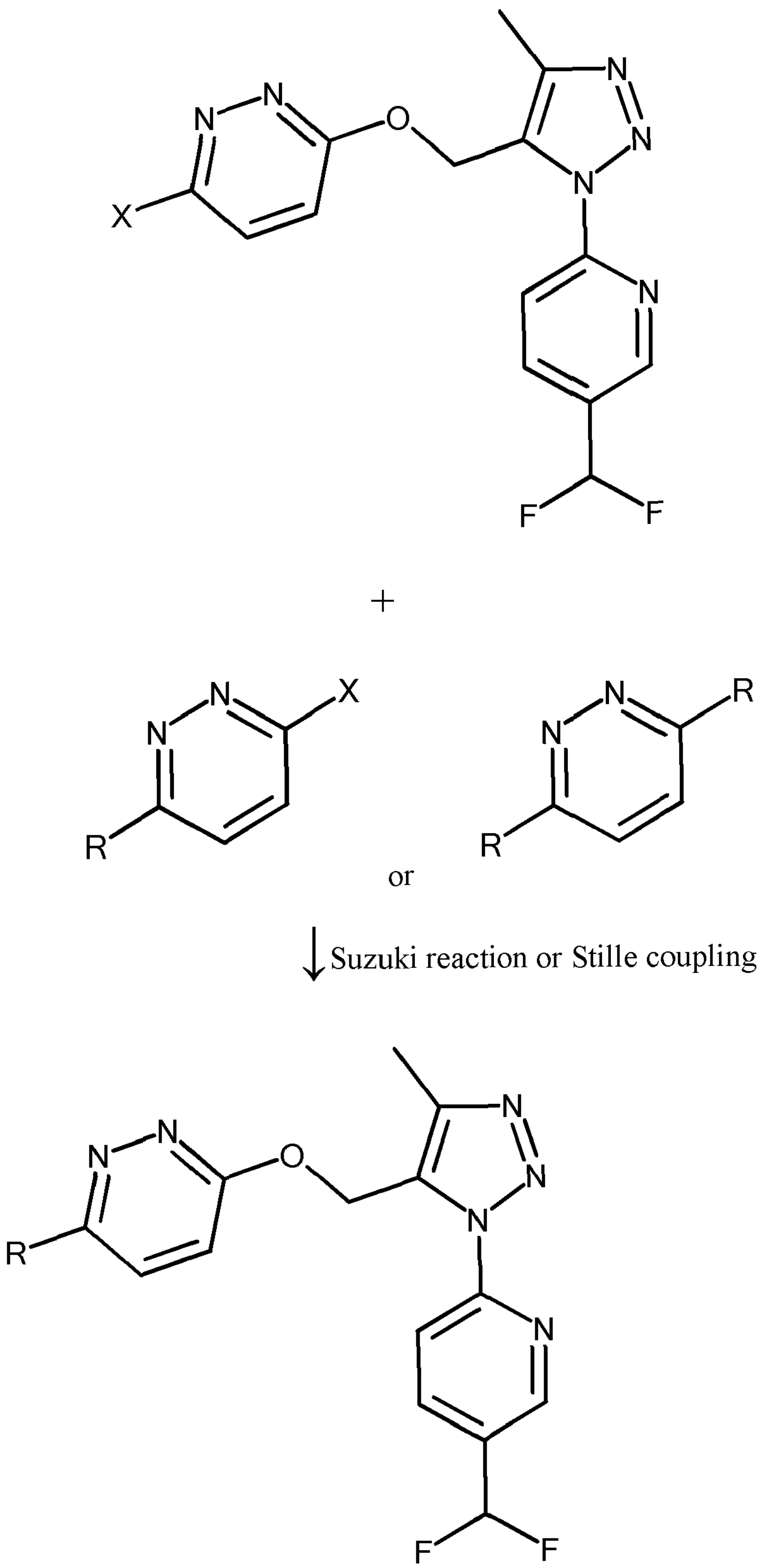


**16.** A method for the preparation of the compound of claim 1, comprising the following chemical reaction route

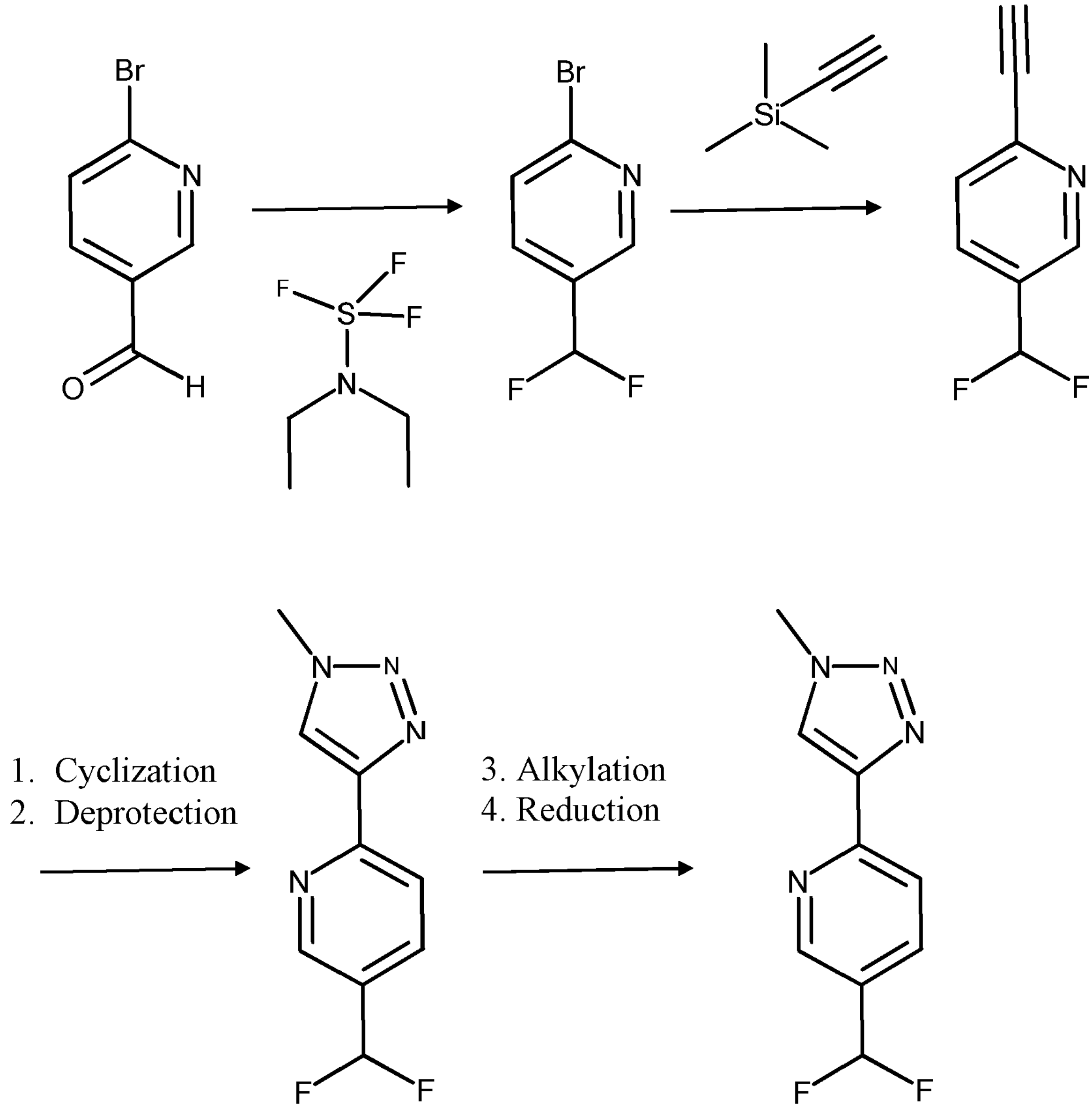
1. Cyclization
2. Deprotection
3. Alkylation
4. Reduction

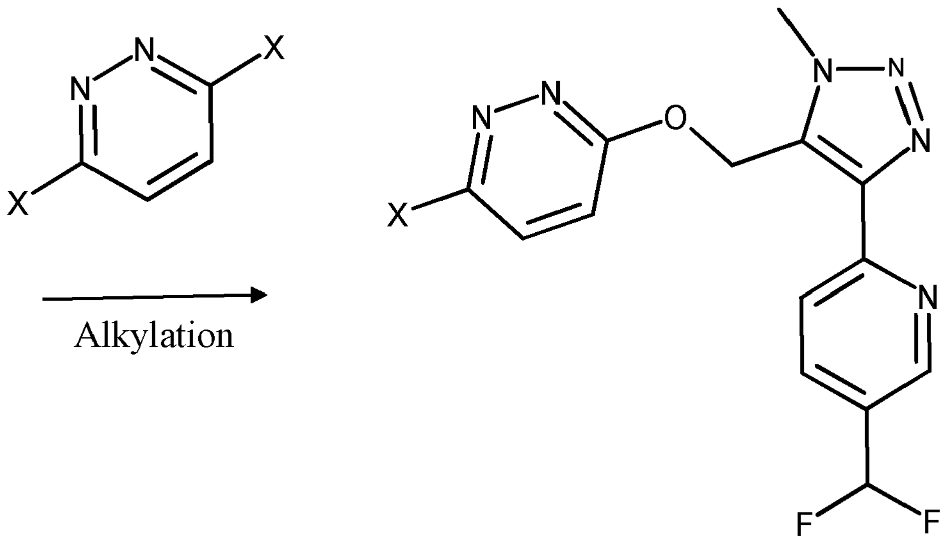


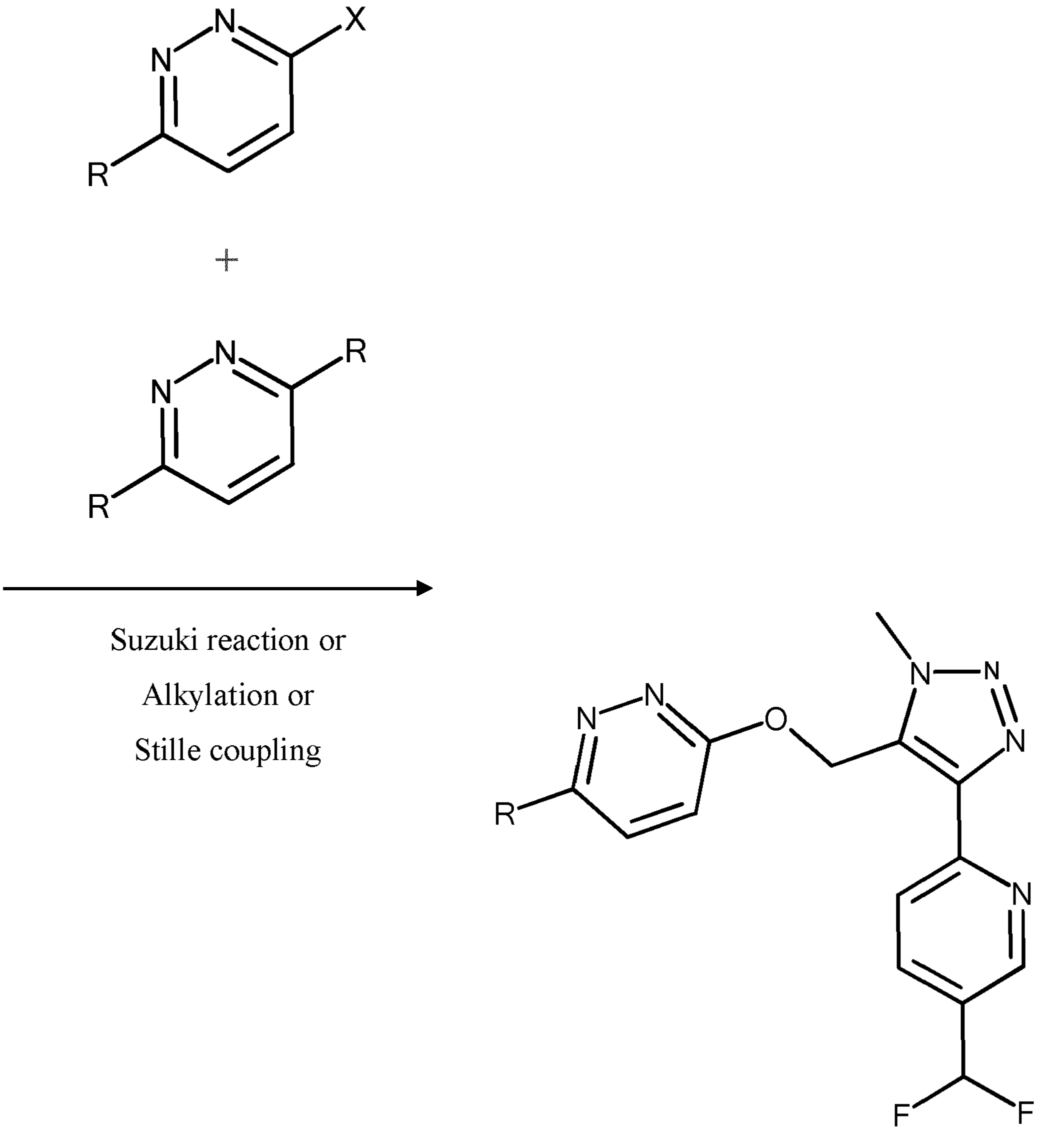


**17.** A pharmaceutical composition containing at least one compound according to one or more of the claims 1 to 11 or

a pharmaceutically acceptable salt thereof together with one or more pharmaceutically acceptable carrier.

**18.** A pharmaceutical composition for the treatment or prevention of acute neurological disorders, chronic neurological disorders, cognitive disorders, Alzheimer's disease, memory deficits, schizophrenia, positive, negative and/or cognitive symptoms associated with schizophrenia, cognitive impairment associated with schizophrenia, bipolar disorders, autism, Down syndrome, neurofibromatosis type I, post operative cognitive decline, sleep disorders, disorders of circadian rhythms, amyotrophic lateral sclerosis, dementia caused by AIDS, psychotic disorders, substance-induced psychotic disorder, anxiety disorders, generalized anxiety disorder, panic disorder, delusional disorder, obsessive compulsive disorders, acute stress disorder, drug addictions, movement disorders, Parkinson's disease, restless leg syndrome, cognition deficiency disorders, multi-infarct dementia, mood disorders, depression, major depressive disorder, neuropsychiatric conditions, psychosis, attention-deficit hyperactivity disorder, neuropathic pain, stroke, attentional disorders, eating disorders, anorexia, anorexia nervosa, cachexia, weight loss, muscle atrophy, pain conditions, chronic pain, nociceptive pain, post-operative pain, osteoarthritis pain, rheumatoid arthritis pain, musculoskeletal pain, burn pain, ocular pain, pain due to inflammation, pain due to bone fracture, hyperalgesia, neuropathic pain, herpes-related pain, HIV-related neuropathic pain, traumatic nerve injury, recovery after traumatic brain injury, post-stroke pain, post-ischemia pain, fibromyalgia, chronic headache, migraine, tension-type headache, diabetic neuropathic pain, phantom limb pain, visceral pain and cutaneous pain

**19.** The pharmaceutical composition according to claim 18 comprising a therapeutically effective amount of 0.1 to 1000 mg, preferably 1 to 500mg of the compound according to any one of claims 1 to 11, or a pharmaceutically acceptable salt thereof,.

**20.** A compound according to one or more of claims 1 to 11, or a pharmaceutically acceptable salt thereof or a pharmaceutical composition according to claim 17 for use in the prevention or treatment of cognitive disorders, post operative cognitive decline, Alzheimer's disease, schizophrenia, positive, negative and/or cognitive symptoms associated with schizophrenia, cognitive impairment associated with schizophrenia, cognitive deficits associated with Down syndrome, cognitive deficits associated with autism, cognitive deficits associated with neurofibromatosis type I, or cognitive deficits after stroke.

Europäisches Patentamt
European Patent Office
Office européen des brevets

# EUROPEAN SEARCH REPORT

Application Number
EP 20 18 9671

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y<br>A | WO 2020/016443 A1 (BOEHRINGER INGELHEIM INT [DE]) 23 January 2020 (2020-01-23)<br>* page 10 - page 15; table 2 *<br>* claims 1-12 *<br>* page 65 *<br>* page 67 - page 69 *<br>----- | 1-14, 17-20<br>15,16 | INV.<br>C07D401/14<br>C07D403/14<br>C07D409/14<br>C07D413/14<br>C07D498/04<br>A61P25/00<br>A61K31/501 |
| Y | WO 2014/001279 A1 (ANIONA APS [DK]) 3 January 2014 (2014-01-03)<br>* page 3; claims 1-7 *<br>----- | 1-14, 17-20 | |

TECHNICAL FIELDS SEARCHED (IPC)

C07D

The present search report has been drawn up for all claims

1

| Place of search | Date of completion of the search | Examiner |
|---|---|---|
| Munich | 12 January 2021 | Lewis, Sara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 20 18 9671

This annex lists the patent family members relating to the patent documents cited in the above-mentioned European search report. The members are as contained in the European Patent Office EDP file on
The European Patent Office is in no way liable for these particulars which are merely given for the purpose of information.

12-01-2021

| Patent document cited in search report | Publication date | Patent family member(s) | Publication date |
|---|---|---|---|
| WO 2020016443 A1 | 23-01-2020 | BR 102019014802 A2 | 04-02-2020 |
| | | TW 202019915 A | 01-06-2020 |
| | | US 2020024261 A1 | 23-01-2020 |
| | | WO 2020016443 A1 | 23-01-2020 |
| WO 2014001279 A1 | 03-01-2014 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SIEGHART.** *Pharmacol. Rev.,* 1995, vol. 47, 181-234
- **BALLARD, T.M. et al.** RO4938581, a novel cognitive enhancer acting at GABAA α5 subunit-containing receptors. *Psychopharmacology,* 2009, vol. 202, 207-223
- *J. Pharmacol. Exp. Ther.,* 2006, vol. 316, 1335-1345